# EUROPEAN PATENT SPECIFICATION

(11) **EP 0 674 625 B1**
(45) Date of publication and mention of the grant of the patent: **10.12.1997**
(21) Application number: 94902215.6
(22) Date of filing: 12.11.1993
(51) Int. Cl.: C07D 231/44, A61K 31/415, A61K 31/47, C07D 401/06, C07D 231/38, C07D 231/52, C07D 403/06

(54) **AMINO-SUBSTITUTED PYRAZOLES HAVING CRF ANTAGONISTIC ACTIVITY**
AMINOSUBSTITUIERTE PYRAZOLE MIT CRF ANTAGONISTISCHER AKTIVITÄT
PYRAZOLES AMINO-SUBSTITUES A ACTIVITE ANTAGONISTE DU FACTEUR LIBERATEUR DE CORTICOTROPHINES (CRF)

(30) Priority: 17.12.1992 US 991763
(43) Date of publication of application: 04.10.1995
(73) Proprietor: PFIZER INC., New York, N.Y. 10017 (US)
(72) Inventor: BRIGHT, Gene, Michael, Groton, CT 06340 (US)
(74) Representative: Ruddock, Keith Stephen
(86) International application number: US9310716
(87) International publication number: WO9413644

(56) References cited:
- US-A- 5 063 245

## Description

This invention relates to substituted pyrazoles, pharmaceutical compositions containing them, and their use in the treatment of stress-related and other diseases. The compounds have corticotropin-releasing factor (CRF) antagonist activity.

CRF antagonists are mentioned in U.S. Patents 4,605,642 and 5,063,245 referring to peptides and pyrazolinones, respectively. The importance of CRF antagonists is set out in the literature, e. g. as discussed in U.S. Patent 5,063,245, which is incorporated herein by reference. A recent outline of the different activities possessed by CRF antagonists is found in M. J. Owens et al., Pharm. Rev., Vol. 43, pages 425 to 473 (1991), also incorporated herein by reference. Based on the research described in these two and other references, CRF antagonists are considered effective in the treatment of a wide range of diseases including stress-related illnesses, such as stress-induced depression, anxiety, and headache; abdominal bowel syndrome, inflammatory diseases; immune suppression; human immunodeficiency virus (HIV) infections; Alzheimer's disease; gastrointestinal diseases; anorexia nervosa; hemorrhagic stress; drug and alcohol withdrawal symptoms; drug addiction, and fertility problems.

The present invention relates to a compound of the formula 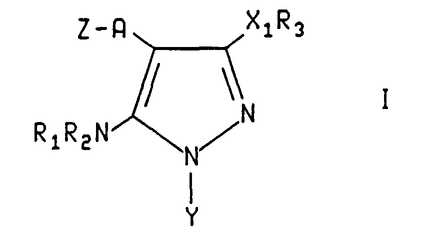 and the pharmaceutically acceptable acid addition salts thereof,
wherein
A is CH₂;
X₁ is a covalent bond, CH₂, O, S, or NR, wherein R is hydrogen, linear C₁-C₆ alkyl, or branched C₃-C₈ alkyl;
R₁, R₂ and R₃ are each independently linear C₁-C₆ alkyl, branched C₃-C₈ alkyl, C₃-C₈ alkenyl wherein the double bond is not adjacent to the N or X₁ when X₁ is oxygen or sulfur, or C₃-C₇ cycloalkyl (CH₂)ₙ wherein n is 0, 1, 2, 3 or 4; or R₁ and R₂ when taken together with the nitrogen form a saturated four, five or six membered ring optionally condensed with benzo; and R₃ may also be (CH₂)_{q}Q₁R₁₉ wherein q is 0, 1 or 2, Q₁ is O, S, NH or N(C₁-C₆ alkyl) when X₁ is a covalent bond or Q₁ is a covalent bond when X₁ is not a covalent bond, and R₁₉ is hydrogen, linear C₁-C₆ alkyl, branched C₃-C₈, C₃-C₈ alkenyl, or C₃-C₆ cycloalkyl (CH₂)ₙ wherein n is 0 to 4;
Y is phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, or piperidinyl, each of which may be substituted by one to three of any one of fluoro, chloro, bromo, or methyl, or one of trifluoromethyl; with the proviso that Y is not unsubstituted phenyl; and
Z is
   (a) 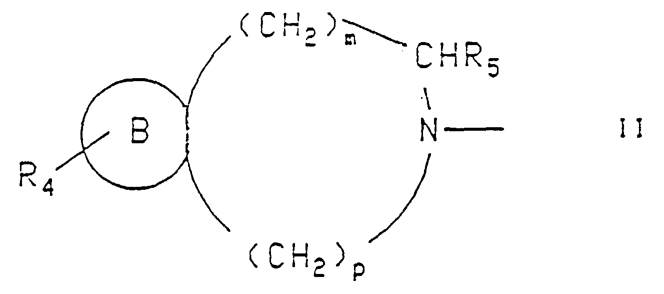 wherein
      the B ring is phenyl, naphthyl, pyridyl, pyrimidinyl, pyrazinyl, pyridazinyl, triazinyl, pyrrolyl, pyrazolyl, imidazolyl, triazolyl, thienyl, or indolyl, each of which may be substituted by methyl, methoxy, fluoro, chloro, bromo or iodo; or a saturated 5- or 6-membered carbocyclic ring or a partially unsaturated ring having one or two double bonds;
      R₄ is hydrogen, C₁-C₆ alkyl, C₁-C₆ alkoxy, hydroxy, fluoro, chloro, bromo, iodo, or trifluoromethyl;
      R₅ is hydrogen, linear C₁-C₆ alkyl, branched C₃-C₈ alkyl, C₃-C₈ alkenyl, or (CH₂)ₒ-X₂-(CH₂)ᵣ-Q₂-R₆;
      R₆ is hydrogen, linear C₁-C₅ alkyl, branched C₃-C₈ alkyl, or C₃-C₈ alkenyl;
      X₂ and Q₂ are each independently O, S, NH, N(C₁-C₆ alkyl), or one of X₂ and Q₂ may be a covalent bond;
      m is 0 or 1;
      o is 1 or 2;
      p is 1 or 2; and
      r is 0, 1, or 2;
   (b) 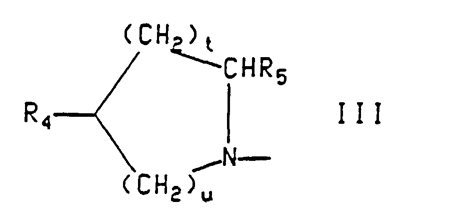 wherein R₄ and R₅ are as defined above, and t and u are each independently 1 or 2;
   (c) -NR₇R₈ wherein R₇ and R₈ are each independently hydrogen, C₁-C₆ linear alkyl, branched C₃-C₈ alkyl, C₃-C₈ alkenyl, (CH₂)ᵥCH₂OH, (CH₂)ᵥNR₉R₁₀, wherein v is 0 to 3, and R₉ and R₁₀ are each independently hydrogen, or linear C₁-C₆ alkyl; C₁-C₁₂ cycloalkyl, (C₃-C₁₂ cycloalkyl) (CH₂)ₙ, (C₆-C₁₀ bicycloalkyl) (CH₂)ₙ, wherein n is 0 to 4, benzofused C₃-C₆ cycloalkyl, C₁-C₆ hydroxyalkyl, phenyl, phenyl (C₁-C₃ alkylene), each of which may be substituted by one or two of hydroxy, fluoro, chloro, bromo, C₁-C₅ alkyl, or C₁-C₅ alkoxy; or R₇ and R₈ may be taken together with the nitrogen to form a saturated or partially unsaturated 5- to 7-membered ring which may contain one of O, S, NH or N(C₁-C₆ alkyl) and which may be substituted by C₁-C₆ alkyl, hydroxy or phenyl wherein any double bond(s) are not adjacent to any heteroatoms;
   (d) 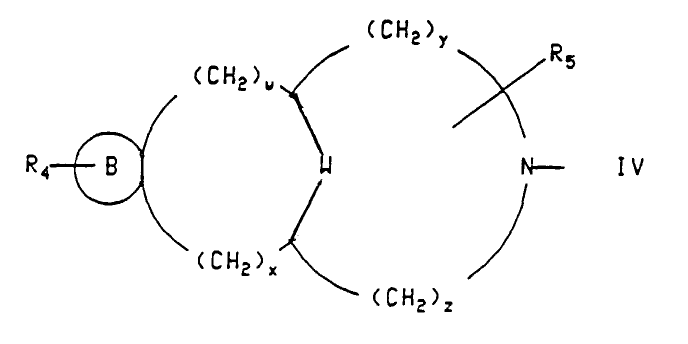 wherein B, R₄ and R₅ are as defined above, w, x, y and z are each independently 1 or 2, and W is (CH₂)_{q} wherein q is as defined above, N(C₁-C₆ alkyl), or oxygen;
   (e) 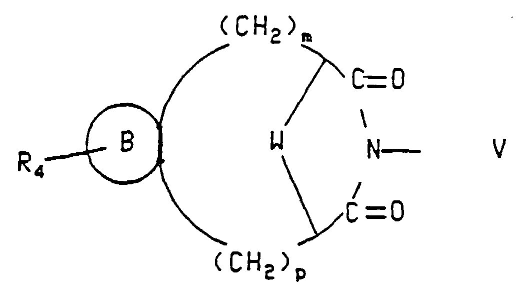 wherein W B, R₄, m and p are as defined above;
   (f) 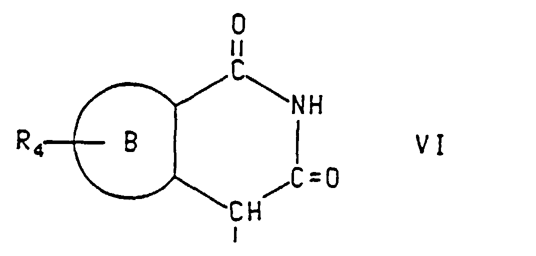 wherein B and R₄ are as defined above;
   (g)

      O(CH₂)ᵥR₁₁

      wherein v is 0 to 3 and R₁₁ is linear C₁-C₆ alkyl, branched C₃-C₈ alkyl, phenyl, naphthyl, 1,2,3,4-tetrahydronaphthyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, piperidinyl, or thienyl, each of which may be substituted by one or two of any one of fluoro, chloro, bromo, methyl, or trifluoromethyl;
   (h) 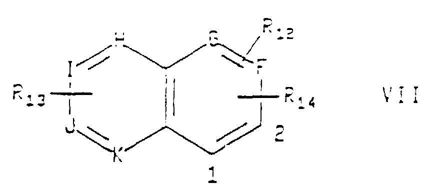 wherein the moiety of formula VII is linked via position 1 or 2 to the moiety A shown in formula I while R₁₄ is attached to position 2 or 1, respectively; F, G, H, I, J and K are independently C or N, provided that not more than three of H, I, J and K are N with not more than two adjacent nitrogens; R₁₂ and R₁₃ each independently are hydrogen, linear C₁-C₆ alkyl, branched C₃-C₈ alkyl, C₃-C₈ alkenyl, fluoro, chloro, bromo, trifluoromethyl, hydroxy, thiol, C₁-C₁₂ alkoxy, C₁-C₁₂ thioalkanyl, or C₃-C₁₂ alkenoxy or C₃-C₁₂ thioalkenyl wherein the double bond is not adjacent to the oxygen or suflur, and R₁₄ is hydroxy, C₁-C₁₂ alkoxy, C₃-C₁₂ alkenoxy wherein the double bond is not adjacent to the oxygen, or -X₂-(CH₂)ᵣQ₂R₆ wherein X₂, r, Q₂ and R₆ are as defined above in paragraph (a) except that Q₂ is not sulfur, or R₁₄ is NR₁₅R₁₆ wherein R₁₅ and R₁₆ are each independently hydrogen, linear C₁-C₆ alkyl, branched C₃-C₈ alkyl, C₃-C₈ alkenyl wherein the double bond is not adjacent to the nitrogen, or C₃-C₇ cycloalkyl-(CH₂)ₙ wherein n is as defined above, or R₁₅ and R₁₆ together with the nitrogen form a saturated five or six membered ring optionally condensed with benzo; or
   (i) 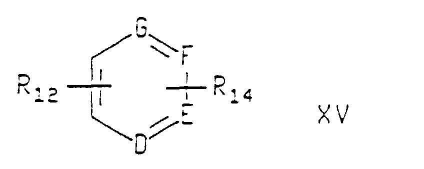 wherein D, E, F and G are independently C or N, provided that not more than two of D, E, F and G are N, and R₁₂ and R₁₄ are as defined in paragraph (h), A, defined above, is linked to a carbon in formula XV, and R₁₄ is linked to a carbon coated adjacent to the carbon to which A is linked.

More specific compounds of formula I of the invention include those wherein Y is phenyl substituted by three substituents one each at positions 2, 4 and 6, e.g. 2,4,6-trichlorophenyl, 2,6-dichloro-4-trifluoromethylphenyl, or 2,6-dichloro-4-fluorophenyl. Other more specific compounds of formula I include those wherein XR₃ is ethyl or methytthio, those wherein R₁ and R₂ are each methyl, and those wherein Z is NR₇R₈ and R, is phenyl or phenyl substituted by one of fluoro, chloro, nitro, methyl or methoxy and R₈ is as defined above, preferably, (CH₂)₃OH, CH₂CH₂OH or methyl.

Preferred compounds of formula I are those wherein Z is 1,2,3,4-tetrahydroisoquinolin-2-yl substituted by R₅ which is -(CH₂)ₒ-X₂-(CH₂)ᵣ-Q₂-R₆, more specifically R₅ is -(CH₂)ₖOH wherein k is an integer of 1 to 4, or -CH₂OCH₂CH₂OR₆. Other preferred compounds of formula I are those wherein Z is 1,2,3,4-tetrahydroquinolin-2-yl wherein R₅ is substituted at position 3, and the absolute configuration at the 3 position is either S or R or R,S.

Preferred compounds of the formula I include those wherein Z is as defined in above subparagraph (h); and those wherein Z is as defined in (h), A is linked to position 1, F, G, H, I, J and K are each carbon, and R₁₄ is methoxy, ethoxy, isopropoxy, or cyclopropylmethoxy at position 2.

Other preferred compounds of formula I are those wherein Z is as defined in above subparagraph (h), A is linked to position 1, K is nitrogen, F, G, H, I, and J are each carbon, and R₁₄ is -X₂-(CH₂)ᵣQ₂R₆ at position 2; those wherein Z is as defined in (h), A is linked to position 1, K is nitrogen, F, G, H, I, and J are each carbon, and R₁₄ is methoxy, ethoxy, isopropoxy, or cyclopropylmethoxy at position 2; and those wherein Z is as defined in (h), A is at position 1, and R₁₄ is ethoxy, isopropoxy or cyclopropylmethoxy at position 2. In these preferred compounds of formula I wherein Z is as defined in (h), R₁₂ and R₁₃ are preferably hydrogen.

Other preferred compounds of formula I are those wherein Z is as defined in subparagraph (a), B is phenyl, p and m are each 1, and R₅ is CH₂OCH₃.

Preferred compounds of formula I include those wherein Z is 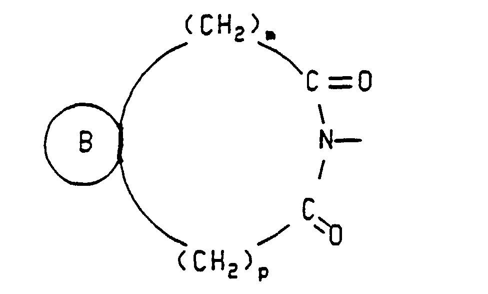 wherein B is phenyl, m is 0, and p is 1.

Specific most preferred compounds of the invention include:
2-{1-[1-(2,6-dichloro-4-trifluoromethylphenyl)-5-dimethylamino-3-ethyl-1H-pyrazol-4-ylmethyl]-napthalen-2-yloxy}-ethanol;
enantiomeric [4-(3-methoxymethyl-3,4-dihydro-1H-isoquinolin-2-ylmethyl)-5-methylsulfanyl-2-(2,4,6-trichlorophenyl)-2H-pyrazol-3-yl]-dimethylamine derived from (+)-3-hydroxymethyl-1,2,3,4-tetrahydroisoquinoline;
enantiomeric [2-(2,6-dichloro-4-trifluoromethylphenyl)-4-(3-ethoxymethyl-3,4-dihydro-1H-isoquinolin-2-ylmethyl)-5-ethyl-2H-pyrazol-3-yl]-dimethylamine derived from (+)-3-hydroxymethyl-1,2,3,4-tetrahydroisoquinoline;
[2-(2,6-dichloro-4-trifluoromethylphenyl)-5-ethyl-4-(7-methoxyquinolin-8-ylmethyl)-2H-pyrazol-3-yl]-dimethylamine;
[2-(2,6-dichloro-4-trifluoromethylphenyl)-4-(2-ethoxy-naphthalen-1-ylmethyl)-5-ethyl-2H-pyrazol-3-yl]-dimethylamine;
[4-(2-ethoxynaphthalen-1-ylmethyl)-5-ethyl-2-(2,4,6-trichlorophenyl)-2H-pyrazol-3-yl]-dimethylamine;
[4-(7-methoxyquinolin-8-ylmethyl)-5-methylsulfanyl-2-(2,4,5-trichlorophenyl)-2H-pyrazol-3-yl]-dimethylamine;
2-{1-[5-dimethylamino-3-methylsulfanyl-1-(2,4,6-trichlorophenyl)-1H-pyrazol-4-ylmethyl]-naphthalen-2-yloxy}-ethanol;
enantiomeric [2-(2,6-dichloro-4-trifluoromethylphenyl)-5-ethyl-4-(3-methoxymethyl-3,4-dihydro-1H-isoquinolin-2-yimethyl)-2H-pyrazol-3-yl]-dimethylamine derived from(+)-3-hydroxymethyl-1,2,3,4-tetrahydroisoquinoline;
[4-(2-cyclopropylmethoxynaphthalen-1-ylmethyl)-5-methylsulfanyl-2-(2,4,6-trichlorophenyl)-2H-pyrazol-3-yl]-dimethylamine;
2-{[5-dimethylamino-3-ethyl-1-(2,4,6-trimethylphenyl)-1H-pyrazol-4-ylmethyl]naphthalen-2-yloxy}-ethanol;
2-{1-[5-dimethylamino-3-methylsulfanyl-1-(2,4,6-trimethylphenyl)-1H-pyrazol-4-ylmethyl]-naphthalen-2-yloxy}-ethanol;
[2-(2,6-dichloro-4-trifluoromethylphenyl)-5-methoxymethyl-4-(2-methoxynaphthalen-1-ylmethyl)-2H-pyrazol-3-yl]-dimethylamine;
2-{1-[5-dimethylamino-3-ethyl-1-(2,4,6-trichloropnenyl)-1H-pyrazol-4-ylmethyl]naphthalen-2-yloxy}-ethanol;
[5-ethyl-4-(2-methoxy-naphthalen-1-ylmethyl)-2-(2,4,6-trimethylphenyl)-2H-pyrazol-3-yl]-dimethylamine;
2-{2-[1-(2,6-dichloro-4-trifluoromethylphenyl)-5-dimethylamino-3-ethyl-1H-pyrazol-4-ylmethyl]-1,2,3,4-tetrahydroisoquinolin-3-ylmethoxy}-ethanol;
[4-(3-methoxymethyl-3,4-dihydro-1H-isoquinolin-2-ylmethyl)-5-methylsulfanyl-2-(2,4,6-trimethylphenyl)-2H-pyrazol-3-yl]-dimethylamine; and
2-{2-[5-dimethylamino-3-ethyl-1-(2,4,6trichlorophenyl)-1H-pyrazol-4-ylmethyl]1,2,3,4-tetrahydroisoquinolin-3-ylmethoxy}-ethanol.

The invention includes a pharmaceutical composition for the treatment of illnesses induced or facilitated by corticotropin releasing factor which comprises a compound of the formula I as defined above in an amount effective in the treatment of said illnesses, and a pharmaceutically acceptable carrier, and a composition for the treatment of inflammatory disorders, stress and anxiety related disorders including stress-induced depression and headache, abdominal bowel syndrome, immune suppression, HIV infections, Alzheimer's disease, gastrointestinal diseases, anorexia nervosa, hemorrhagic stress, drug and alcohol withdrawal symtoms, drug addiction, and fertility problems, which comprises a compound of the formula I or IA as defined above in an amount effective in the treatment of said disorders, and a pharmaceutically acceptable carrier. Preferred and more specific compositions of the invention are those containing preferred and more specific compounds of formula I as described above.

The invention further relates to the use of a compound as defined by formula I or a salt thereof for the manufacture of a medicament for the treatment of illnesses induced or facilitated by coritcotropin releasing factor by administering to a subject in need of such treatment a compound of formula I as defined above, and a method for the treatment of stress and anxiety related disorders including stress-induced depression and headache, abdominal bowel syndrome, inflammatory disorders, immune suppression, HIV infections, Alzheimer's disease, gastrointestinal diseases, anorexia nervosa, hemorrhagic stress, drug and alcohol withdrawal symtoms, drug addiction, and fertility problems, particularly depression.

The invention also relates to an intermediate compound of the formula 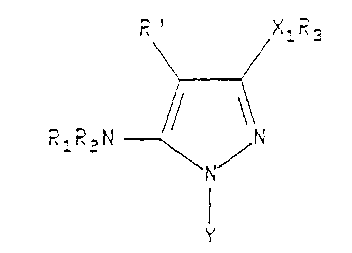 wherein R' is CH₂OH or C(O)O(C₁-C₃ alkyl), R₁, R₂ and R₃ are each independently linear C₁-C₆ alkyl, branched C₃-C₈ alkyl, C₃-C₈ alkenyl wherein the double bond is not adjacent to the N or X₁ when X₁ is oxygen or sulfur, C₃-C₇ cycloalkyl (CH₂)ₙ wherein n is 0, 1, 2, 3 or 4; or R₁ and R₂ when taken together with the nitrogen form a saturated four, five or six membered ring optionally condensed with benzo;
X₁ is a covalent bond, CH₂NR wherein R is hydrogen or linear C₁-C₆ alkyl, O, or S; and
Y is phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, or piperidinyl, each of which may be substituted by one to three of any one of fluoro, chloro, bromo, or methyl, or one of trifluoromethyl; with the proviso that Y is not unsubstituted phenyl.

Whenever reference herein is made to groups (CH₂)_{q}Q₁R₁₉ and (CH₂)ₒ-X₂-(CH₂)ᵣ-Q₂-R₆, then X₁ and Q₁, and X₂ and Q₂, respectively, are not both a heteroatom when q or r, respectively, is 1.

Whenever Y or R₁₁ is a heterocyclic group, the attachment of the group is through a carbon atom.

The compounds of formula I may be prepared by reaction of a compound of the formula 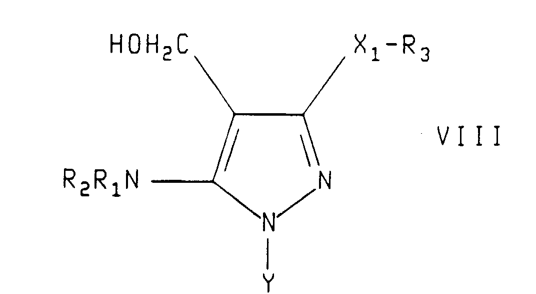 with a sulfonyl chloride such as methylsulfonylchloride, in a solvent such as methylene chloride or toluene, at temperatures of about -10 to about 30°C, followed by reaction with a compound of the formula ZH or ZMetal wherein Z is as defined above and Metal is an alkali metal such as sodium, lithium or potassium. The reaction with ZH proceeds generally in the presence of a solvent such as methylene chloride or toluene at temperatures of about 50 to about 100°C in the presence of a strong base such as an alkali metal hydride, e.g. sodium hydride, lithium hydride or potassium hydride, except when ZH is a sufficiently strong base itself, in which case a stoichiometric excess of ZH may be used or a stoichiometric amount of ZH in the presence of a suitably strong base such as a trialkylamine, e.g. triethylamine. The reaction with ZMetal proceeds in solvents such as N,N-dimethylformamide at temperatures in the range of about 50 to about 100°C.

The compounds of formula VIII may be prepared by reacting a compound of the formula 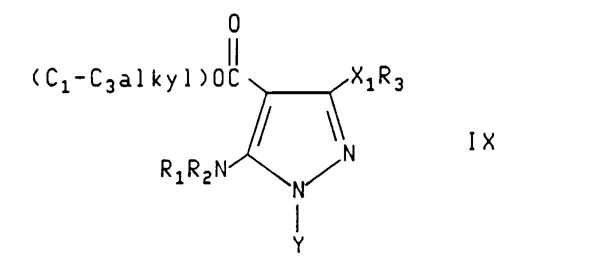 wherein X₁, Y, R₁, R₂ and R₃ are as defined with reference to formula I, except that R₁ and R₂ are not hydrogen, with a reducing agent which is compatible with the chemical substituents on the aminopyrazole ring, such as diisobutylaluminum hydride in a reaction inert solvent such as tetrahydrofuran or ether, at temperatures of about -5 to 30°C.

The compounds of the formula IX may be prepared from the corresponding compounds wherein R₁ and R₂ are hydrogen (formula X, not shown) by reacting first with an alkali metal hydride such as sodium hydride and then with alkylating agents R₁Hal and R₂Hal wherein Hal is chloro or bromo and R₁ and R₂ are as defined with reference to formula I except hydrogen, in a solvent such as tetrahydrofuran at temperatures of about 5 to 80°C.

The compounds of the formula X may be prepared by reacting a 2-cyano-acrylic acid ester of the formulae 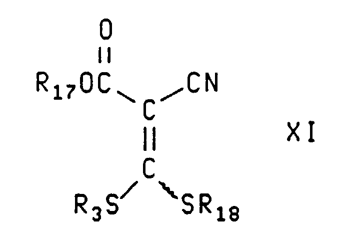 to make a compound of the formula I wherein X₁ is S, 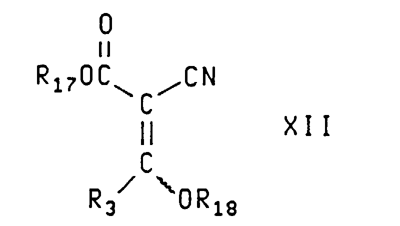 to make a compound of formula I wherein X₁ is a covalent bond, 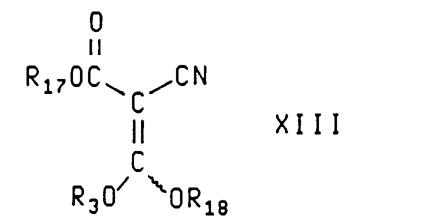 to make a compound of formula I wherein X₁ is O, or 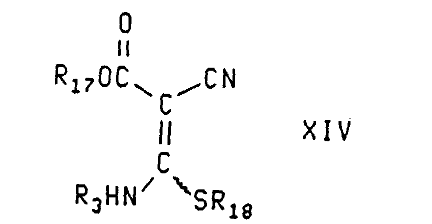 to make a compound of formula I wherein X₁ is NR and R is hydrogen, wherein R₁₇ is C₁-C₃ alkyl and R₁₈ is C₁-C₂ alkyl, with a hydrazine of the formula NH₂NHY wherein Y is as defined with reference to formula I. The reaction is carried out in a solvent, such as a C₁-C₈ alcohol, at about 50 to 150°C, conveniently the reflux temperature of the reaction mixture. The wavy line 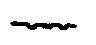 in some of the formulae herein indicates that either isomer of this compound is included, in accordance with accepted convention for indicating stereoisomers.

The intermediates of formula IX obtained by the above reaction from the compound of formula XIV have the formula 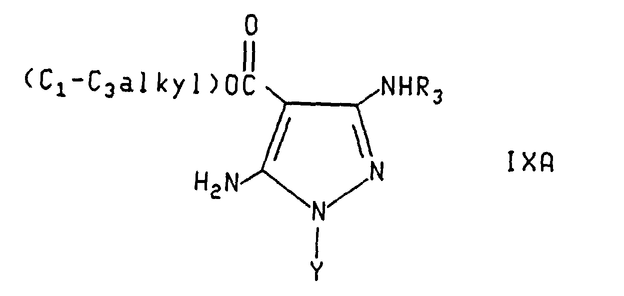 They may be trialkylated by using at least three equivalents of alkali metal hydride and R₂Hal wherein Hal is chloro or bromo, in the manner described above for conversion of the compounds of formula X to the compounds of formula IX, to obtain the compounds of formula I wherein R₁, R₂ and R are as defined above with reference to formula I other than hydrogen.

The above intermediates of the formula IXA may be reacted with a compound providing an N-protecting group to replace the hydrogen in the NHR₃ group followed by alkylation with R₂Hal or R₃Hal and removal of the N-protecting group to provide the compounds of the formula I wherein X₁ is NRR₃ wherein R is hydrogen, and R₂ and R₃ are as defined above with reference to formula I other than hydrogen.

Reaction of the compounds of the formula 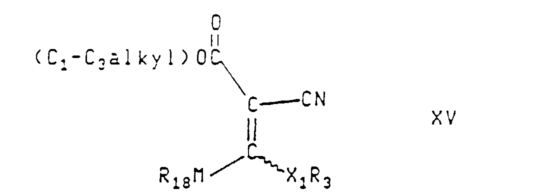 wherein M is X₁ is S and R₃ is R₁₈ is C₁-C₆ alkyl with amines such as RNH₂ or RR₃NH in an appropriate solvent such as ethanol at temperatures of about 0° to about 100°C results in compounds of the formula XV in which R₁₈-M and X₁-R₃ are each RNH or NRR₃, wherein R is as defined with reference to formula I and R₃ is linear alkyl, branched C₃-C₈ alkyl, or C₃-C₈ alkenyl wherein the double bond is not adjacent to the nitrogen.

The compounds of formula I wherein Z is as defined above in paragraphs (a), (h) or (i) wherein R₅ or R₁₄ is X₂(CH₂)ᵣQ₂R₆, wherein Q₂ is oxygen, and X₂, r, and R₆ are as previously defined except that R₆ is not hydrogen, may be prepared by alkylation of the corresponding compound wherein R₅ or R₁₄ are (CH₂)ₒ-X₂-(CH₂)₂-Q₂-R₆ and -X₂-(CH₂)ᵣQ₂R₆, respectively, wherein R₆ is hydrogen and Q₂ is oxygen. In these cases wherein R₅ and R₁₄ have a terminal hydroxy group, the hydroxy is first reacted with a strong base such as an alkali metal hydride, e.g. lithium, sodium or potassium hydride, in a solvent such as dimethylformamide at about 50° to 100°C.

The resulting alkali metal alkoxide is then reacted with an alkyl or aryl sulfonyl ester of the formula HO(CH₂)ᵣQ₂R₆ wherein R₆ is as defined in paragraph (a) except hydrogen. This reaction is carried out in the presence of a solvent such as methylene chloride or toluene at about 50° to 100°C. The above sulfonyl esters may be prepared by the same method as described above for the activation of the compound of formula IX.

The above alkali metal hydride may be replaced by other strong bases including organometallic bases such as n-butyl lithium or amine anion bases such as lithium diisopropylamide. In such case, the metal alkoxide formation reaction may be carried out in tetrahydrofuran at temperatures of about -5° to about 65°C.

The above alkylation may also be used to prepare compounds of the formula I wherein X₁ is oxygen and R₃ is (CH₂)_{q}Q₁R₁₉ wherein q, Q₁ and R₁₉ are as defined above with reference to formula I except that R₁₉ is not hydroxy, from the corresponding compounds wherein X₁R₃ is hydroxy.

The compounds of formula IA wherein A is CH(C₁-C₆ alkyl), or CH(CH₂)ₙ(C₃-C₈ alkenyl) wherein n is O to 4 (having formula IB, not shown) may be prepared from the compounds of formula IX by reaction with a Grignard reagent of the formula R₁₉MgHal wherein R₁₉ is C₁-C₆ alkyl, or (CH₂)ₙ(C₃-C₈ alkenyl) wherein n is 0 to 4, in a conventional manner, e.g. in diethyl ether or tetrahydrofuran solvent at about -78° to 50°C, to form a ketone of the formula 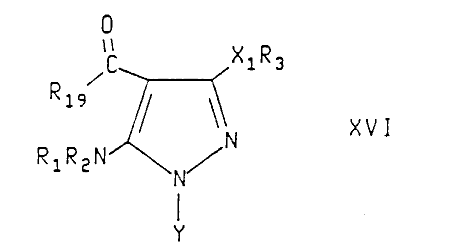 The ketone XVI may be converted to the corresponding enamine by reaction with a compound of the formula ZH wherein Z is (a) to (d) as defined above under standard acid catalyzed dehydrogenation conditions. The enamine may be converted into the compounds of formula IA wherein A is CHR₁₉ by hydrogenation with hydrogen under pressure in the presence of a noble metal catalyst or reduction with a hydride such as sodium or lithium cyanoborohydride in diethylether or tetrahydrofuran (THF).

Alternatively, the compounds of formula IB may be prepared from compounds IX by reaction with ZH wherein Z is (a) to (d) as defined above in the presence of a hydride reducing agent such as sodium or lithium cyanoborohydride.

The compounds of formula IA wherein A is C(C₁-C₆ alkyl)₂, C(C₁-C₆ alkyl)(C₃-C₈ alkenyl) or C(C₃-C₈ alkenyl)₂ may be prepared from the compound of formula IX by reaction with concentrated hydrochloric acid under reflux to form a compound of the formula 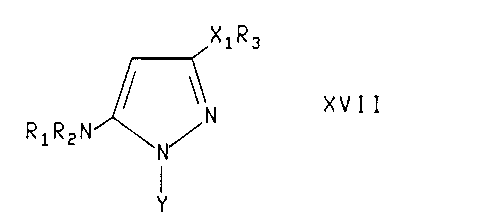 The compound XVII may be brominated, e.g. with pyridinium bromide in THF, to form the corresponding 4-bromide of formula XVIII (not shown) which may be 4-metalated in situ, such as with t-butyl lithium in diethyl ether at -78°C, and then treated in situ with an iminium compound of the formula 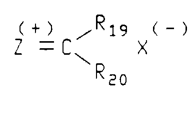 wherein R₁₉ is as defined above, R₂₀ is R₁₉, Z is (a) to (d) as defined above, and X is halogen.

The compounds of formula IA wherein A is CHR₁₉ wherein R₁₉ is as defined above, Z is (h) or (i) as defined above and R¹⁴ does not have acidic hydrogens, such as hydroxyls, may be prepared from compounds of the formula I wherein Z is (h) or (i) and the other substituents are as defined above with reference to formula I by treatment with a strong base such as t-butyl lithium in ether or THF and subsequent alkylation in the same solvent with a halide of the formula R₁₉X wherein R₁₉ and X are as defined above.

When the compounds of the invention contain a chiral center, it is understood that the invention includes the racemic mixture and the individual enantiomers of such compounds. For instance, the compounds of the invention wherein Z is 1,2,3,4-tetrahydroisoquinolinyl have a chiral center when Z is substituted at position 3 by R₅, wherein R₅ is as defined with reference to formula I except hydrogen, as follows: 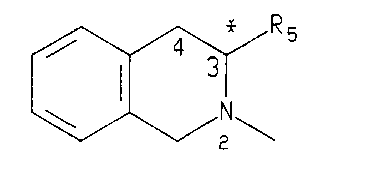 wherein the chiral center is indicated by an asterisk.

Preferred compounds of the invention of formula I or IA include those derived from the dextrorotatory (+) enantiomer of the intermediate compound ZH of the formula: 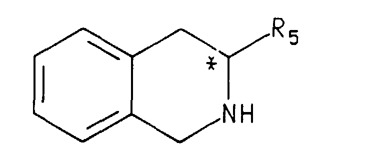 wherein R₅ is hydroxymethyl or (C₁-C₆ alkoxy)methyl.

The acid addition salts are prepared in a conventional manner by treating a solution or suspension of the free base of formula I or IA with one chemical equivalent of a pharmaceutically acceptable acid. Conventional concentration or crystallization techniques are employed in isolating the salts. Illustrative of suitable acids are acetic, lactic, succinic, maleic, tartaric, citric, gluconic, ascorbic, benzoic, cinnamic, fumaric, sulfuric, phosphoric, hydrochloric, hydrobromic, hydroiodic, sulfamic, sulfonic acids such as methanesulfonic, benzenesulfonic, p-toluenesulfonic, and related acids.

The compound of the invention may be administered alone or in combination with pharmaceutically acceptable carriers, in either single or multiple doses. Suitable pharmaceutical carriers include inert solid diluents or fillers, sterile aqueous solution and various organic solvents. The pharmaceutical compositions formed by combining the novel compounds of formula I or IA and the pharmaceutically acceptable carriers are then readily administered in a variety of dosage forms such as tablets, powders, lozenges, syrups, injectable solutions and the like. These pharmaceutical compositions can, if desired, contain additional ingredients such as flavorings, binders, excipients and the like. Thus, for purposes of oral administration, tablets containing various excipients such as sodium citrate, calcium carbonate and calcium phosphate may be employed along with various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions of the novel compound of formula I in sesame or peanut oil, aqueous propylene glycol, or in sterile aqueous solution may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. The sterile aqueous media employed are all readily available by standard techniques known to those skilled in the art.

Additionally, it is possible to administer the compounds of the present invention topically when treating inflammatory conditions of the skin and this may be done by way of creams, jellies, gels, pastes, and ointments, in accordance with standard pharmaceutical practice.

The effective dosage for the compound of formula I or IA depends on the intended route of administration and other factors such as age and weight of the patient, as generally known to a physician. The dosage also depends on the illness to be treated, although the daily dosage for the illnesses to be treated according to the invention, as listed above, will generally range from about 0.1 to about 50 mg/kg of the body weight of the patient to be treated. More specifically, for treatment of inflammatory diseases about 0.1 to about 100 mg/kg will be needed, for Alzheimer's disease about 0.1 to about 50 mg/kg, as well as for stress-induced illnesses, gastrointestinal diseases, anorexia nervosa, hemorrhagic stress, drug and alcohol withdrawal symptoms, and fertility problems.

The methods for testing the compounds of formula I or IA for their CRF antagonist activity are as described in Endocrinology, 116, 1653-1659 (1985) and Peptides, 10, 179-188 (1985) which determine the binding activity of a test compound to a CRF receptor. The binding activity for the compounds of formula I and IA generally ranges from about 0.2 nanomolar to about 10 micromolar..

The following Examples illustrate the invention.

### Example 1

### A. 5-Amino-3-methylsulfanyl-1-(2,4,6-trichlorophenyl)-1H-pyrazole-4-carboxylic acid methyl ester

An ethanol (3.5 l) solution of 2-cyano-3,3-bis-methylsulfanyl-acrylic acid methyl ester (454.3 g, 2.23 mol) and 2,4,6-trichlorophenylhydrazine (472.7 g, 2.23 mol) was vigorously refluxed for 1.5 hours. The reaction mixture was allowed to stand overnight at ambient temperature. Water (850 ml) was added, and the resulting mixture was briskly stirred for 30 minutes. The granular precipitate was filtered and washed with a water/ethanol(1:3 in volume)solution (1 l). The air-dried product was further dried under vacuum (45°C) for 2 days to afford the title compound as a yellow crystalline solid, m.p. 160-162°C.

### B. 5-Dimethylamino-3-methylsulfanyl-1-(2,4,6-trichlorophenyl)-1H-pyrazole-4-carboxylic acid methyl ester

To a well-stirred and ice-bath-chilled slurry of sodium hydride (232.4 g of 60% sodium hydride mineral oil dispersion, 139.4 g, 5.81 mol of sodium hydride) in anhydrous tetrahydrofuran (4 l), 711.6 g of the compound of Step A (1.94 mol) dissolved in anhydrous tetrahydrofuran was rapidly added dropwise, followed by slow dropwise addition of methyl iodide (1376 g, 9.7 mol). The reaction was then stirred at ambient temperature under nitrogen for 18 hours. The solvent was removed in vacuo. Ethyl acetate (2 l) and water (3 l) were added to dissolve the residue. The separated aqueous phase was extracted twice with 2 l portions of ethyl acetate. The combined organic extracts were washed with brine, dried over anhydrous sodium sulfate and concentrated in vacuo to afford the title compound as an amorphous solid in quantitative yield.

¹³C NMR (CDCl₃): 163.0, 156.9, 152.7, 136.0, 135.8, 134.2, 128.8, 51.1, 42.0, 13.4.

### C. (5-Dimethylamino-3-methylsulfanyl-1-(2,4,6-trichlorophenyl)-1H-pyrazol-4-yl)-methanol

To a well-stirred/ice-bath-chilled solution of the compound of Step B (322 g, 0.816 mol) in anhydrous tetrahydrofuran (4.5 l), diisobutylaluminum hydride (2.863 l of a 1.0 M tetrahydrofuran solution, 2.86 mol) was added dropwise over 2 hours. The reaction mixture was then stirred for an additional 40 minutes at 0-5°C prior to quenching (with ice-bath cooling) by addition of methanol (400 ml). A 1:1 (in volume) saturated aqueous Rochelle Salts/water solution (4 l), and ethyl acetate (3 l) was added, with good stirring. The organic phase was separated, extracted sequentially with equal volumes of water and brine, and finally, dried with anhydrous magnesium sulfate. Concentration in vacuo afforded a solid residue which crystallized from cyclohexane (3 I) to afford the title compound 132 g as a colorless crystalline solid, m.p. 108-110°C.

### Example 2

### A. 5-Amino-1-(2,6-dichloro-4-trifluoromethyl-phenyl)-3-methyl-sulfanyl-1H-pyrazole-4-carboxylic acid methyl ester

2-Cyano-3,3-bis-methylsulfanyl-acrylic acid methyl ester (49.8 g, 0.245 mol) and 2,6-dichloro-4-trifluoromethylphenylhydrazine (60.0 g, 0.245 mol) in anhydrous ethanol (390 ml) were combined, and the reaction mixture was vigorously refluxed for 1.5 hours. To the still-warm, well-stirred solution, water (480 ml) was added continuously (dropwise, rapid stream) over 10 minutes. Overnight stirring at ambient temperature afforded a heavy colorless solid precipitate of the title compound, isolated by suction filtration and in vacuo drying (80.7 g).

¹H NMR (CDCl₃): 2.44 (3H, s), 3.82 (3H, s), 5.18 (2H, s), 7.73 (2H, s).

### B. 1-(2,6-Dichloro-4-trifluoromethyl-phenyl)-5-dimethylamino-3-methylsulfanyl-1H-pyrazole-4-carboxylic acid methyl ester

To a well-stirred solution of the compound of Step A (35.4 g, 88.45 mmol) and methyl iodide (55.08 ml, 125.53 g, 0.884 mol) in anhydrous tetrahydrofuran (255 ml) chilled to 5°C, sodium hydride (10.62 g of 60% sodium hydride in mineral oil dispersion; 6.37 g, 0.266 mol of sodium hydride) was added portionwise over 10 minutes. The mixture was then vigorously refluxed for 5 hours. Inspection by thin layer chromatography showed complete reaction. The mixture was concentrated in vacuo to a solid which was then dissolved in an ethyl acetate/water mixture (150 ml of each), with the pH adjusted to 9 with sodium carbonate. The organic extract was separated, dried over anhydrous sodium sulfate, and concentrated in vacuo to afford the title compound as a colorless amorphous solid, 41.16 g.

¹H NMR (CDCl₃): 2.44 (3H, s), 2.72 (6H, s), 3.86 (3H, s), 7.71 (2H, s).

### C. [1-(2,6-Dichloro-4-trifluoromethyl-phenyl)-5-dimethyl-amino-3-methylsulfanyl-1H-pyrazol-4-yl]-methanol

To a well-stirred solution of the compound of Step B (6.4 g, 14.9 mmol) in anhydrous tetrahydrofuran (175 ml) maintained at -78°C, diisobutyl aluminum hydride in toluene (49.3 ml of a 1.0 M solution, 49.3 mmol) was slowly added dropwise over 10 minutes. Thin layer chromatography inspection of an aliquot after 20 minutes of stirring at -78°C showed incomplete reaction. The reaction mixture was immediately warmed to ambient temperature. Within 20 minutes at ambient temperature, reaction was found to be complete. (In addition to the desired product, a less polar byproduct identified as the corresponding C-4 methyl derivative, is formed). Methanol (162 ml) was added, cautiously at first, to quench the reaction. Warming of the mixture to 35°C for 15 minutes produced a granular precipitate. The reaction was concentrated in vacuo to a solid, which was extracted with ethyl acetate (150 ml). The solids were separated from the organic extract by filtration. The filtrate was concentrated in vacuo to an oil. Flash chromatography of the entire sample (silica gel, 40 micron mesh; elution with ethyl acetate/hexane, 1:3 in volume) afforded 1.93 g of the title compound as a colorless amorphous foam.

¹H NMR (CDCl₃): 2.50 (3H, s), 2.70 (6H, s), 4.50 (2H, s), 7.70 (2H, s).

### Example 3

### A. 5-Amino-1-(4-bromo-2,6-dimethyl-phenyl)-3-methyl-sulfanyl-1H-pyrazole-4-carboxylic acid methyl ester

In accordance with Example 2A, 4-bromo-2,6-dimethylphenylhydrazine (11.17 g, 55 mmol) was reacted with 2-cyano-3,3-bis-methylsulfanyl-acrylic acid methyl ester (13.8 g, 55 mmol) in 90 ml of ethanol. The title compound was obtained as a light-yellow amorphous solid, 13.0 g.

¹H NMR (CDCl₃): 2.06 (6H, s), 2.48 (3H, s), 3.86 (3H, s), 4.94 (2H, s), 7.32 (2H, s).

### B. 1-(4-Bromo-2,6-dimethyl-phenyl)-5-dimethylamino-3-methylsulfanyl-1H-pyrazole-4-carboxylic acid methyl ester

Utilizing the compound of Step A (13.0 g, 35 mmol, sodium hydride (4.2 g of 60% sodium hydride in mineral oil dispersion, 105 mmol of sodium hydride), methyl iodide (10.9 ml, 175 mmol) and tetrahydrofuran (90 ml) as solvent, the method of Example 1B afforded the title compound (14.15 g) as a yellow amorphous solid.

¹³C NMR (CDCl₃): 163.1, 155.5, 151.2, 138.5, 135.8, 131.3, 122.8, 100.8, 51.0, 42.0, 17.6, 13.4.

### C. [1-(4-Bromo-2,6-dimethyl-phenyl)-5-dimethyl-amino-3-methylsulfanyl-1H-pyrazol-4-yl]-methanol

Utilizing the compound of Step B (12.0 g, 30 mmol), diisobutylaluminum hydride (100 ml of a 1.0 M toluene solution, 100 mol), and anhydrous tetrahydrofuran (170 ml), the title compound was prepared by the method of Example 2C (3.6 g, isolated as a colorless oil).

¹³C NMR (CDCl₃): 151.8, 148.1, 138.7, 137.2, 131.1, 122.3, 106.7, 61.7, 42.6, 17.8, 15.7.

### Example 4

### A. 5-Amino-3-methyl-1-(2,4,6-trichloro-phenyl)-1H-pyrazole-4-carboxylicacid methyl ester

To a solution of 2-cyano-3-methyl-3-ethoxy-acrylic acid methyl ester (3.5 g, 0.021 mol) in glacial acetic acid (20 ml), 2,4,6-trichlorophenylhydrazine (4.38 g, 0.021 mol) was added with stirring, followed by triethylamine (2.0 ml, 1.46 g, 0.014 mol). The reaction was refluxed for 13 hours. Solvent removal in vacuo afforded a dark oil, which was dissolved in methylene chloride/water (100 ml of each). The separated organic phase was extracted with an equal volume of water, dried over anhydrous sodium sulfate, and concentrated in vacuo to an orange oil (5.8 g). Flash chromatography of the entire sample (silica gel, 40 micron mesh; elution with ethyl acetate/hexane=1:3 in volume) afforded the title compound (3.20 g) as a light-orange amorphous solid. Thin layer chromatography (TLC) R_{f} (silica gel plates, u.v. detection, ethyl acetate/hexane=1:3 in volume): 0.43.

### B. 5-Dimethylamino-3-methyl-(2,4,6-trichloro-phenyl)-1H-pyrazole-4-carboxylic acid methyl ester

To a well-stirred and ice-bath-chilled solution of the compound of Step A (3.2 g, 9.6 mmol) and methyl iodide (3.0 ml, 6.84 g, 48 mmol) in anhydrous tetrahydrofuran (20 ml), sodium hydride (1.15 g of 60% sodium hydride mineral oil dispersion, 690 mg, 29 mmol of sodium hydride) was added portionwise. The reaction mixture was stirred at 5°C for 15 minutes; then an ambient temperature for 20 hours. Concentration in vacuo afforded a light-yellow solid, which was dissolved in ethyl acetate/water (100 ml of each) with the pH adjusted to 12 with sodium carbonate. The separated aqueous phase was twice extracted with 50 ml portions of ethyl acetate. The three combined organic extracts were dried over anhydrous sodium sulfate and concentrated in vacuo to afford the title compound (3.5 g) as an orange amorphous solid. TLC R_{f} (siiica gel plates, u.v. detection, ethyl acetate/hexane=1:3 in volume): 0.73.

### C. [5-Dimethylamino-3-methyl-1-(2,4,6-trichlorophenyl)-1H-pyrazol-4-yl]-methanol

To a well-stirred solution of the compound of Step B (350 mg, 0.96 mmol) in anhydrous tetrahydrofuran (3 ml) chilled to -78°C, diisobulylaluminum hydride (2.90 ml of 1.0 M diisobutylaluminum hydride in tetrahydrofuran; 2.9 mmol of diisobutylaluminum hydride) was added dropwise. After stirring for one hour at -78°C and an additional hour at 5 ° C, the reaction was stirred at ambient temperature for 2 hours. The reaction was then quenched by dropwise (at 5°C) addition of methanol (7 ml). The mixture was stirred for 15 minutes at ambient temperature, and then for 10 minutes at 35-40°C. Concentration in vacuo afforded a yellow solid which was extracted with ethyl acetate (7 ml). The remaining insoluble solids were removed by filtration. The filtrate was extracted with an equal volume of water, dried (anhydrous sodium sulfate), and concentrated in vacuo to afford the title compound as a light-orange oil (0.32 g). The product was used in the next step without further purification.

### D. Enantiomeric {2-[5-Dimethylamino-3-methyl-1-(2,4,6-trichlorophenyl) -1H-pyrazol-4-yl-methyl]-1,2,3,4-tetrahydro-isoquinolin-3-yl}-methanol

To an ice-bath-chilled, well-stirred solution of the compound of Step C (160 mg, 0.48 mmol) and triethylamine (0.08 ml, 0.60 mmol) in anhydrous methylene chloride (3 ml), methanesulfonyl chloride (0.04 ml, 59.2 mg, 0.52 mmol) was added all at once. After 15 minutes of stirring at 5°C to complete in situ formation of the mesylate of the compound of Step C, the dextrorotatory enantiomer of 3-hydroxymethyl-1,2,3,4-tetrahydroisoquinoline (310 mg, 1.9 mmol), anhydrous N,N-dimethylformamide (0.51 ml) and acetonitrile (1.2 ml) were added. After stirring 1/2 hour at ambient temperature, the reaction mixture was heated at 55°C for 19 hours. Concentration in vacuo afforded an orange residue which was dissolved in ethyl acetate/water (100 ml of each) with the pH adjusted to 10 with sodium carbonate. The separated aqueous phase was extracted twice with 50 ml portions of ethyl acetate. The combined organic extracts were dried over anhydrous sodium sulfate and concentrated in vacuo to an orange semi-solid (0.7 g). Flash chromatography of the entire sample (silica gel, 40 micron mesh; eluting with ethyl acetate/hexane=1.3 volume) afforded the title compound (170 mg) as a light-orange oil.

¹³C NMR (CDCl₃): 151.5, 151.0, 136.2, 135.3, 135.0, 133.6, 133.3, 129.0, 128.6, 127.0, 126.5, 126.1, 107.5, 62.0, 58.0, 47.6, 45.6, 42.6, 26.2, 13.1.

### Example 5

### A. 2-Cyano-3,3-diethoxy-acrylic acid methyl ester

A reaction mixture consisting of methylcyanoacetate (5.0 ml, 57 mmol) and tetraethylorthocarbonate (17.99 ml, 86 mmol) in acetic anhydride (8.11 ml, 86 mmol) was heated at 130°C for 5 hours, and then heated at 110°C for 18 hours. The cooled reaction residue was extracted twice with 125 ml portions of hexane. A hexane-insoluble orange oil containing approximately 40% by weight of the desired compound (as established by NMR inspection) remained. This crude product was used in the next step without further purification. TLC R_{f} (silica gel plates, u.v. detection, ethyl acetate/hexane=15:85 in volume): 0.13.

### B. 5-Amino-3-ethoxy-1-(2,4,6-trichloro-phenyl)-1H-pyrazole-4-carboxylic acid methyl ester

The crude product obtained in Step A (4.76 g, containing approximately 1.9 g, 9.6 mmol of 2-cyano-3,3-diethoxy-acrylic acid methyl ester) was combined with 2,4,6-trichlorophenylhydrazine (2.02 g, 9.6 mmol) in ethanol (15 ml). The resulting mixture was refluxed for 18 hours. The solvent was removed in vacuo, and the residue was extracted with methylene chloride/water (100 ml of each) with the pH adjusted to 10 with sodium carbonate. The separated aqueous extract was washed with two 50 ml portions of methylene chloride. The three combined organic extracts were dried over anhydrous sodium sulfate and concentrated in vacuo to an oil (5.98 g). The purity of this crude title compound was significantly improved by flash chromatography (silica gel, 40 micron mesh; elution with ethyl acetate/hexane=15:85 in volume), yielding 910 mg of the title compound as an orange oil. TLC R_{f} (silica gel plates, u.v. detection; ethyl acetate hexane=15.85 in volume): 0.26. The product was used in the next step without further purification.

### C. 5-Dimethylamino-3-ethoxy-1-(2,4,6-trichloro-phenyl)-1H-pyrazole-4-carboxylic acid methyl ester

To an ice-bath-chilled solution of the compound of Step B (910 mg, 2.5 mmol) and methyl iodide (778 µl, 12.5 mmol) in anhydrous tetrahydrofuran (10 ml), sodium hydride (300 mg of 60% sodium hydride mineral oil dispersion; 180 mg, 7.5 mmol of sodium hydride) was added portionwise over 5 minutes. The reaction mixture was stirred at ambient temperature for 18 hours. The solvent was removed in vacuo, and the residue was extracted into methylene chloride/water (60 ml of each). The separated aqueous phase was extracted twice with 30 ml portions of methylene chloride. The organic extracts were combined, dried over anhydrous sodium sulfate, and concentrated in vacuo to an amber oil (980 mg). Flash chromatography of the entire sample (silica gel, 40 micron mesh; elution with ethyl acetate/hexane=5.95 in volume) afforded the title compound as a colorless oil (353 mg.)

TLC R_{f} (silica gel plates, u.v. detection, ethyl acetate/hexane=5:95 in volume): 0.26.

### D. [5-Dimethylamino-3-ethoxy-1(2,4,6-trichlorophenyl)-1H-pyrazol-4-yl]-methanol

Utilizing the procedure of Example 2C, the compound of Step 5C (340 mg, 0.87 mmol) was converted into the corresponding alcohol (230 mg of colorless oil). The product after workup (without chromatography) was used in the next step without further purification.

TLC R_{f} (silica gel plates, u.v. detection, ethyl acetate/hexane=15:85 in volume): 0.20.

### E. Enantiomeric {2-[5-Dimethylamino-3-ethoxy-1-(2,4,6-trichlorophenyl)-1H-pyrazol-4-ylmethyl]-1,2,3,4-tetrahydroisoquinoline-3-yl}-methanol

To a well-stirred/ice-bath-chilled methylene chloride (1 ml) solution of the compound of Step 5D (estimated 69 mg, 0.19 mmol) and triethylamine (32 µl, 0.23 mmol), methanesulfonyl chloride (16 µl, 0.21 mmol) was added. After 15 minutes of stirring, the dextrorotatory isomer of 3-hydroxymethyl-1,2,3,4-tetrahydroisoquinoline (124 mg, 0.72 mmol) and anhydrous N,N-dimethylformamide (0.1 ml) were added; and the resulting mixture was heated at 50°C for 4 hours. After ambient temperature stirring for an additional 48 hours, the solvent was removed in vacuo, and the residue was extracted into methylene chloride/water (60 ml of each) with the pH adjusted to 10 (sodium carbonate). The separated aqueous phase was extracted twice with 30 ml portions of methylene chloride. The combined organic extracts were dried (anhydrous sodium sulfate) and concentrated in vacuo to an oil (410 mg). Flash chromatography of the entire sample (silica gel, 40 micron mesh; elution with ethyl acetate/hexane=1:4 in volume) afforded the title compound (7 mg) as a colorless foam.

¹H NMR (CDCl₃); (3H, t, J=8 Hz), 2.5-2.8 (7H; 6H, s overlapping with 1H, m), 2.95 (1H, dd), 3.2-4.1 (7H, broad, overlapping multiplets) 4.25 (2H, q, J=8 Hz), 6.96-7.38 (4H, broad m), 7.45 (2H, s).

### Example 6

### Enantiomeric {2-[1-(4-Bromo-2,6-dimethyl-phenyl)-5-dimethylamino-3-methylsulfanyl-1H-pyrazol-4-ylmethyl]-1,2,3,4-tetrahydro-isoquinoline-3-yl}-methanol

Utilizing the dextrorotatory isomer of 3-hydroxymethyl-1,2,3,4-tetrahydroisoquinoline (528 mg, 3.24 mmol) dissolved in acetonitrile/anhydrous N,N-dimethylformamide (15 ml and 0.5 ml, respectively) and the in situ-generated mesylate of Example 3C (300 mg, 0.81 mmol), triethylamine (169 µl, 1.20 mmol), and methanesulfonyl chloride (81.5 µl, 1.05 mmol) in anhydrous methylene chloride (6 ml)] the title compound (21 mg, 5% yield) was prepared as a colorless amorphous solid by the Example 4D coupling method/workup and flash chromatography (silica gel, 40 micron mesh; elution with ethyl acetate/hexane=1:9 in volume).

TLC R_{f} (silica gel plates, u.v. detection, ethyl acetate/hexane=1:4 in volume): 0.30. HRMS m/z 514.1402 (M, C₂₅H₃₀BrN₄OS).

### Example 7

### Racemic {2-[5-Dimethylamino-3-methylsulfanyl-1-{2,4,6-trichloro-phenyl)-1H-pyrazol-4-ylmethyl]-1,2,3,4-tetrahydro-isoquinolin-3-yl}-methanol

To a stirred solution of the compound of Example 1C (700 mg, 1.9 mmol) and triethylamine (0.304 ml, 2.2 mmol) in anhydrous methylene chloride chilled to 5°C, methanesulfonyl chloride (0.164 ml, 2.1 mmol) was added to form the corresponding mesylate in situ. The reaction mixture was stirred at 5°C for 25 minutes. A solution of (±)-3-hydroxymethyl-1,2,3,4-tetrahydroisoquinoline (1.24 g, 7.6 mmol) in acetonitrile/anhydrous N,N-dimethylformamide (5 ml and 1.5 ml, respectively) was added, and the reaction mixture was heated with a 55-60°C oil bath for 18 hours. The solvent was removed in vacuo, and the residue was dissolved in a ethyl acetate/water mixture (100 ml of each) with the pH adjusted to 9.5 (sodium carbonate). The organic extract was separated, dried over anhydrous sodium sulfate, and concentrated in vacuo to an amber glass. Flash chromatography of the entire sample (silica gel, mesh; elution with ethyl acetate, hexane=1:10 in volume) afforded the title compound (800 mg) as a colorless amorphous solid.

¹³C NMR: (CDCl₃): 151.8, 149.6, 135.2, 135.5, 134.7, 133.6, 133.4, 129.0, 128.7, 127.1, 126.4, 126.0, 108.7, 62.2, 57.7, 48.3, 45.3, 42.8, 25.9, 14.7.

### Example 8

### Enantiomeric {2-[5-Dimethylamino-3-methylsulfanyl-1-(2,4,6-trichlorophenyl)-1H-pyrazol-4-ylmethyl]-1,2,3,4-tetrahydro-isoquinolin-3-yl}-methanol

To a stirred solution of the compound of Example 1C (258 mg, 0.704 mmol) and triethylamine (0.112 ml, 0.80 mmol) in anhydrous methylene chloride chilled to +5°C, methanesulfonyl chloride (0.061 ml, 0.79 mmol) was added to form the corresponding mesylate in situ. The reaction was stirred at 5°C for 20 minutes. A solution of the dextrorotatory enantiomer of 3-hydroxymethyl-1,2,3,4-tetrahydroisoquinoline (230 mg, 1.4 mmol) in acetonitrile/anhydrous N,N-dimethylformamide (2.5 ml and 1 ml, respectively) was added, and the resulting mixture was heated with a 60°C oil bath for 18 hours. The solvent was removed in vacuo and the resulting solid residue was dissolved in methylene chloride/water (25 ml of each) with the pH adjusted to 9.5 (sodium carbonate). The organic phase was separated, washed with an equal volume of water, dried (anhydrous sodium sulfate), and concentrated in vacuo to a solid. The entire sample was pulped in isopropyl alcohol (3 ml). After stirring for 1 hour at ambient temperature, a solid byproduct was filtered. The filtrate was concentrated in vacuo to an oil (0.41 g). Flash chromatography of the entire sample (silica gel, 40 micron mesh; elution with ethyl acetate/hexane=1:5 in volume) afforded the title compound (60 mg) as a colorless amorphous solid.

¹³C NMR (CDCl₃): identical to that of the racemic compound prepared in Example 7.

### Example 9

### Enantiomeric {2-[5-Dimethylamino-3-methylsulfanyl-1-(2,4,6-trichloro-phenyl)-1H-pyrazol-4-ylmethyl]-1,2,3,4-tetrahydro-isoquinolin-3-yl}-methanol

Following the procedure of Example 4D, and utilizing the levorotatory enantiomer of 3-hydroxymethyl-1,2,3,4-tetrahydroisoquinoline as the nucleophilic substrate, the title compound was prepared as a colorless amorphous solid.

¹³C NMR spectrum: identical in all respects to the one for the title (racemic) compound of Example 7.

### Example 10

### Enantiomeric {2-[1-(2,6-Dichloro-4-trifluoromethyl-phenyl)-5-dimethylamino-3-methyl-sulfanyl-1H-pyrazol-4-ylmethyl]-1,2,3,4-tetrahydro-isoquinolin-3-yl)-methanol

Utilizing the dextrorotatory enantiomer of 3-hydroxymethyl-1,2,3,4-tetrahydroisoquinoline (376 mg, 2.3 mmol) dissolved in acetonitrile/anhydrous N,N-dimethylformamide (1.5 ml and 0.5 ml, respectively) and the in situ-generated mesylate of the compound of Example 2C, the title compound (56 mg) was obtained as a colorless oil by the method of Example 4D and flash chromatography (silica gel, 40 micron mesh; elution with methanol/methylene chloride=1:4 in volume). TLC R_{f} (silica gel plates, u.v. detection, methanol/methylene chloride=1:4 in volume): 0.4. HRMS m/z 544.1078 (M, C₂₄H₂₄Cl₂F₃N₄OS).

### Example 11

### A. Racemic (2,3-Dihydro-1H-indol-2-yl)-methanol

Racemic 2,3-dihydro-1H-indole-2-carboxylic acid (5.0 g, 30.6 mmol) was suspended in methanol (50 ml). Sodium methoxide (1.66 g, 30.6 mmol) was added, and the resulting slurry was stirred several minutes before the solvent was removed in vacuo. The entire sample was suspended in anhydrous tetrahydrofuran (60 ml). Lithium aluminum hydride (1.0 M solution in tetrahydrofuran; 30.6 ml, 30.6 mmol) was added dropwise over 15 minutes. The reaction mixture was refluxed for 3 hours. The ice-bath-chilled mixture was then cautiously quenched by dropwise addition of 15 percent aqueous sodium hydroxide (4 ml). The reaction was filtered, and the filtrate was concentrated in vacuo to afford (±)-2-hydroxymethyl-indoline (3.69 g) as a viscous brown oil, used in the next step without further purification. TLC R_{f} (silica gel plates; u.v. detection, elution with ethyl acetate/hexane=1:4 in volume): 0.15.

### B. Racemic {1-[5-Dimethylamino-3-methylsulfanyl-1-(2,4,6-trichlorophenyl)-1H-pyrazol-4-ylmethyl]-2,3-dihydro-1H-indol-2-yl}-methanol

By the procedure of Example 4D, the racemic mixture of Step A (895 mg, 5 mmol) was reacted with the in situ prepared mesylate derived from 500 mg (1.5 mmol) of the compound of Example 1C. Flash chromatography (siiica gel, 40 micron mesh, eluting with ethyl acetate/hexane=1:4 in volume) of the crude product after workup as described in Example 4D afforded the title compound as a colorless cil (124 mg).

¹³C NMR (CDCl₃): 153.0, 150.7, 148.6, 136.2, 135.7, 135.0, 129.6, 128.7, 127.2, 124.5, 119.2, 109.7, 108.9, 67.8, 63.1, 45.1, 42.8, 31.7, 14.4.

### Example 12

### A. 2-Benzyl-4H-isoquinoline-1,3-dione

A mixture consisting of homophthalic acid (25 g, 0.139 mol) and benzylamine (15.2 ml, 14.91 g, 0.139 mol) was heated at 165-180°C for two hours, producing a vigorous release of water vapor. Cooling afforded a hard green solid which was pulverized, triturated with diethyl ether, and isolated by filtration to afford the title compound as a light-green granular solid (29.4 g) used in the next step without further purification.

TLC R_{f} (silica gel plates; u.v. detection, eluting with ethyl acetate/hexane=15:85 in volume): 43; ¹³C NMR (CDCl₃): 169.9, 164.8, 137.1, 134.1, 133.7, 129.3, 129.0, 128.4, 127.7, 127.5, 127.1, 125.4, 43.3, 36.5.

### B. Racemic 2-Benzyl-3-hydroxy-3,4-dihydro-2H-isoquinoline-1-one

To a well-stirred ice-bath-chilled solution of 2-benzyl-4H-isoquinoline-1,3-dione (5.0 g, 20 mmol) in methanol (40 ml), sodium borohydride (3.0 g, 80 mmol) was added portionwise over 20 minutes. The reaction mixture was stirred at ambient temperature for 48 hours. TLC inspection of a reaction aliquot confirmed substantial formation of product, with some starting material remaining. TLC R_{f} of the title compound (silica gel plates; u.v. detection; elution with ethyl acetate/hexane=3.7 in volume): 0.24. The ice-bath-chilled reaction mixture was cautiously quenched with water (3 ml). Anhydrous sodium sulfate was added to dry the mixture, which was then filtered. The filtrate was concentrated in vacuo to a dark green oil (3.5 g). Flash chromatography (silica gel, 40 micron mesh; elution with ethyl acetate hexane=3.7 volume) afforded the title compound as a colorless amorphous solid (1.35 g). The labile material was used immediately in the next step.

### C. Racemic (2-Benzyl-1-oxo-1,2,3,4-tetrahydro-isoquinolin-3-yl)-acetic acid methyl ester

Sodium hydride (307 mg of 60% sodium hydride mineral oil dispersion; 184 mg, 7.7 mmol of sodium hydride) was added portionwise over several minutes to a well-stirred, ice-bath-chilled solution of the racemic mixture of Step B (1.35 g, 5.34 mmol) in anhydrous tetrahydrofuran (20 ml). Methyl diethylphosphonoacetate (1.76 ml, 2.015 g, 9.6 mmol) was added portionwise over several minutes, and the resulting mixture was then stirred for 24 hours at ambient temperature. The solvent was removed in vacuo, and the residue was dissolved in ethyl acetate/water (50 ml of each). The separated aqueous extract was extracted twice with 10 ml portions of ethyl acetate. The combined organic extracts were dried over anhydrous sodium sulfate and concentrated in vacuo to a brown oil (2.4 g). Flash chromatography of the entire sample (silica gel, 40 micron mesh; elution with ethyl acetate/hexane=15:85 in volume) afforded the title compound (650 mg), as a colorless viscous oil.

¹³C NMR (CDCl₃): 171.3, 163.7, 137.8, 135.6, 132.2, 128.9, 128.7, 128.3, 128.1 (2), 127.6, 127.3, 51.8, 51.7, 48.7, 36.2, 32.5.

### D. Racemic 2-(2-Benzyl-1,2,3,4-tetrahydro-isoquinolin-3-yl)-ethanol

To a well-stirred ice-bath-chilled solution of the racemic mixture of Step C (650 mg, 2.0 mmol) in anhydrous tetrahydrofuran (10 ml), a solution of lithium aluminum hydride in tetrahydrofuran (6.11 ml of a 1.0M solution, 6.11 mmol of lithium aluminum hydride) was added dropwise over 5 minutes. The reaction mixture was then stirred for 24 hours at ambient temperature before being ice-bath-chilled and cautiously quenched with 15% aqueous sodium hydroxide (2 ml). Anhydrous sodium sulfate was added to dry the mixture, which was then filtered. The filtrate was concentrated in vacuo to afford the title compound as a colorless oil in quantitative yield.

TLC R_{f} (silica gel plates; u.v. detection, elution with ethyl acetate/hexane=1:4 in volume): 0.21.

### E. Racemic 2-(1,2,3,4-Tetrahydro-isoquinolin-3-yl)-ethanol

(±)-2-(2-Benzyl-1,2,3,4-tetrahydro-isoquinolin-3-yl)-ethanol (610 mg, 2.3 mmol) was hydrogenated at 40 p.s.i.g. on a Parr Apparatus (305 mg of 10% palladium-on-carbon catalyst; methanol/concentrated hydrochloric acid solvent (15 ml and 0.25 ml, respectively) for 5 hours. The catalyst was filtered, and the filtrate was concentrated in vacuo to an oil, which was dissolved in dilute aqueous (pH 9) sodium carbonate/methylene chloride (50 ml of each). The aqueous phase was separated and extracted with two 10 ml portions of methylene chloride. The combined organic extracts were dried over anhydrous sodium sulfate and concentrated in vacuo to afford the title compound as a viscous colorless oil (quantitative yield).

¹³C NMR (CDCl₃): 135.4, 134.2, 129.3, 126.3, 126.1, 125.9, 63.2, 55.5, 47.9, 37.2, 35.5.

### F. Racemic 2-{2-[5-Dimethylamino-methylsulfanyl-1-(2,4,6-trichlorophenyl)-1H-pyrazol-4-ylmethyl]-1,2,3,4-tetrahydro-isoquinolin-3-yl}-ethanol

Racemic 2-(1,2,3,4-tetrahydro-isoquinolin-3-yl)-ethanol (400 mg, 2.4 mmol) was reacted with the in situ-prepared mesylate of the compound of Example 1C by the method of Example 4D affording the title compound (80 mg) as a colorless viscous oil.

¹³C NMR (CDCl₃): 151.9, 149.8, 136.2, 135.5, 134.8, 133.1 (2), 129.3, 128.7, 126.9, 126.2, 126.0, 108.0, 62.3, 55.3, 48.0, 46.1, 42.3, 32.5, 29.1, 14.8.

### Example 13

### [4-(3-Butoxymethyl-3,4-dihydro-1H-isoquinolin-2-ylmethyl)-5-methyl-sulfanyl-2-(2,4,6-trichloro-phenyl)-2H-pyrazol-3-yl)]-dimethyl-amine

To an anhydrous tetrahydrofuran (2 ml) solution of the racemic mixture of Example 7 (0.21 g, 0.41 mmol), sodium hydride (82 mg of 60% sodium hydride mineral oil dispersion; 49 mg, 2.0 mmol of sodium hydride) was added, and the resulting mixture was stirred 10 minutes before adding n-butyliodide (0.19 ml, 307 mg, 1.7 mmol). The reaction mixture was stirred for 24 hours. Thin layer chromatography inspection of a reaction aliquot showed incomplete reaction. More sodium hydride (82 mg of 60% sodium hydride mineral oil dispersion; 49 mg, 2.0 mmol of sodium hydride), anhydrous tetrahydrofuran (0.5 ml), and n-butyliodide (0.19 ml, 307 mg, 1.7 mmol) was added; and the resulting mixture was stirred 24 hours at ambient temperature to essentially complete reaction. The solvent was removed in vacuo and the residue was dissolved in ethyl acetate/water (50 ml of each). The separated aqueous extract was extracted with three 10 ml portions of ethyl acetate. The combined organic extracts were dried (anhydrous sodium sulfate) and concentrated in vacuo to a viscous orange oil (0.3 g). Chromatography involving sequential flash and then gravity methods of elution (silica gel, 40 micron mesh; elution with ethyl acetate/hexane=1:0 in volume) afforded the title compound (150 mg), as a viscous yellow oil.

¹³C NMR (CDCl₃): 152.0, 149.9, 136,1, 135.3, 134.9, 134.7, 134.0, 129.1, 128.6, 126.4, 126.0, 125.5, 109.4, 71.1, 69.8, 56.2, 50.4, 47.9, 42.4, 31.9, 30.8, 19.4, 15.2, 13.9.

### Example 14

### Racemic [4-(3-Methoxymethyl-3,4-dihydro-1H-isoquinolin-2-ylmethyl)-5-methylsulfanyl-2-(2,4,6-trichloro-phenyl)-2H-pyrazol-3-yl]-dimethyl-amine

To a solution of the racemic mixture of Example 7 (200 mg, 0.39 mmol) in anhydrous tetrahydrofuran (2.0 ml), sodium hydride (78 mg of 60% sodium hydride mineral oil dispersion; 46 mg, 2.0 mmol of sodium hydride) was added; and the resulting mixture was stirred for 15 minutes at ambient temperature. Methyl iodide (0.10 ml, 1.6 mmol) was added, and the mixture was stirred for 18 hours at ambient temperature. The solvent was removed in vacuo, and the residue was dissolved in ethyl acetate/water (20 ml of each). The aqueous phase was separated and extracted with three equal volumes of ethyl acetate. The combined organic extracts were dried (anhydrous sodium sulfate) and concentrated in vacuo to a yellow oil. Flash chromatography of the entire sample (silica gel, 40 micron mesh; elution with ethyl acetate/hexane= 1:10 in volume) afforded the title compound (118 mg) as a light-yellow oil.

¹³C NMR (CDCl₃): 152.0, 149.8, 136.1, 135.3, 134.8, 134.6, 133.9, 129.1, 128.6, 126.5, 126.1, 125.6, 109.2, 71.8, 58.9, 56.0, 50.6, 47.6, 42.4, 30.6, 15.2.

### Example 15

### Enantiomeric [4-(3-Methoxymethyl-3,4-dihydro-1H-isoquinolin-2-ylmethyl)-5-methylsulfanyl-2-(2,4,6-trichloro-phenyl)-2H-pyrazol-3-yl]-dimethyl-amine

To a well-stirred solution of the compound of Example 8 (200 mg, 0.39 mmol) in anhydrous tetrahydrofuran (3 ml), sodium hydride (78 mg of 60% sodium hydride mineral oil dispersion, 46.8 mg, 2.0 mmol of sodium hydride) was added. The mixture was stirred for 5 minutes at ambient temperature before methyl iodide (0.097 ml, 1.6 mmol) was added. After stirring for 16 hours at ambient temperature, the solvent was removed in vacuo, and the residue was extracted in to an ethyl acetate/water (60 ml of each) mixture. The separated aqueous phase was then extracted with three equal volume portions of fresh ethyl acetate. The combined ethyl acetate extracts were dried (anhydrous sodium sulfate) and concentrated in vacuo to an orange oil (510 mg). Flash chromatography of the entire sample (silica gel, 40 micron mesh; elution with ethyl acetate/hexane=1:10 in volume) afforded the single enantiomeric title product as a light-yellow oil (50 mg, 24% yield). The TLC R_{f} data and ¹H NMR¹³C NMR spectra were identical in all respects to those obtained with the corresponding racemic product obtained by Example 14.

### Example 16

### Enantiomeric [4-(3-lsopropoxymethyl-3,4-dihydro-1H-isoquinolin-2-ylmethyl)-5-methylsulfanyl-2-(2,4,6-trichloro-phenyl)-2H-pyrazol-3-yl]-dimethyl-amine

To a well-stirred solution of the compound of Example 8 (310 mg, 0.61 mmol) in anhydrous tetrahydrofuran (3 ml), sodium hydride (140 mg of 60% sodium hydride mineral oil dispersion, 84 mg, 3.5 mmol of sodium hydride) was added. The reaction mixture was stirred at ambient temperature for 5 minutes before isopropyl iodide (0.42 ml, 4.2 mmol) was added. The reaction was then stirred at ambient temperature for 23 hours. Additional anhydrous tetrahydrofuran (1 ml) and sodium hydride (120 mg of 60% sodium hydride mineral oil dispersion, 72 mg, 3.0 mmol of sodium hydride) were added, followed 5 minutes later by a second portion of isopropyliodide (0.30 ml, 3.0 mmol). Ambient temperature stirring was continued for an additional 5 hours. The solvent was removed in vacuo, and the residue was extracted with ethyl acetate/water (60 ml of each). The separated aqueous phase was extracted twice with 30 ml portions of fresh ethyl acetate. The combined organic extracts were dried over anhydrous sodium sulfate and concentrated in vacuo to afford an orange oil (600 mg). Chromatography (silica gel, 40 micron mesh; elution with ethyl acetate/hexane=1:10 in volume) afforded the title compound (2.0 mg) as a light-yellow oil. The TLC R_{f} and NMR spectral properties of the title compound are identical in all respects to those of the racemic counterpart prepared by Example 17.

### Example 17

### Racemic [4-(3-Isopropoxymethyl-3,4-dihydro-1H-isoquinolin-2-ylmethyl)-5-methylsulfanyl-2-(2,4,6-trichloro-phenyl)-2H-pyrazol-3-yl]-dimethyl-amine

Utilizing the procedure of Example 15, the compound of Example 7 (215 mg, 0.42 mmol) in anhydrous tetrahydrofuran (2 ml) was reacted first with sodium hydride (100 mg of 60% sodium hydride mineral oil dispersion; 60 mg, 2.5 mmol of sodium hydride), and then with isopropyl iodide (0.29 ml, 497 mg, 2.9 mmol), affording the title compound (20 mg) as a yellow oil.

¹³C NMR (CDCl₃): 152.1, 149.7, 136.1, 135.3, 134.7 (2), 134.0, 129.1, 128.6, 126.4, 126.0, 125.5, 109.4, 71.9, 67.0, 56.6, 50.3, 48.0, 42.4, 30.9, 22.3, 22.1, 15.2.

### Example 18

### Racemic Dimethyl-{4-[3-(3-methyl-butoxymethyl)-3,4-dihydro-1H-isoquinolin-2-ylmethyl]-5-methylsulfanyl-2-(2,4,6-trichloro-phenyl)-2-pyrazol-3-yl)-amine

Utilizing the procedure of Example 16, the compound of Example 7 (210 mg, 0.41 mmol) in anhydrous tetrahydrofuran (2 ml) was reacted first with sodium hydride (100 mg of 60% sodium hydride mineral oil dispersion; 60 mg, 2.5 mmol of sodium hydride), and then with isopentyl iodide (570 mg, 2.9 mmol), affording the title compound (44 mg) as a yellow oil.

¹³C NMR (CDCl₃): 152.0, 149.9, 136.1, 135.3, 134.7 (2), 134.0, 129.1, 128.6, 126.4, 126.0, 125.5, 109.3, 69.8, 56.2, 50.3, 47.9, 42.4, 38.6, 30.9, 25.1, 22.7, 22.6, 15.2.

### Example 19

### Racemic {4-[3-(2-Methoxy-ethoxymethyl)-3,4-dichloro-1H-isoquinolin-2-ylmethyl]-5-methylsulfanyl-2-(2,4,6-trichloro-phenyl)-2H-pyrazol-3-yl}-dimethyl-amine

To a solution of the compound of Example 7 (64 mg, 0.12 mmol) in anhydrous tetrahydrofuran (0.5 ml), sodium hydride (17.5 mg of 60% sodium hydride mineral dispersion; 10.5 mg, 0.44 mmol of sodium hydride) was added; and the resulting mixture was stirred for 15 minutes. 1-iodo-2-methoxy-ethane (0.1 ml, 0.5 mmol) was added. After stirring for 48 hours followed by 6 hours at reflux, thin layer chromatography inspection of an aliquot showed incomplete reaction. Additional sodium hydride (33 mg of 60% mineral oil dispersion, 20 mg, 1.0 mmol of sodium hydride), 1-iodo-2-methoxyethane (100 µl, 0.5 mmol) and anhydrous tetrahydrofuran (0.8 ml) were added; and the mixture was refluxed for 18 hours to complete reaction. The solvent was removed in vacuo, and the residue was dissolved in ethyl acetate/water (20 ml of each). The separated aqueous phase was extracted with three equal volumes of ethyl acetate. The combined organic extracts were dried over anhydrous sodium sulfate and concentrated in vacuo to afford an orange gum. Flash chromatography (silica gel, 40 micron mesh; elution with ethyl acetate/hexane=1:4 in volume) afforded the title compound (23 mg) as a light-yellow oil.

¹³C NMR (CDCl₃): 152.1, 149.7, 136.3, 135.4, 134.7 (2), 134.0, 129.1, 128.6, 126.5, 126.0, 125.5, 109.4, 72.0, 70.5, 70.3, 59.1, 56.0, 50.2, 47.9, 42.4, 30.7, 15.2.

### Example 20

### Enantiomeric 2-{2-[5-Dimethylamino-3-methylsulfanyl-1-(2,4,6-trichloro-phenyl)-1H-pyrazol-4-ylmethyl]-1,2,3,4-tetrahydro-isoquinolin-3-ylmethoxy}-ethanol and the corresponding tert-butyl-dimethylsilyl ether

To a solution of the compound of Example 8 (100 mg, 0.195 mmol) in anhydrous tetrahydrofuran (0.2 ml), sodium hydride (18.4 mg of 60% sodium hydride mineral oil dispersion, 11.04 mg, 0.46 mmol of sodium hydride) was added; and the resulting mixture was stirred for 5 minutes at ambient temperature before adding 1-iodo-2-(tert-butyldimethylsilyloxy)ethane (222 mg, 0.39 mmol). The reaction was then heated at 85°C for 2.5 hours. Thin layer chromatography of a reaction aliquot indicated alkylation to be complete. The solvent was removed in vacuo and the residue was dissolved in an ethylacetate/water mixture (50 ml of each). The separated organic extract was then dried over anhydrous sodium sulfate and concentrated in vacuo to a yellow oil (285 mg). Flash chromatography purification of the entire sample (silica gel, 40 micron mesh; eluting with ethylacetate/hexane=2.5:97.5 in volume) afforded the purified silylated title compound (14 mg) as a colorless oil.

¹³C NMR (CDCl₃): ppm 152.0, 149.9, 136,1, 135.3, 134.8, 134.6,133.9, 129.1, 128.6, 126.4, 126.0, 125.5, 109.3, 72.7, 70.3, 62.7, 56.2, 50.5, 47.7, 42.4, 30.6, 25.9, 22.6, 18.4, 15.2. The entire sample of silylated title compound (14 mg, 0.021 mmol) was dissolved in tetrahydrofuran. Tetrabutylammonium fluoride (42 µl of a 1.00 M solution in tetrahydrofuran, 0.042 mmol) was added, and the resulting mixture was stirred for 1 hour at ambient temperature. Thin layer chromatography revealed the presence of a small amount of the silylated compound. An additional 4 µl of 1.0 M tetrabutylammonium fluoride in tetrahydrofuran effected complete desilylation within 30 minutes. The solvent was removed in vacuo, and the residue was dissolved in an ethylacetate/water (20 ml of each) mixture. The layers were separated, and the aqueous portion was extracted twice with 10 ml portions of fresh ethyl acetate. The combined organic extracts were dried over anhydrous sodium sulfate and concentrated to an oil (20 mg). Flash chromatography of the entire sample (silica gel, 40 micron mesh; elution with ethylacetate/hexane=1:3 in volume) afforded the first title compound (11.5 mg) as a colorless oil.

TLC R_{f} (silica gel plates; u.v. detection, ethylacetate/hexane=1:3 in volume): 0.20.

### Example 21

### 1-[5-Dimethylamino-3-methylsulfanyl-1-(2,4,6-trichloro-phenyl)-1H-pyrazol-4-yl-methyl]-naphthalen-2-ol and Dimethyl-[5-methylsulfanyl-4-(naphthalene-2-yloxymethyl)-2-(2,4,6-trichloro-phenyl)-2H-pyrazol-3-yl]-amine

Utilizing the procedure of Example 4D, the mesylate of the compound of Example 1C (3 mmol) was prepared in situ in anhydrous methylene chloride (12 ml). Separately, sodium hydride (480 mg of 60% sodium hydride mineral oil dispersion, 288 mg, 12 mmol of sodium hydride) was added portionwise over several minutes to a stirred solution of 2-napthol (1.73 g, 12 mmol) in anhydrous N,N-dimethylformamide (4 ml). After stirring at ambient temperature for 20 minutes, the sodium salt of the 2-napthol/N,N-dimethylformamide mixture was added to the above-prepared mesylate/methylene chloride solution. The resulting mixture was stirred at 50°C for 18 hours. The solvents were removed in vacuo and the residue was extracted into ethylacetate/water (60 ml of each), with the pH adjusted to 6 (1N aqueous hydrochloric acid). The separated aqueous phase was extracted with 60 ml of fresh ethyl acetate. The combined organic extracts were extracted with an equal volume of water, dried over anhydrous sodium sulfate and concentrated in vacuo to afford an amber oil (2.9 g). Flash chromatography of the entire sample (silica gel, 40 micron mesh; elution with ethylacetate/hexane=1:9 in volume) afforded the first title compound (the product of C-alkylation; 388 mg) as a colorless foam, and the second title compound (the product of O-alkylation; 46 mg) as a colorless oil.

The first title compound (product of C-alkylation): ¹³C NMR (CDCl₃): 152.9, 149.1, 148.2, 136.5 (2), 135.1, 133.2, 129.4, 128.9, 128.6, 128.5, 126.1, 123.7, 123.0, 119.5, 118.0, 113.0, 42.7, 19.6, 16.0.

The second title compound (product of O-alkylation): ¹³C NMR (CDCl₃): 156.5, 153.4, 149.7, 136.1, 135.6, 134.6, 129.4, 129.1, 128.7, 127.7, 126.8, 126.4, 123.8, 119.3, 107.3, 107.0, 60.5, 42.6, 15.7.

### Example 22

### Dimethyl-(5-methylsulfanyl-4-phenoxymethyl-2-(2,4,6-trichloro-phenyl)-2H-pyrazol-3-yl]-amine and 2-[5-Dimethylamino-3-methylsulfanyl-1-(2,4,6-trichloro-phenyl)-1H-pyrazol-4-ylmethyl]-phenol

To a solution of phenol (564 mg, 6.0 mmol) in anhydrous N,N-dimethylformamide (1.0 ml), sodium hydride (240 mg of 60% sodium hydride mineral oil dispersion; 144 mg, 6.0 mmol of sodium hydride) was added portionwise over 5 minutes. The mixture was then stirred for 15 minutes at ambient temperature. Separately-utilizing the general procedure of Example 4D, the mesylate of the compound of Example 1C (1.5 mmol) was prepared in situ in anhydrous methylene chloride (6 ml). The entire phenol sodium salt/N,N-dimethylformamide sample was added all at once to the above-prepared (well-stirred/ice-bath-chilled) mesylate solution: and the resulting mixture was heated at 50°C for 18 hours. The solvent was removed in vacuo and the resulting residue was extracted into ethylacetate/water (60 ml of each) with the pH of the aqueous phase adjusted to 6.0 (1N hydrochloric acid). The separated aqueous phase was twice extracted with 30 ml portions of fresh ethyl acetate. The organic extracts were combined, extracted with an equal volume of water, dried over anhydrous sodium sulfate, and concentrated in vacuo to a solid (900 mg). Flash chromatography (silica gel, 40 micron mesh; elution with ethylacetate/hexane=1:9 in volume) of the entire sample afforded an impure sample (90 mg) of the C-alkylated title compound, and a likewise partially-purified sample (190 mg) of O-alkylated title compound. Final purification of both compounds by separate silica gel flash chromatography of each sample (elution with hexane/methylene chloride=2:3 in volume) afforded 11 mg and 58 mg respectively, of the C-alkylated title compound and the O-alkylated title compound.

The second title compound (product of C-alkylation): ¹³C NMR (CDCl₃): 154.4, 149.4, 149.0, 136.4, 136.1, 135.0, 130.2, 128.8, 127.8, 126,1, 120.3, 116.1, 111.2, 42.7, 24.2, 15.2.

The first title compound (product of O-alkylation): ¹³C NMR (CDCl₃): 158.6, 153.1, 149.5, 136.1, 135.6, 134.5, 129.5, 128.7, 121.0, 115.1, 107.2, 60.4, 42.6, 15.7.

### Example 23

### [4-(2-Methoxy-naphthalen-1-ylmethyl)-5-methylsulfanyl-2-(2,4,6-trichloro-phenyl)-2H-pyrazol-3-yl]-dimethyl-amine

To a well-stirred solution of the first title compound of Example 21 (121 mg, 0.25 mmol) in anhydrous tetrahydrofuran (1.0 ml), sodium hydride (25 mg of 60% sodium hydride mineral oil dispersion; 15 mg, 0.63 mmol of sodium hydride) was added portionwise over 5 minutes. After stirring the mixture for 10 minutes at ambient temperature, methyl iodide (78 µl, 1.25 mmol) was added. The reaction mixture was stirred 1 hour (ambient temperature) before a second portion of methyl iodide (78 µl, 1.25 mmol) was added. The reaction was stirred for 18 hours at ambient temperature before in vacuo removal of solvent. The residue was extracted into ethylacetate/water (60 ml of each). The separated aqueous extract was extracted twice with 30 ml portions of fresh ethyl acetate. The combined organic extracts were dried over anhydrous sodium sulfate and concentrated in vacuo to afford a yellow oil (138 mg). Flash chromatography, using the entire sample (silica gel, 40 micron mesh; elution with ethyl acetate/hexane=1:9 in volume) afforded the title compound (74 mg) as a colorless amorphous solid.

¹³C NMR (CDCl₃): 154.9, 149.9, 148.4, 136.3 (2), 135.3, 133.5, 129.3, 128.5, 128.4, 128.3, 125.9, 124.2, 123.3, 121.1, 113.5, 112.7, 56.5, 41.7, 19.8, 15.2.

### Example 24

### [4-(2-lsopropoxy-naphthalen-1-ylmethyl)-5-methylsulfanyl-2-(2,4,6-trichlorophenyl)-2H-pyrazol-3-yl]-dimethyl-amine

To a well-stirred solution of the first title compound of Example 21 (120 mg, 0.24 mmol) in anhydrous tetrahydrofuran (1.0 ml), sodium hydride (29 mg of 60% sodium hydride mineral oil dispersion; 17.4 mg, 0.73 mmol of sodium hydride) was added portionwise over 5 minutes. After stirring the mixture at ambient temperature for 30 minutes, 2-iodopropane (192 µl, 1.92 mmol) was added; and the resulting mixture was stirred (ambient temperature) for 18 hours. TLC inspection of a reaction aliquot indicated incomplete reaction. A second portion of 2-iodopropane (200 µl, 2.0 mmol) was added, and the mixture was stirred for an additional 18 hours. The solvent was removed in vacuo and the residue was extracted into ethyl acetate/water (60 ml of each). The separated aqueous layer was extracted twice with 20 ml portions of fresh ethyl acetate. The combined organic extracts were dried over anhydrous sodium sulfate and concentrated in vacuo to afford a yellow oil (161 mg). Flash chromatography (silica gel, 40 micron mesh; elution with ethyl acetate/hexane=5:95 in volume) afforded the title compound (70 mg) as a colorless foam.

¹³C NMR (CDCl₃): 153.4, 150.0, 148.4, 136.3 (2), 135.2, 133.8, 129.4, 128.4, 128.2, 128.1, 125.7, 124.5, 123.3, 116.2, 112.8, 71.6, 41.6, 22.6, 20.1, 15.3.

### Example 25

### 4-[5-Dimethylamino-3-methylsulfanyl-1-(2,4,6-trichlorophenyl)-1H-pyrazol-4-yl-methyl]-4H-isoquinoline-1,3-dione

To a well-stirred suspension of homophthalimide (467 mg, 2.9 mmol) in anhydrous N,N-dimethylformamide (1.8 ml), sodium hydride (128 mg of 60% sodium hydride mineral oil dispersion, 76.8 mg, 3.2 mmol of sodium hydride) was added all at once. The resulting mixture was heated at 65°C for 20 minutes. Utilizing the method of Example 4D, the compound of Example 1C (1.9 mmol) was prepared in situ. With both mixtures chilled to 5°C, the entire sodium homophthalimide N,N-dimethylformamide sample and mesylate solution were combined. Acetonitrile (5 ml) was added, and the mixture was heated for 18 hours at 65°C. The solvents were removed in vacuo to afford a solid which was extracted into methylene chloride/water (100 ml of each), with the pH adjusted to 9.0. The separated organic phase was extracted with an equal volume of dilute aqueous sodium carbonate (pH 9); and then extracted with an equal volume of water. Anhydrous sodium sulfate drying and concentration in vacuo afforded a foam (909 mg). Crystallization of the entire sample from isopropyl alcohol (30 ml) afforded 140 mg (14.4% yield) of the title compound (colorless crystals, m.p. 186-187°C).

### Example 26

### 2-[5-Dimethylamino-3-methylsulfanyl-1-(2,4,6-trichlorophenyl)-1H-pyrazol-4-ylmethyl]-isoindole-1,3-dione

To a well-stirred ice-bath-chilled solution of phthalimide (427 mg, 2.9 mmol) in anhydrous N,N-dimethylformamide (4 ml), sodium hydride (128 mg of 60% sodium hydride mineral oil dispersion, 77 mg, 3.2 mmol of sodium hydride) was added. The resulting mixture was stirred at ambient temperature for 20 minutes. Separately, to an ice-bath-chilled solution of the compound of Example 1C (700 mg, 1.9 mmol) and triethylamine (304 µl, 2.2 mmol) in anhydrous methylene chloride (9 ml), methanesulfonyl chloride (104 µl, 2.1 mmol) was added all at once; and the resulting mixture was stirred (5°C) for 15 minutes to complete the in situ formation of the mesylate of the compound of Example 1C. While stirring the freshly prepared mesylate solution (5°C), the entire sodium phthalimide/N,N-dimethylformamide mixture (also chilled to 5°C), was added all at once; and the resulting reaction mixture was gently refluxed for 18 hours. The solvent was removed in vacuo, and the residue was extracted into methylene chloride/dilute aqueous sodium bicarbonate (pH 8; 100 ml of each). The separated organic phase was dried over magnesium sulfate and concentrated in vacuo to an oil (762 mg). Flash chromatography with the entire sample (30 g silica gel, 40 micron mesh; elution with ethyl acetate/hexane in volume ratios of 6:94, 8:92, 12:88, and 1:4, respectively for each of four successively-collected 250 ml volumes of eluent) afforded the title compound (160 mg), as a colorless amorphous solid.

¹³C NMR (CDCl₃): 167.8, 151.7, 148.6, 136.3, 135.6, 134.7, 133.9, 132.1, 128.6, 123.3, 106.4, 42.5, 31.9, 15.0.

### Example 27

### A. 5-Amino-1-(2,6-dichloro-4-trifluoromethyl-phenyl)-3-ethyl-1H-pyrazole-4-carboxylic acid methyl ether

A solution consisting of 2-cyano-3-ethyl-3-ethoxy-acrylic acid methyl ester (20.14 g, 109 mmol) and 2,6-dichloro-4-trifluoromethylphenylhydrazine (26.93 g, 109 mmol) in glacial acetic acid (42 ml) was heated at reflux for 4 hours; and then allowed to stir at ambient temperature for 18 hours. The solvent was removed in vacuo and the residual oil was extracted into 150 ml of ethyl acetate. Residual acetic was removed from the extract by mixing with aqueous saturated sodium bicarbonate. The separated ethyl acetate extract was dried (anhydrous sodium sulfate and concentrated in vacuo to a brown oil (47 g). Flash chromatography of the entire sample (silica gel; 40 micron mesh; elution with ethylacetate/hexane = 15:85 in volume) afforded the title compound (19.5 g) as a waxy solid. TLC R_{f} (silica gel plates; u.v. detection; ethylacetate/hexane = 15:85 in volume): 0.30.

¹³C NMR (CDCl₃): 165.2, 157.0, 151.5, 137.0, 127.5, 126.1 (2), 123.8, 120.4, 116.7, 93.2, 50.8, 22.1, 12.8.

### B. 1-(2,6-Dichloro-4-trifluoromethyl-phenyl)-5-dimethylamino-3-ethyl-1H-pyrazole-4-carboxylic acid methyl ester

By the general method of Example 4B, utilizing 19.4 g (52 mmol) of the compound of Step A, the title compound was prepared and isolated as an orange oil (22.73 g). TLC R_{f} (silica gel plates; u.v. detection; ethylacetate/hexane = 15:85 in volume): 0.73.

¹³C NMR (CDCl₃) 163.7, 158.6, 156.5, 136.3, 127.6, 125.8, 125.7, 124.0, 120.4, 116.8, 102.4, 51.0, 42.1, 22.7, 13.0.

### C. [1-(2,6-Dichloro-4-trifluoromethylphenyl)-5-dimethylamino-3-ethyl-1H-pyrazol-4-yl]-methanol

To a dry ice-acetone bath chilled solution of the Step B compound (9.0 g, 22.6 mmol) in anhydrous tetrahydrofuran (80 ml), a 1.0M solution of diisobutylaluminum hydride (75 ml, 75 mmol of diisobutylaluminum hydride) was added dropwise over 5 minutes. The reaction was then stirred at 5°C (icebath) for 30 minutes. The reaction mixture was quenched by addition of water (4.5 ml) and stirred for 10 minutes prior to warming to about 50°C. The solvent was removed from the now heterogeneous (gelatinous) mixture in vacuo. The residue was pulped with ethyl acetate (100 ml), and the mixture filtered through celite. The filtrate solvent was removed in vacuo. The resulting residue was pulped with 40 ml of an ethylacetate/hexane (1:9 in volume) mixture, affording a colorless solid which was filtered and dried (5.1 g). Further purification of the entire sample by three successive pulpings in 20 ml of hexane (and product isolation by filtration) afforded the title compound (3.6 g) as a colorless solid. TLC R_{f} (silica gel plates; u.v. detection; ethylacetate/hexanes = 1:4 in volume): 0.40.

¹³C NMR (CDCl₃): 156.5, 152.1, 136.5, 127.7, 125.7 (2), 124.1, 120.5, 116.9, 106.7, 61.4, 43.1, 20.6, 13.4.

### D. 8-[1-(2,6-Dichloro-4-trifluoromethylphenyl)-5-dimethylamino-3-ethyl-1H-pyrazol-4-ylmethyl]-quinolin-7-ol

By the general method of Example 21, and utilizing 7-hydroxyquinoline (237 mg, 1.63 mmol) in place of 2-napthol as the nucleophilic reactant and the in situ formed mesylate of the Step C compound as the substrate, the title compound (the product of C-alkylation, 208 mg) was prepared and isolated as a light yellow amorphous solid. TLC R_{f} (silica gel plates; u.v. detection, ethylacetate/hexane = 1:4 in volume): 0.36. HRMS m/z 509.10872 (M+1, C₂₄H₂₂N₄OCl₂F₃).

### Example 28

### Enantiomeric {2-[1-(2,6-Dichloro-4-trifluoromethylphenyl)-5-dimethylamino-3-ethyl-1H-pyrazol-4ylmethyl]-1,2,3,4-tetrahydro-isoquinolin-3yl}-methanol

By the general method of Example 4D, and utilizing (+)-3-hydroxymethyl-1,2,3,4-tetrahydroisoquinoline as the nucleophilic reactant and the compound of Example 27C (762 mg, 2.0 mmol) as the substrate, the title compound was prepared and isolated as an amorphous solid (190 mg). TLC R_{f} (silica gel plates; u.v. detection; ethylacetate/hexane=1.4 in volume): 0.16.

¹³C NMR (CDCl₃) 156.5, 151.4, 136.7, 136.6, 133.6, 133.3, 129.0, 127.8, 126.9, 126.5, 126.0, 125.7, 125.5, 124.2, 120.5, 117.0, 107.2, 61.9, 58.2, 47.8, 45.7, 42.6, 26.6, 20.7, 13.2.

### Example 29

### A. 5-Amino-3-ethyl-1-(2,4,6-trichlorophenyl)-1H-pyrazole-4-carboxylic acid methyl ester

By the general method of Example 27A, 2-cyano-3-ethyl-3-ethoxy-acrylic acid methyl ester (145 g, 0.79 mol) was reacted with 2,4,6-trichlorophenylhydrazine (167 g, 0.79 mol) to afford (following flash chromatography on 40 micron mesh silica gel; elution with ethylacetate/hexane=1:4 in volume) the title compound as an orange oil (176 g). TLC R_{f} (silica gel plates, u.v. detection; ethylacetate/hexane=1.4 in volume): 0.43.

¹H NMR (CDCl₃): 7.43 (2H, s), 5.14 (2H, broad s), 3.78 (3H, s), 2.74 (2H, q, J=7.6 Hz), 1.20 (3H, t, J=7.6 Hz).

### B. 5-Dimethylamino-3-ethyl-1-(2,4,6-trichlorophenyl)-1H-pyrazole-4-carboxylic acid methyl ester

By the general method of Example 4B, utilizing the Step A compound (4.64 g, 1.3 mmol), the title compound (2.9 g) was prepared and isolated as an orange solid. TLC R_{f} (silica gel plates; u.v. detection; ethylacetate/hexane=1:10 in volume): 0.42.

¹H NMR (CDCl₃): 7.44 (2H, s), 3.84 (3H, s), 2.84 (2H, q, J=7.6 Hz), 2.70 (6H, s), 1.23 (3H, t, J=7.6 Hz).

### C. [5-Dimethylamino-3-ethyl-1-(2,4,6-trichlorophenyl)-1H-pyrazol-4-yl]-methanol

By the general method of Example 27C and utilizing the Step B compound of this Example (1.50 g, 4.0 mmol), the title compound was prepared and isolated as a colorless waxy solid (320 mg). TLC R_{f} (silica gel plates; u.v. detection; ethylacetate/hexane=1:4 in volume): 0.16.

¹H NMR (CDCl₃) 7.36 (2H, s), 4.50 (2H, m), 2.65 (6H, s), 2.58 (2H, q, J=7.6 Hz), 1.21 (3H, t, J=7.6 Hz).

### D. Enantiomeric {2-[5-Dimethylamino-3-ethyl-1-(2,4,6-trichlorophenyl)-1H-pyrazol-4-ylmethyl]-1,2,3,4-tetrahydroisoquinolin-3-yl}-methanol

By the general method of Example 4D and utilizing the dextrorotatory enantiomer (+)-3-hydroxy-1,2,3,4-tetrahydroisoquinoline (240 mg, 1.4 mmol) as the nucleophilic reactant and the in situ formed mesylate of the Step C compound as the substrate, the title compound was prepared and isolated as a light yellow oil [12 mg of pure material following flash chromatography (silica gel; 40 micron mesh; elution with ethylacetate/hexane=1:6 in volume) of the crude product].

¹³C NMR (CDCl₃) 155.5, 151.9, 135.3, 134.9, 134.1, 133.5, 128.9, 128.6, 126.7, 126.2, 109.3, 86.8, 71.6, 58.2, 50.6, 43.1, 31.3, 20.5, 13.8. HRMS m/z 493.1345 (M+1, C₂₄H₂₈N₄OCl₃).

### Example 30

### [2-(2,6-Dichloro-4-trifluoromethylphenyl)-4-(3-ethoxymethyl-3,4-dihydro-1H-isoquinolin-2-ylmethyl)-5-methylsulfanyl-2H-pyrazol-3-yl)]-dimethylamine, dihydrochloride salt

To an ambient temperature solution of the free base form of the title compound of this Example (38 mg) in anhydrous hydrochloric acid/diethyl ether (0.5 ml), 5 drops of a saturated anhydrous hydrochloric acid/diethyl ether solution were added. Immediately, a white crystalline salt (the title compound) formed which was filtered and dried in vacuo (35 mg, m.p. 75.0-75.3°C).

### Example 31

The following compounds were prepared according to the method of the Example listed in Table 1.

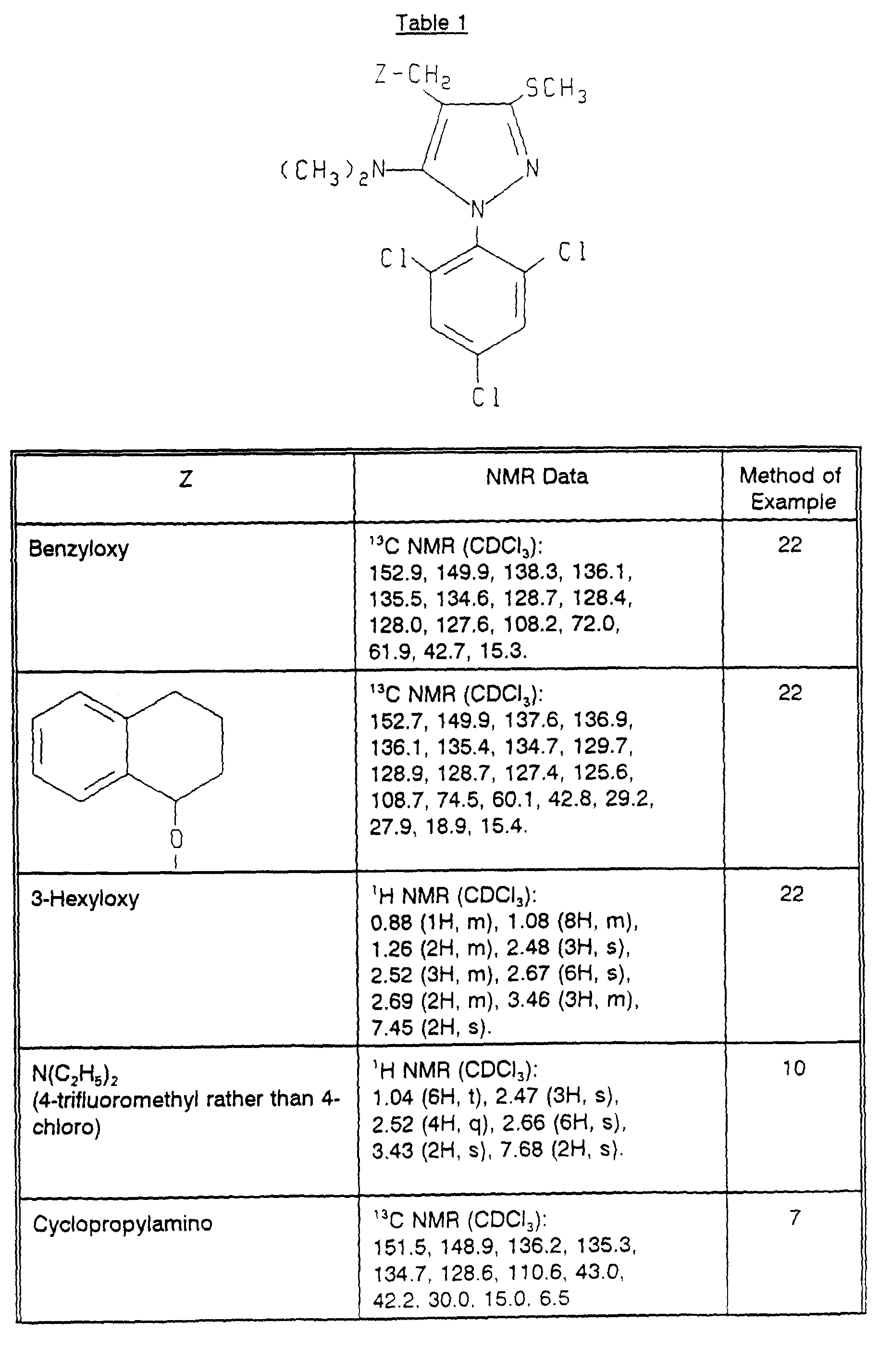

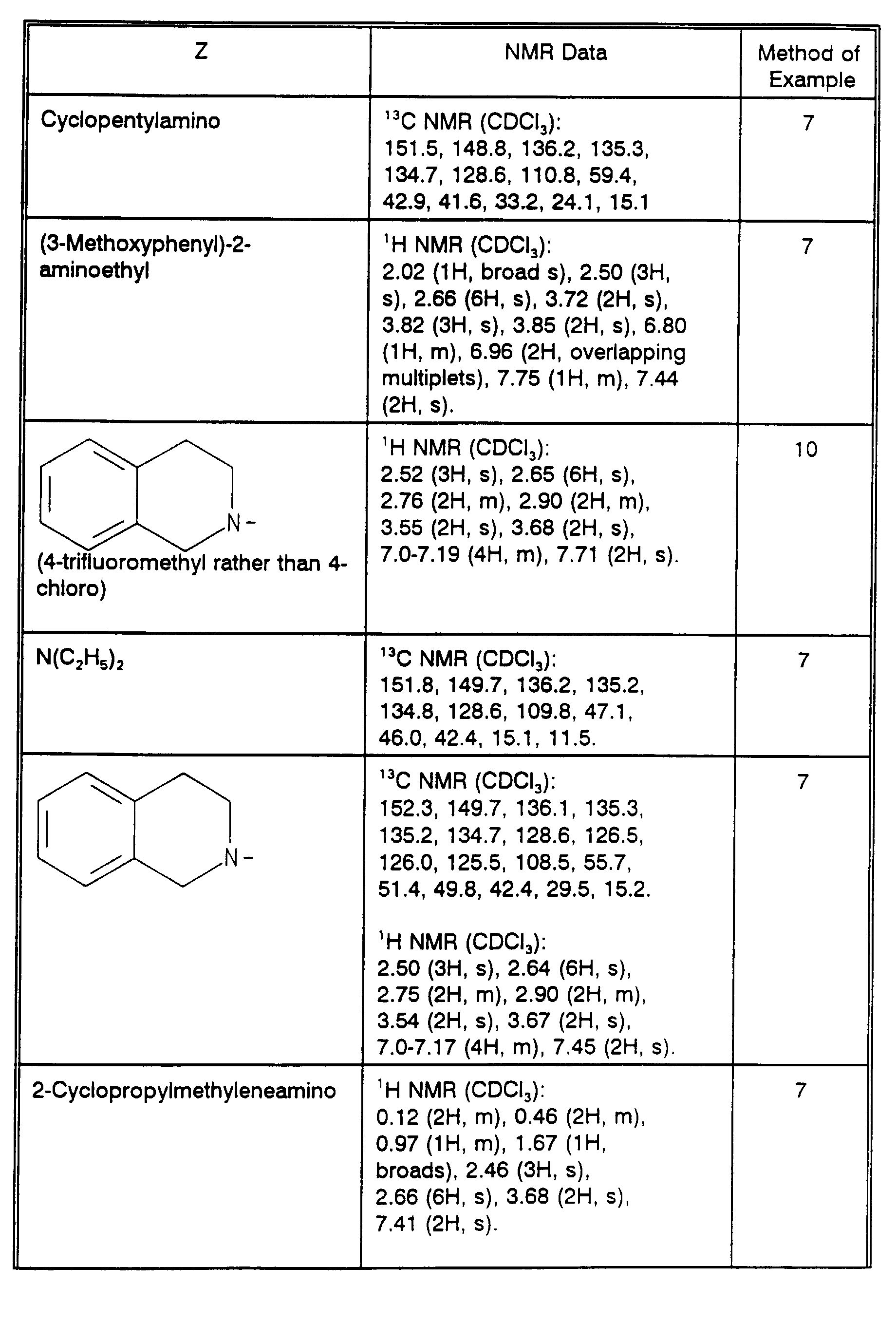

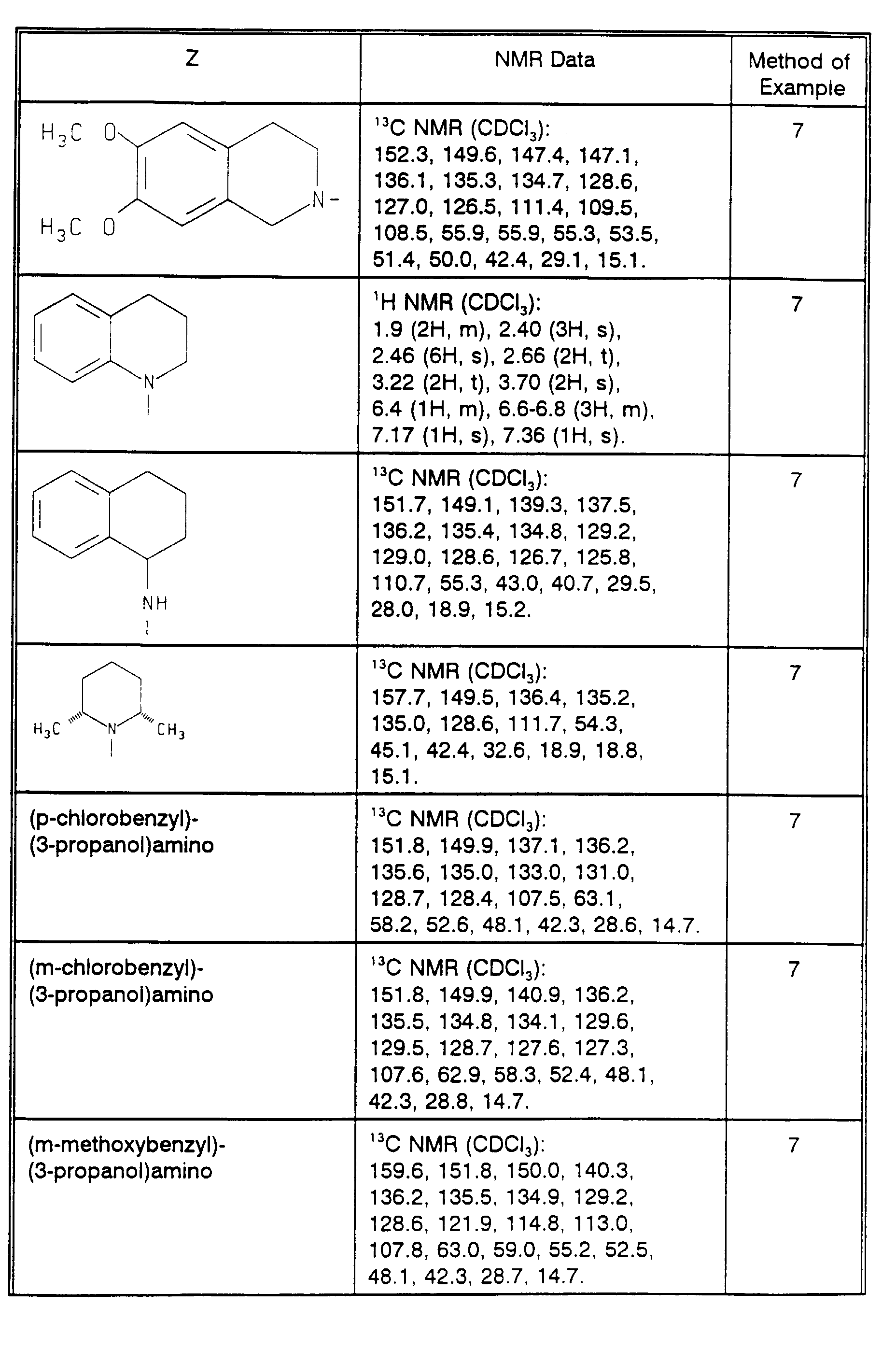

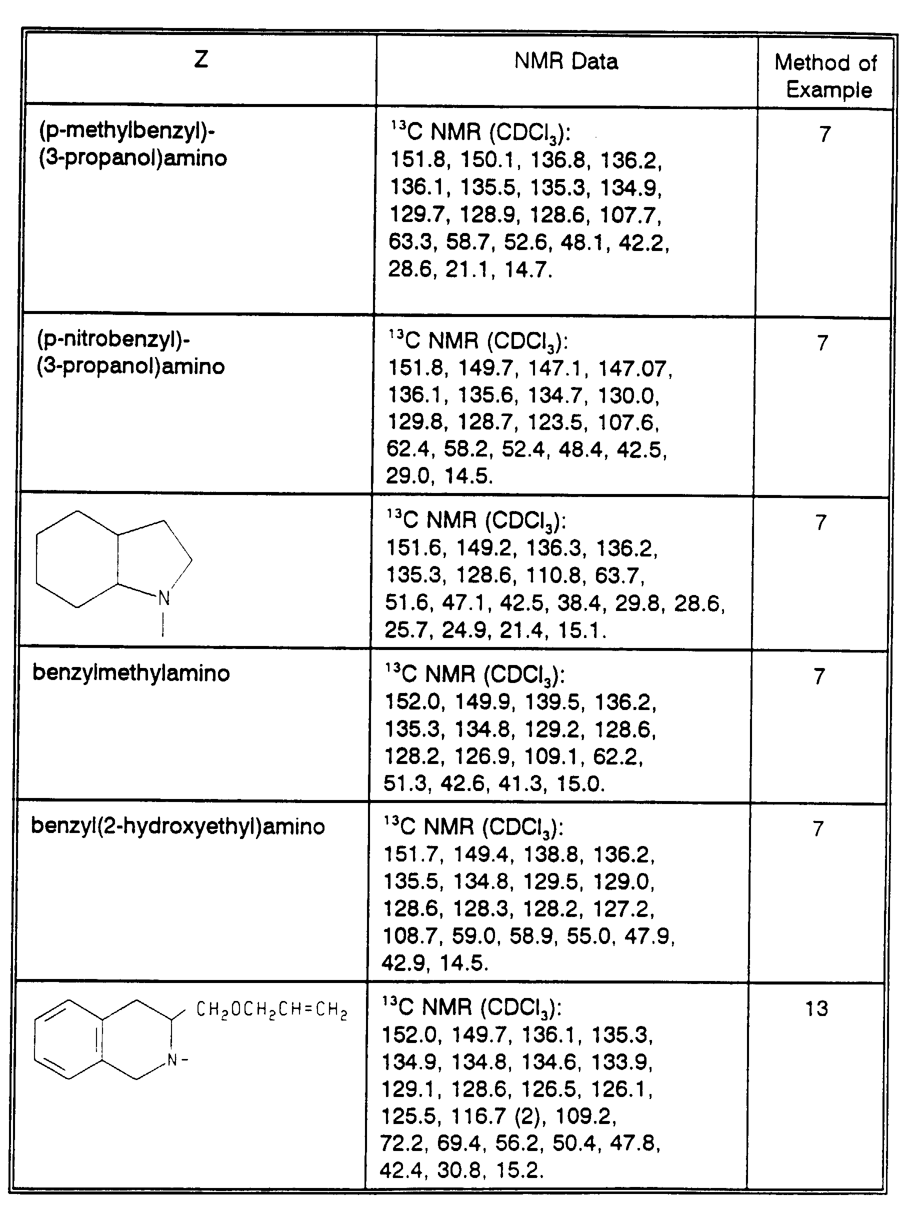

### Example 32

The following compounds were prepared according to the coupling method of Example 4D, followed by the alkylation according to the method of Example 15 for those compounds of Table 2 wherein R" is not hydrogen, and they were derived from the dextrorotary enantiomer (+)-3-substituted-1,2,3,4-tetrahydroisoquinoline prepared by the general method of Preparation 2 hereafter. The pyrazole starting material was prepared by the method of the Example listed in Table 2.

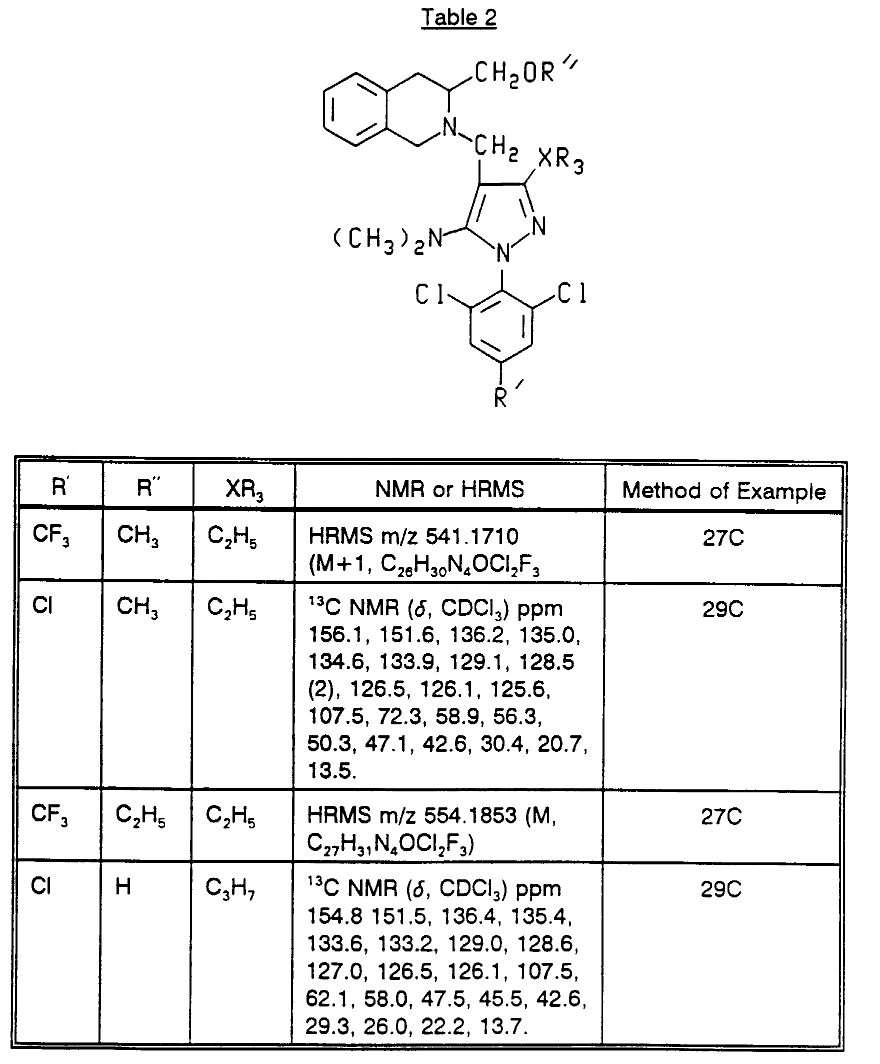

### Example 33

The following compounds were prepared according to the methods of the Examples listed in Table 3.

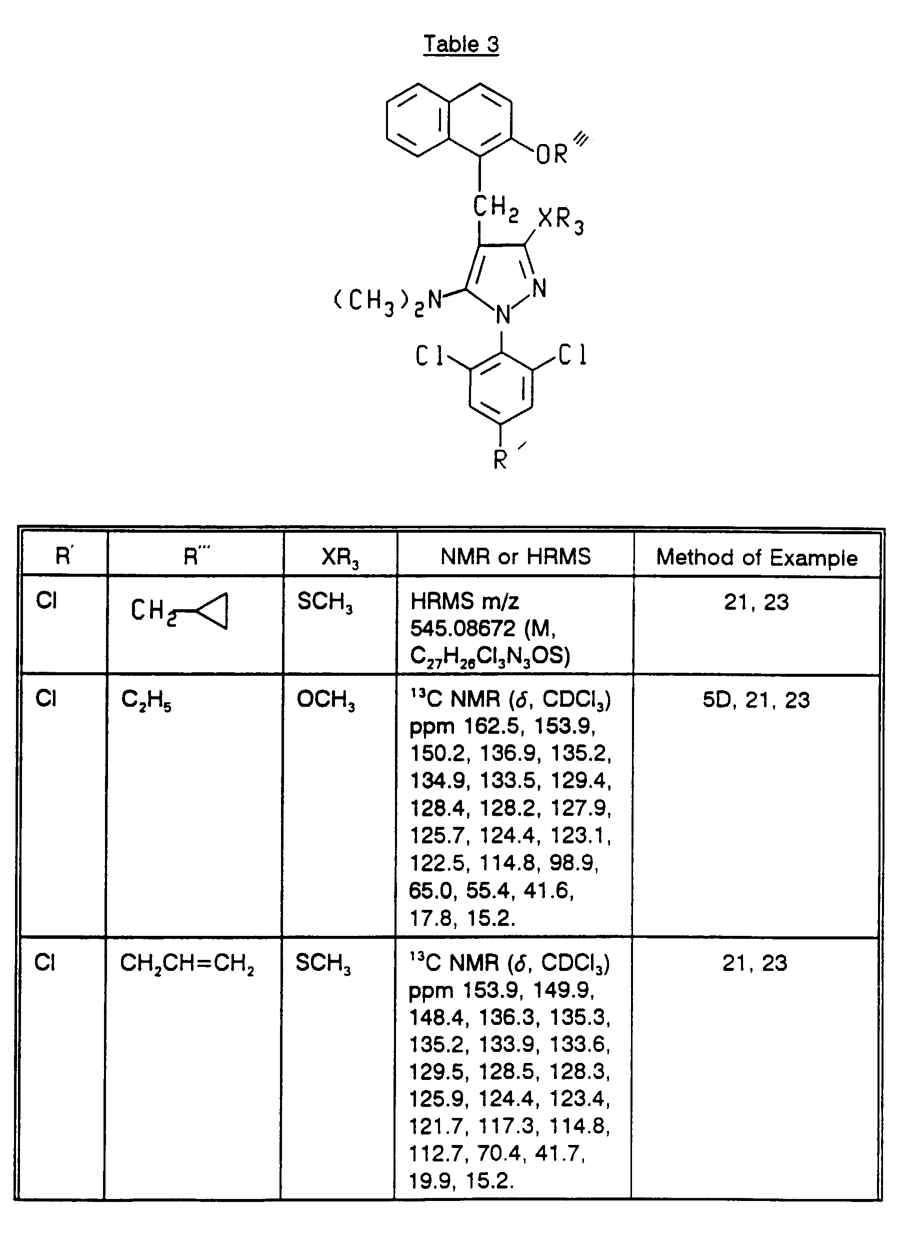

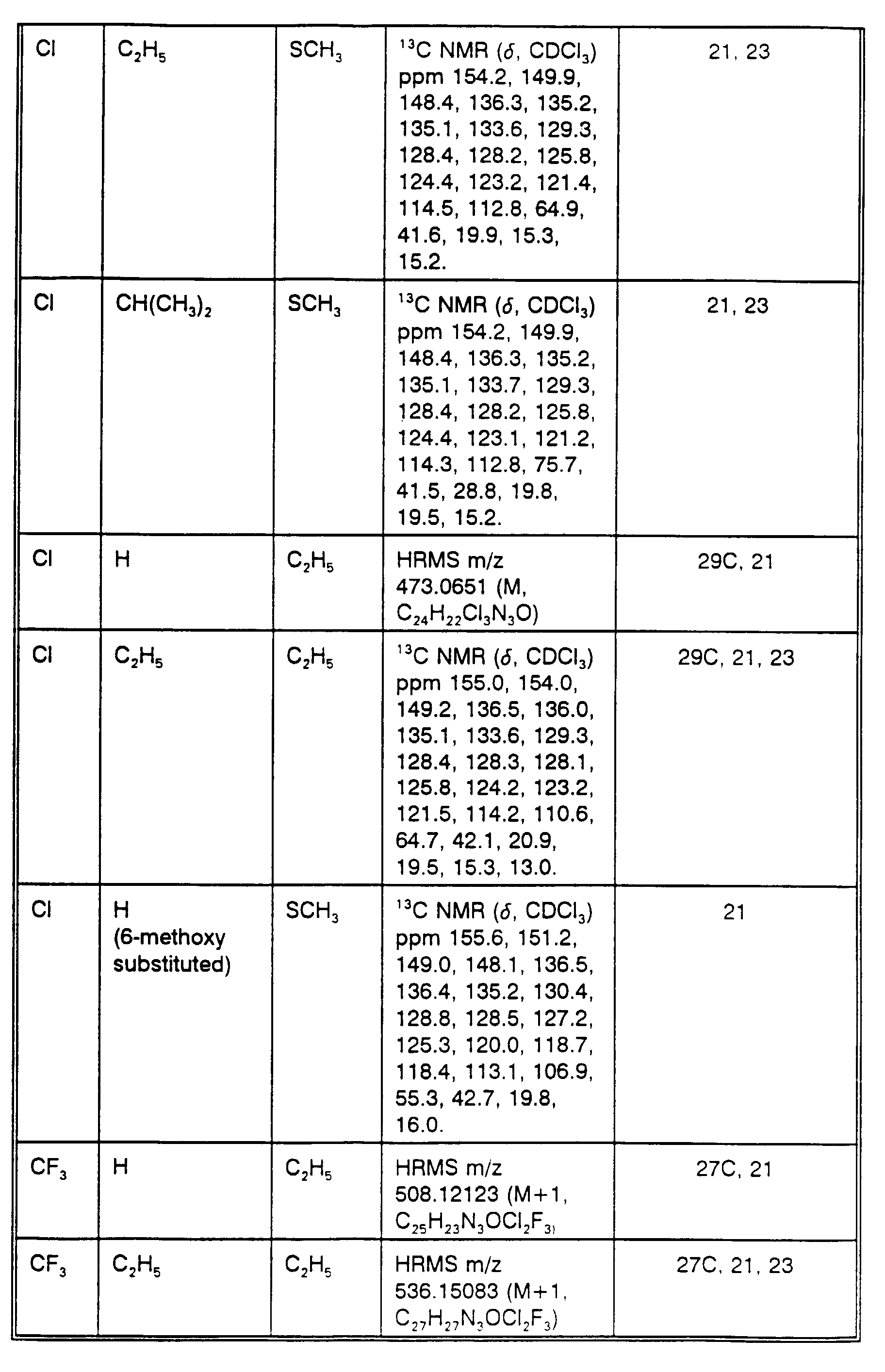

### Example 34

The following compounds were prepared according to the method of Example 23. The starting compounds of use in this method were prepared by the method of Example 21.

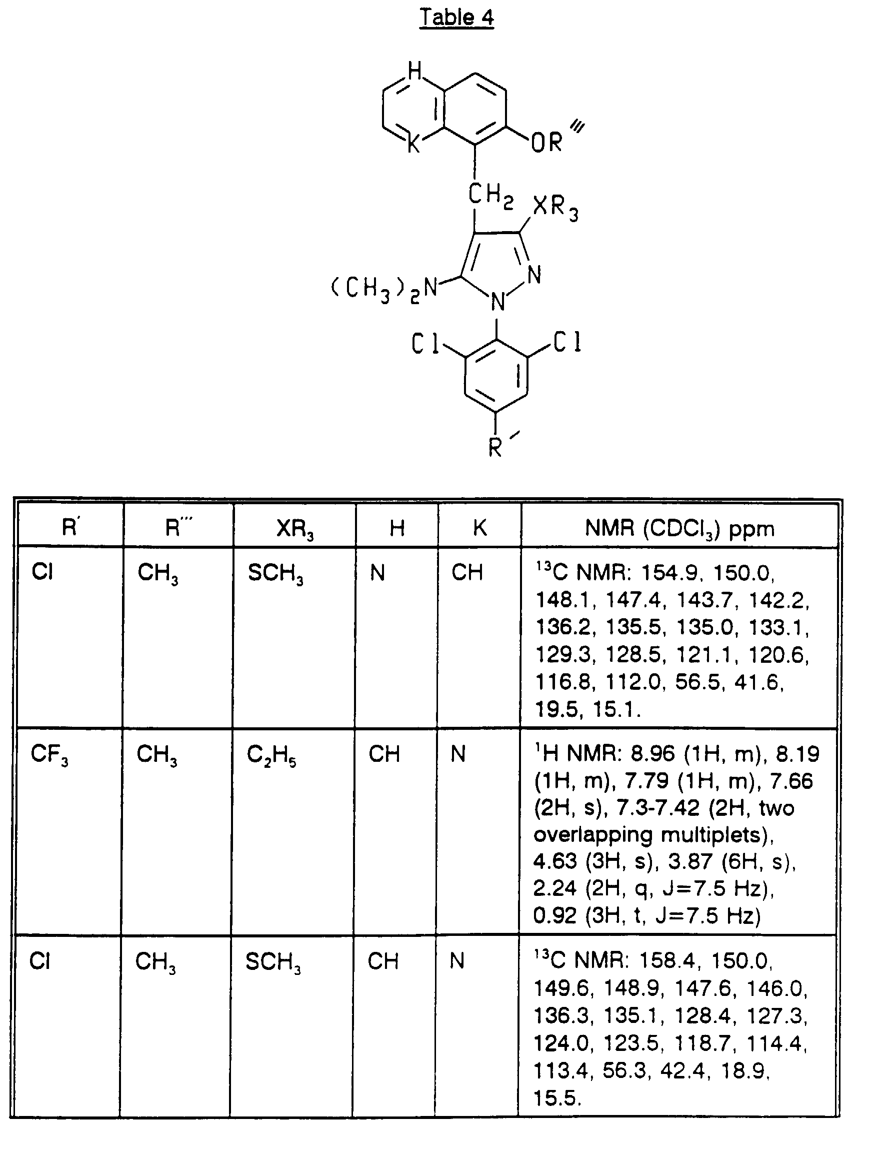

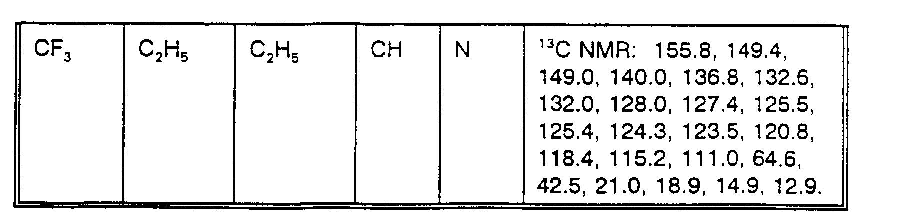

### Example 35

### A. 2-{1-[1-(2,6-Dichloro-4-trifluoromethylphenyl)-5-dimethylamino-3-ethyl-1H-pyrazol-4-ylmethyl]-napthalen-2-yloxy}-ethanol tert-butyl-dimethylsilyl ether

To a well-stirred solution of 1-[1-(2,6-Dichloro-4-trifluoromethylphenyl)-5-dimethylamino-3-ethyl-1H-pyrazol4-ylmethyl]-napthalen-2-ol (130 mg, 0.26 mmol) (listed in Table 3) in tetrahydrofuran (1.0 ml), sodium hydride (31 mg of 60% sodium hydride mineral oil dispersion; 19 mg, 0.78 mmol of sodium hydride) was added portionwise over five minutes; and five minutes thereafter 744 mg (2.6 mmol) of 1-iodo-2-(tert-butyldimethylsilyloxy)ethane was added before heating the mixture for 18 hours at 45-50°C. TLC examination showed incomplete reaction. Twice more, 744 mg (2.6 mmol) additions of 1-iodo-2-(tert-butyldimethylsilyloxy)ethane were made, each time followed by heating the reaction at 50°C for 18 hours. After removing solvent in vacuo, the residue was extracted with ethyl acetate/water (100 ml of each). The separated organic extract was dried (anhydrous sodium sulfate) and concentrated in vacuo to an oil. Flash chromatography (silica gel, 40 micron mesh; elution with ethyl acetate/hexane 5:95 in volume) afforded the title compound as an oil (53 mg). ¹H NMR (CDCl₃): 0.07 (6H, s), 0.88 (9H, s), 0.92 (3H, t, J=7.5 Hz), 2.24 (2H, q, J=7.5 Hz), 2.42 (6H, s), 3.96 (2H, t), 4.15 (2H, t), 7.16-7.32 (3H, m), 7.58 (2H, s), 7.64-7.75 (2H, m), 7.8-7.88 (1H, m).

### B. 2-{1-[1-(2,6-Dichloro-4-trifluoromethylphenyl)-5-dimethylamino-3-ethyl-1H-pyrazol-4-ylmethyl]-napthalen-2-yloxy}-ethanol

A solution of the compound of step A (50 mg, 0.075 mmol) and tetrabutylammonium fluoride (150 µl of a 1.00 M tetrahydrofurane solution, 0.15 mol) in tetrahydrofuran (0.25 ml) was stirred at ambient temperature for 2 hours. The entire sample was dissolved in ethyl acetate/water (50 ml of each). The separated organic extract was then extracted twice with equal volumes of water, dried (anhydrous sodium sulfate), and concentrated in vacuo to an oil (50 mg). Flash chromatography of the entire sample (silica gel, 40 micron mesh; elution with ethyl acetate/hexane = 3:7 in volume) afforded the title compound (27 mg) as an amorphous solid.

TLC R_{f} (silica gel plates, u.v. detection, ethyl acetate/water = 3:7 in volume): 0.34; ¹H NMR (CDCl₃): 1.00 (3H, t, J=7.5 Hz), 2.12 (1H, broad m), 2.34 (2H, q, J=7.5 Hz), 2.47 (6H, s), 3.95-4.08 (2H, m), 4.24 (2H, t), 4.33 (2H, s), 7.25-7.46 (3H, m), 7.68 (2H, s), 7.75-7.94 (2H, m), 8.00 (1H, m).

### Example 36

### 2-{8-[1-(2,6-Dichloro-4-trifluoromethyl-phenyl)-5-dimethylamino-3-ethyl-1H-pyrazol-4-ylmethyl]-quinolin-7-yloxy}-ethanol

By the method of Example 35, the compound of Example 27D (200 mg, 0.39 mmol) was converted into the title compound (36 mg, isolated as an amorphous solid). ¹HNMR(CDCl₃): 0.96 (3H, t), 2.02 (1H, broad), 2.34 (6H, s), 3.86 (2H, m), 4.13 (2H, t), 4.61 (2H, s), 7.14-7.42 (2H, overlapping multiplets), 7.61 (2H, s), 7.71 (1H, d), 8.08 (1H, dd), 8.88 (1H, m).

The following Preparations illustrate the preparation of intermediates.

### Preparation 1

### Racemic (1,2,3,4-Tetrahydro-isoquinolin-3-yl)-methanol[also referred to as(±)-3-hydroxymethyl-1,2,3,4-tetrahydroisoquinoline]

To a well stirred, ice-bath-chilled slurry of 1,2,3,4-tetrahydroisoquinoline-3-carboxylic acid hydrochloride (75 g, 0.351 mol. Aldrich Chemical Co.) in anhydrous methanol (600 ml), sodium methoxide (37.92 g, 0.702 mol) was added in small solid portions over a 10 minute period. After 30 minutes of brisk stirring, the methanol was removed and the colorless residue was dried in vacuo overnight. The entire sample was stirred in anhydrous tetrahydrofuran causing the organic portion to dissolve completely. A 1.0 M solution of lithium aluminum hydride in tetrahydrofuran (351 ml, 0.351 mol) was added in a rapid stream to the well-stirred mixture over a 20 minute period (mild exotherm). The reaction mixture was then vigorously refluxed for 2 hours. At 5°C, the reaction was quenched by cautious addition of 15% aqueous sodium hydroxide. The mixture was filtered, and the filtrate was concentrated in vacuo to a yellow solid. The entire sample was then dissolved in methylene chloride (400 ml) and filtered to remove residual inorganic salts. Solvent removal in vacuo afforded the title compound as an orange solid (47.01 g, 70% yield). TLC R_{f} (silica gel plates, u.v. detection, methanol/methylene chloride=5:95 in volume): 0.46; ¹³C NMR (CDCl₃): 135.4, 134.1, 129.3, 126.3, 126.1, 125.9, 65.4, 55.0, 47.8, 30.9.

### Preparation 2

### Dextrorotatory enantiomer of (1,2,3,4-Tetrahydro-isoquinolin-3-yl)-methanol (also referred to as (+)-3-hydroxymethyl-1,2,3,4-tetrahydroisoquinoline)

To a solution of (±)-3-hydroxymethyl-1,2,3,4-tetrahydroisoquinoline (Preparation 1; 47.01 g, 0.288 mol) in isopropyl alcohol (159 ml), a solution of (S)-(+)-mandelic acid -(43.81 g, 0.288 mol) in isopropyl alcohol (159 ml) was added. The resulting solution was allowed to stand at ambient temperature for 48 hours, during which time a heavy orange crystalline mass formed. The isolated crystalline solid (13.06 g) was dissolved in hot isopropyl alcohol (63 ml). After standing for 1 hour at ambient temperature, the newly-formed crystalline solid was isolated by filtration (8.2 g, m.p. 138°C). The recrystallization procedure was repeated twice more, using 63 ml and 60 ml volumes of isopropyl alcohol to afford 7.08 g and 6.76 g of crystalline material, respectively. (In each case, the crystallization was allowed to proceed for 2 hours at ambient temperature prior to filtration.) A 138-139°C m.p. was observed after the final crystallization. The entire sample was dissolved in methylene chloride water (300 ml and 100 ml, respectively) with the pH adjusted to 9.5 (potassium carbonate). The phases were separated, and the aqueous portion was extracted with three 50 ml portions of fresh methylene chloride. The combined organic extracts were dried (anhydrous sodium sulfate) and concentrated in vacuo to afford the optically resolved title compound as a colorless amorphous solid (2.02 g, 8.6% yield). [a]²⁰_{D} + 103° (c=1.83, CH₂Cl₂); ¹³C NMR (CDCl₃): identical to that of the racemic compound prepared in Preparation 1.

### Preparation 3

### Levorotatory enantiomer of (1,2,3,4-Tetrahydro-isoquinolin-3-yl)-methanol [also referred to as (-)-3-hydroxymethyl-1,2,3,4-tetrahydroisoquinoline]

Substituting (R)-(-)-mandelic acid for (S)-(+)-mandelic acid in the Preparation 2 procedure (and utilizing 17.9 g of the alcohol-amine prepared in Preparation 1), the levorotatory title compound (0.65 g, 7.3% yield) was obtained as a colorless amorphous solid. [a]²⁰_{D}-100.4° (CH₂Cl₂, c=1.43); ¹H NMR and ¹³C NMR (CDCl₃): identical in all respects to those observed for the racemic (Preparation 1) and dextrorotatory (Preparation 2) products.

## Claims

1. A compound of the formula 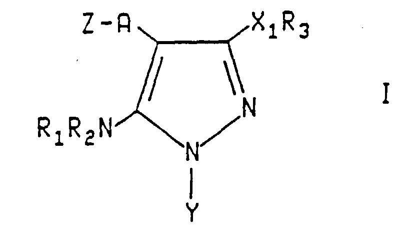 and the pharmaceutically acceptable acid addition salts thereof,
wherein
A is CH₂;
X₁ is a covalent bond, CH₂, NR, wherein R is hydrogen, linear C₁-C₆ alkyl or branched, C₃-C₈ alkyl, O, or S;
R₁, R₂ and R₃ are each independently linear C₁-C₆ alkyl, branched C₃-C₈ alkyl, C₃-C₈ alkenyl wherein the double bond is not adjacent to the N or X₁ when X₁ is oxygen or sulfur, or C₃-C₇ cycloalkyl (CH₂)ₙ wherein n is 0, 1, 2, 3 or 4; or R₁ and R₂ when taken together with the nitrogen form a saturated four, five or six membered ring optionally condensed with benzo; and R₃ may also be (CH₂)_{q}Q₁R₁₉ wherein q is 0, 1 or 2, Q₁ is O, S, NH or N(C₁-C₆ alkyl) when X₁ is a covalent bond or Q₁ is a covalent bond when X₁ is not a covalent bond, and R₁₉ is hydrogen, linear C₁-C₆ alkyl, branched C₃-C₈, C₃-C₈ alkenyl, or C₃-C₆ cycloalkyl (CH₂)ₙ wherein n is 0 to 4;
Y is phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, or piperidinyl, each of which may be substituted by one to three of any one of fluoro, chloro, bromo, or methyl, or one of trifluoromethyl; with the proviso that Y is not unsubstituted phenyl; and
Z is
(a) 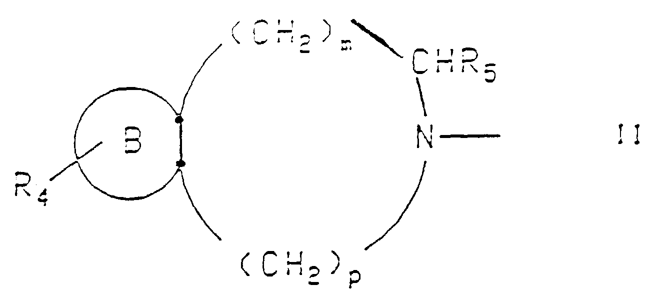 wherein the B ring is phenyl, naphthyl, pyridyl, pyrimidinyl, pyrazinyl, pyridazinyl, triazinyl, pyrrolyl, pyrazolyl, imidazolyl, triazolyl, thienyl, or indolyl, each of which may be substituted by methyl, methoxy, fluoro, chloro, bromo or iodo; or a saturated 5- or 6-membered carbocyclic ring or a partially unsaturated ring having one or two double bonds;
R₄ is hydrogen, C₁-C₆ alkyl, C₁-C₆ alkoxy, or hydroxy, fluoro, chloro, bromo, iodo, or trifluoromethyl;
R₅ is Hydrogen, linear C₁-C₆ alkyl, branched C₃-C₈ alkyl, C₃-C₈ alkenyl, or (CH₂)ₒ-X₂-(CH₂)ᵣ-Q₂-R₆;
R₆ is hydrogen, linear C₁-C₆ alkyl, branched C₃-C₈ alkyl, or C₃-C₈ alkenyl;
X₂ and Q₂ are each independently O, S, NH, N(C₁-C₆ alkyl), or one of X₂ and Q₂ may be a covalent bond;
m is 0 or 1;
o is 1 or 2;
p is 1 or 2;
r is 0, 1, or 2;
(b) 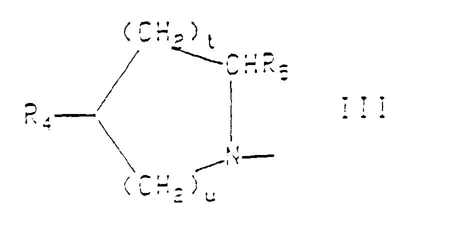 wherein R₄ and R₅ are as defined above, and t and u are each independently 1 or 2;
(c) -NR₇R₈ wherein R₇ and R₈ are each independently hydrogen, C₁-C₆ linear alkyl, branched C₃-C₈ alkyl, C₃-C₈ alkenyl, (CH₂)ᵥCH₂OH, (CH₂)ᵥNF₉R₁₀, wherein v is 0 to 3, and R₉ and R₁₀ are each independently hydrogen, or linear C₁-C₆ alkyl; C₁-C₁₂ cycloalkyl, (C₃-C₁₂ cycloalkyl) (CH₂)ₙ, (C₆-C₁₀ bicycloalkyl) (CH₂)ₙ, wherein n is 0 to 4, benzofused C₃-C₆ cycloalkyl, C₁-C₆ hydroxyalkyl, phenyl, phenyl (C₁-C₃ alkylene), each of which may be substituted by one or two of hydroxy, fluoro, chloro, bromo, C₁-C₅ alkyl, or C₁-C₅ alkoxy; or R₇ and R₈ may be taken together with the nitrogen to form a saturated or partially unsaturated 5- to 7-membered ring which may contain one of O, S, NH or N(C₁-C₆ alkyl) and which may be substituted by C₁-C₆ alkyl, hydroxy or phenyl wherein any double bond(s) are not adjacent to any heteroatoms;
(d) 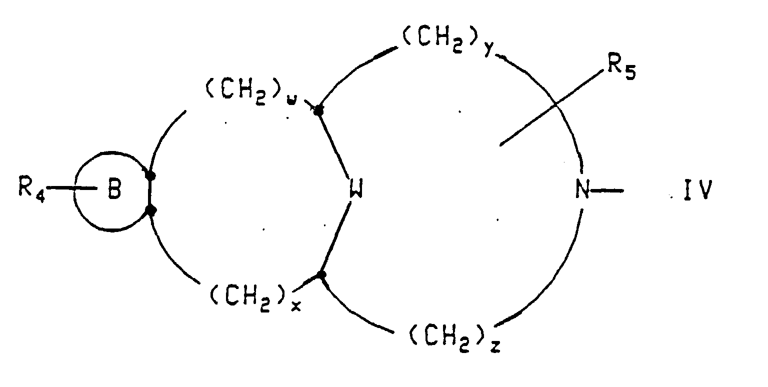 wherein B, R₄ and R₅ are as defined above, w, x, y and z are each independently 1 or 2, and W is (CH₂)_{q} wherein q is as defined above, N(C₁-C₆ alkyl), or oxygen;
(e) 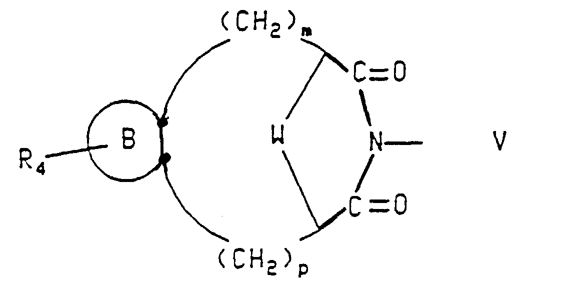 wherein W, B, R₄, m and p are as defined above;
(f) 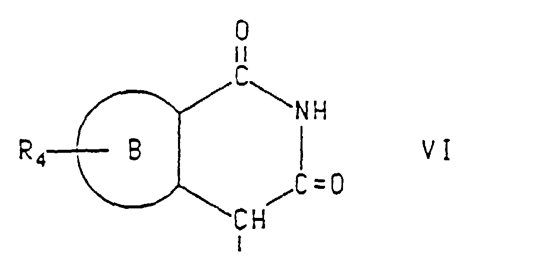 wherein B and R₄ are as defined above;
(g)
O(CH₂)ᵥR₁₁
wherein v is 0 to 3 and R₁₁ is linear C₁-C₆ alkyl, branched C₃-C₈ alkyl, phenyl, naphthyl, 1,2,3,4-tebahydronaphthyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, piperidinyl, or thienyl, each of which may be substituted by one or two of any one of fluoro, chloro, bromo, methyl, or trifluoromethyl;
(h) 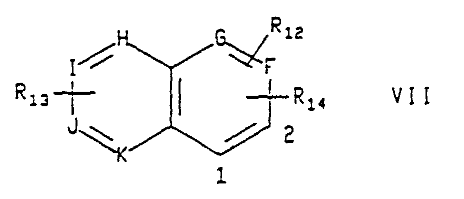 wherein the moiety of formula VII is linked via position 1 or 2 to the moiety A shown in formula I while R₁₄ is attached to position 2 or 1, respectively; F, G, H, I, J and K are independently C or N, provided that not more than three of H, I, J and K are N with not more than two adjacent nitrogens; R₁₂ and R₁₃ each independently are hydrogen, linear C₁-C₆ alkyl, branched C₃-C₆ alkyl, C₃-C₈ alkenyl, fluoro, chloro, bromo, trifluoromethyl, hydroxy, thiol, C₁-C₁₂ alkoxy, C₁-C₁₂ thioalkanyl, C₃-C₁₂ alkenoxy or C₃-C₁₂ thioalkenyl wherein the double bond is not adjacent to the oxygen or sulfur, and R₁₄ is hydroxy, C₁-C₁₂ alkoxy, C₃-C₁₂ alkenoxy wherein the double bond is not adjacent to the oxygen, or -X₂-(CH₂)ᵣQ₂R₆ wherein X₂, r, Q₂ and R₆ are as defined above in paragraph (a) except that Q₂ is not sulfur, or R₁₄ is NR₁₅R₁₆ wherein R₁₅ and R₁₆ are each independently hydrogen, linear C₁-C₆ alkyl, branched C₃-C₈ alkyl, C₃-C₈ alkenyl wherein the double bond is not adjacent to the nitrogen, or C₃-C₇ cycloalkyl-(CH₂)ₙ wherein n is as defined above, or R₁₅ and R₁₆ together with the nitrogen form a saturated five or six membered ring optionally condensed with benzo; or
(i) 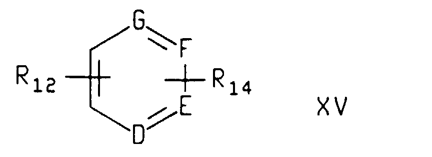 wherein D,E,F and G are independently C or N, provided that not more than two of D,E,F and G are N, and R₁₂ and R₁₄ are as defined in paragraph (h), A, defined above, is linked to a carbon in formula XV; and R₁₄ is linked to a carbon located adjacent to the carbon to which A is linked.

2. A compound according to claim 1 wherein Y is 2,4,6-tri-substituted phenyl.

3. A compound according to claim 2 wherein Y is 2,4,6-trichlorophenyl, 2,6-dichloro-4-trifluoromethylphenyl or 2,6-dibromo-4-fluorophenyl.

4. A compound according to claim 1 or 2 wherein X₁-R₃ is ethyl or methylthio.

5. A compound according to any one of claims 1 to 4 wherein R₁ and R₂ are each methyl.

6. A compound according to any one of claims 1 to 5 wherein Z is NR₇R₈ wherein R₇ is phenyl or phenyl substituted by one of fluoro, chloro, nitro, methyl or methoxy.

7. A compound according to claim 6 wherein R₈ is CH₂CH₂CH₂OH, CH₂CH₂OH, or methyl.

8. A compound according to any one of claims 1 to 5 wherein Z is 1,2,3,4-tetrahydroisoquinolin-2-yl and R₅ is (CH₂)ₒ-X₂-(CH₂)ᵣ-Q₂-R₆.

9. A compound according to claim 8 wherein R₅ is (CH₂)ₖOH wherein k is 1 to 4, or CH₂OCH₂CH₂OR₆.

10. A compound according to any one of claim 1 to 5 wherein Z is 1, 2, 3, 4-tetrahydroisoquinolin-2-yl, wherein R₅ is substituted at position 3, and the absolute configuration at the 3-position is S or R or R,S.

11. A compound according to any one of claims 1 to 5 wherein Z is of the formula 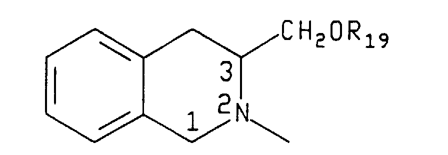 with the absolute configuration at position 3 determined by its derivation from (+)-3-hydroxymethyl-1,2,3,4-tetrahydroisoquinoline, wherein R₁₉ is methyl, ethyl, isopropyl, cyclopropylmethylene, or hydroxyethylene.

12. A compound according to any one of claims 1 to 5 wherein Z is as defined in (h).

13. A compound according to claim 12 wherein A is linked to position 1, R₁₄ is at position 2 and is X₂ - (CH₂)ᵣQ₂R₆.

14. A compound according to claim 13 wherein F, G, H, I, and J, are each carbon, K is carbon or nitrogen, and R₁₄ is 2-methoxy, 2-ethoxy, 2-isopropoxy, or 2-cyclopropylmethoxy.

15. A compound according to any one of claims 1 to 5 wherein Z is 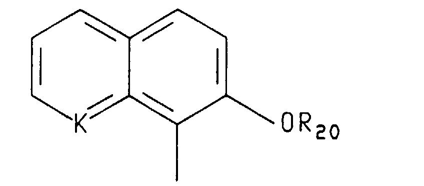 wherein K is C or N and R₂₀ is methyl, ethyl, isopropyl, cyclopropylmethylene, or hydroxyethylene.

16. A compound according to any one of claims 1 to 5 wherein Z is as defined in (a), B is phenyl, p and m are each 1, and R₅ is CH₂OCH₃.

17. A compound according to any one of claims 1 to 5 wherein Z is 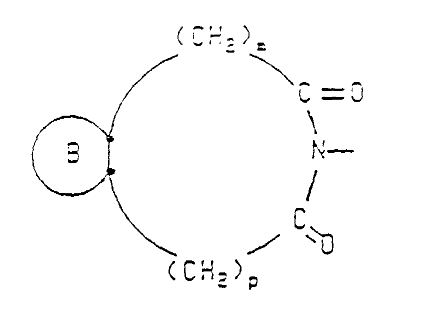 wherein B is phenyl, m is 0, and p is 1.

18. A compound according to claim 1 wherein said compound is
2-{1-[1-(2,6-dichloro-4-trifluoromethylphenyl)-5-dimethylamino-3-ethyl-1H-pyrazol-4-ylmethyl]-napthalen-2-yloxy}-ethanol;
enantiomeric [4-(3-methoxymethyl-3,4-dihydro-1H-isoquinolin-2-ylmethyl)-5-methylsulfanyl-2-(2,4,6-trichlorophenyl)-2H-pyrazol-5-yl]-dimethylamine derived from(+)-3-hydroxymethyl-1,2,3,4-tetrahydroisoquinoline;
enantiomeric [2-(2,6-dichloro-4-trifluoromethylphenyl)-4-(3-ethoxymethyl-3,4-dihydro-1H-isoquinolin-2-ylmethyl)-5-ethyl-2H-pyrazol-3-yl]-dimethylamine derived from (+)-3-hydroxymethyl-1,2,3,4-tetrahydroisoquinoline;
[2-(2,6-dichloro-4-trifluoromethylphenyl)-5-ethyl-4-(7-methoxyquinolin-8-ylmethyl)-2H-pyrazol-3-yl]-dimethylamine;
[2-(2,6-dichloro-4-trifluoromethylphenyl)-4-(2-ethoxy-naphthalen-1-ylmethyl)-5-ethyl-2H-pyrazol-3-yl]-dimethylamine;
[4-(2-ethoxynaphthalen-1-ylmethyl)-5-ethyl-2-(2,4,6-trichlorophenyl)-2H-pyrazol-3-yl]-dimethylamine;
[4-(7-methoxyquinolin-8-ylmethyl)-5-methylsulfanyl-2-(2,4,6-trichlorophenyl)-2H-pyrazol-3-yl]-dimethylamine;
2-{1-[5-dimethylamino-3-methylsulfanyl-1-(2,4,6-trichlorophenyl)-1H-pyrazol-4-ylmethyl]-naphthalen-2-yloxy}-ethanol;
enantiomeric [2-(2,6-dichloro-4-trifluoromethylphenyl)-5-ethyl-4-(3-methoxymethyl-3,4-dihydro-1H-isoquinolin-2-ylmethyl)-2H-pyrazol-3-yl]-dimethylamine derived from (+)-3-hydroxymethyl-1,2,3,4-tetrahydroisoquinoline;
[4-(2-cyclopropylmethoxynaphthalen-1-ylmethyl)-5-methylsulfanyl-2-(2,4,6-trichlorophenyl)-2H-pyrazol-3-yl]-dimethylamine.

19. A composition for the treatment of (a) ilinesses induced or facilitated by corticotropin releasing factor or (b) inflammatory disorders, stress and anxiety related disorders, including stress-induced depression and headache, abdominal bowel syndrome, immune suppression, HIV infections, Alzheimer's disease, gastrointestinal diseases, anorexia nervosa, hemorrhagic stress, drug and alcohol withdrawal symptoms, drug addiction, and fertility problems, which comprises a compound of the formula I as defined in any one of claims 1 to 18 and a pharmaceutically acceptable carrier.

20. A compound of the formula 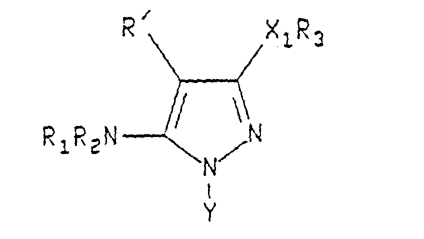 wherein R' is CH₂OH or C(O)O(C₁-C₃ alkyl), R₁, R₂ and R₃ are each independently linear C₁-C₆ alkyl, branched C₃-C₈ alkyl, C₃-C₈ alkenyl wherein the double bond is not adjacent to the N or X₁ when X₁ is oxygen or sulfur, C₃-C₇ cycloalkyl (CH₂)ₙ wherein n is 0, 1, 2, 3 or 4; or R₁ and R₂ when taken together with the nitrogen form a saturated four, five or six membered ring optionally condensed with benzo;
X₁ is a covalent bond, CH₂NR, wherein R is hydrogen or linear C₁-C₆ alkyl, O, or S; and
Y is phenyl, thienyl, benzothienyl, pyridyl, quinolyl, pyrazinolyl, pyrimidyl, imidazolyl, benzimidazolyl, furanyl, benzofuranyl, thiazolyl, benzothiazolyl, isothiazolyl, benzisothiazolyl, isoxazolyl, benzisoxazolyl, triazolyl, pyrazolyl, pyrrolyl, indolyl, azaindolyl, oxazolyl, benzoxazolyl, pyrrolidinyl, thiazolidinyl, morpholinyl, or piperidinyl, each of which may be substituted by one to three of any one of fluoro, chloro, bromo, or methyl, or one of tifluoromethyl; with the proviso that Y is not unsubstituted phenyl.

21. A compound as claimed in any of claims 1 to 18 for use as a medicament.

22. The use of a compound as claimed in any of claims 1 to 18 for the manufacture of a medicament for the treatment of (a) ilinesses induced or facilitated by corticotropin releasing factor, or (b) stress and anxiety related disorders, including stress-induced depression and headache, abdominal bowel syndrome, inflammatory disorders, immune supression, HIV infections, Alzheimer's disease, gastrointestinal diseases, anorexia nervosa, hemorrhagic stress, drug and alcohol withdrawal symptoms, drug addiction, and fertility problems, particularly depression.

## Patentansprüche

1. Verbindung der Formel 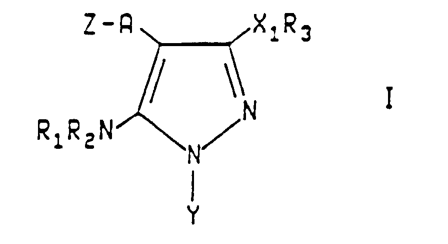 und die pharmazeutisch verträglichen Säureadditionssalze davon,
worin
A CH₂ darstellt;
X₁ eine kovalente Bindung, CH₂, NR, worin R Wasserstoff, lineares C₁-C₆-Alkyl oder verzweigtes C₃-C₈-Alkyl darstellt, O oder S bedeutet;
R₁, R₂ und R₃ jeweils unabhängig voneinander lineares C₁-C₆-Alkyl, verzweigtes C₃-C₈-Alkyl, C₃-C₈-Alkenyl, worin die Doppelbindung zu dem N oder X₁ nicht benachbart ist, wenn X₁ Sauerstoff oder Schwefel darstellt, oder C₃-C₇-Cycloalkyl-(CH₂)ₙ, worin n 0, 1, 2, 3 oder 4 ist, darstellen; oder R₁ und R₂, wenn zusammengenommen mit dem Stickstoffatom einen gesättigten vier-, fünf- oder sechsgliedrigen Ring, gegebenenfalls kondensiert mit Benzo, bilden und R₃ ebenfalls (CH₂)_{q}Q₁R₁₉ sein kann, worin q 0, 1 oder 2 ist, Q₁ O, S, NH oder N(C₁-C₆-Alkyl) bedeutet, wenn X₁ eine kovalente Bindung darstellt, oder Q₁ eine kovalente Bindung darstellt, wenn X₁ keine kovalente Bindung darstellt, und R₁₉ Wasserstoff, lineares C₁-C₆-Alkyl, verzweigtes C₃-C₈, C₃-C₈-Alkenyl oder C₃-C₆-Cycloalkyl-(CH₂)ₙ, worin n 0 bis 4 ist, darstellt;
Y Phenyl, Thienyl, Benzothienyl, Pyridyl, Chinolyl, Pyrazinolyl, Pyrimidyl, Imidazolyl, Benzimidazolyl, Furanyl, Benzofuranyl, Thiazolyl, Benzothiazolyl, Isothiazolyl, Benzisothiazolyl, Isoxazolyl, Benzisoxazolyl, Triazolyl, Pyrazolyl, Pyrrolyl, Indolyl, Azaindolyl, Oxazolyl, Benzoxazolyl, Pyrrolidinyl, Thiazolidinyl, Morpholinyl oder Piperidinyl darstellt, wobei jeder davon mit einem bis drei von Fluor, Chlor, Brom oder Methyl oder einem von Trifluormethyl substituiert sein kann, darstellt, mit der Maßgabe, daß Y nicht unsubstituiertes Phenyl darstellt;
und
Z bedeutet
(a) 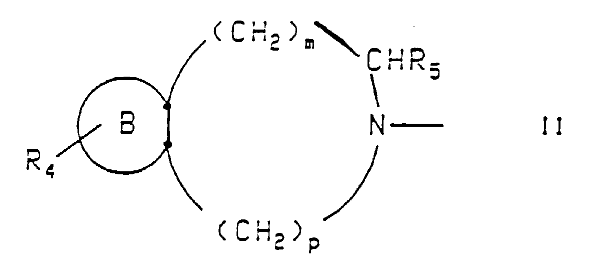 worin der B-Ring Phenyl, Naphthyl, Pyridyl, Pyrimidinyl, Pyrazinyl, Pyridazinyl, Triazinyl, Pyrrolyl, Pyrazolyl, Imidazolyl, Triazolyl, Thienyl oder Indolyl, wobei jeder davon mit Methyl, Methoxy, Fluor, Chlor, Brom oder Jod substituiert sein kann; oder einen gesättigten 5- oder 6-gliedrigen carbocyclischen Ring oder einen teilweise ungesättigten Ring mit einer oder zwei Doppelbindungen darstellt;
R₄ Wasserstoff, C₁-C₆-Alkyl, C₁-C₆-Alkoxy oder Hydroxy, Fluor, Chlor, Brom, Jod oder Trifluormethyl darstellt;
R₅ Wasserstoff, lineares C₁-C₆-Alkyl, verzweigtes C₃-C₈-Alkyl, C₃-C₈-Alkenyl oder (CH₂)ₒ-X₂-(CH₂)ᵣ-Q₂-R₆ darstellt;
R₆ Wasserstoff, lineares C₁-C₆-Alkyl, verzweigtes C₃-C₈-Alkyl oder C₃-C₈-Alkenyl darstellt;
X₂ und Q₂ jeweils unabhängig O, S, NH, N(C₁-C₆-Alkyl) darstellen oder einer von X₂ und Q eine kovalente Bindung sein kann;
m 0 oder 1 ist;
o 1 oder 2 ist;
p 1 oder 2 ist;
r 0, 1 oder 2 ist;
(b) 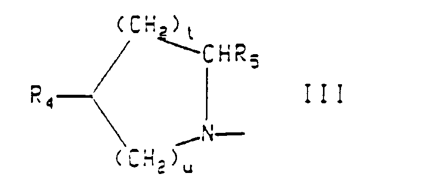 worin R₄ und R₅ wie vorstehend definiert sind und t und u jeweils unabhängig 1 oder 2 sind;
(c) -NR₇R₈, worin R₇ und R₈ jeweils unabhängig Wasserstoff, lineares C₁-C₆-Alkyl, verzweigtes C₃-C₈-Alkyl, C₃-C₈-Alkenyl, (CH₂)ᵥCH₂OH, (CH₂)ᵥNR₉R₁₀, worin v 0 bis 3 ist und R₉ und R₁₀ jeweils unabhängig Wasserstoff oder lineares C₁-C₆-Alkyl darstellen; C₁-C₁₂-Cycloalkyl, (C₃-C₁₂-Cycloalkyl)(CH₂)ₙ, (C₆-C₁₀-Bicycloalkyl) (CH₂)ₙ, worin n 0 bis 4 ist, benzokondensiertes C₃-C₆-Cycloalkyl, C₁-C₆-Hydroxyalkyl, Phenyl, Phenyl(C₁-C₃-alkylen) darstellen, wobei jeder davon mit einem oder zwei von Hydroxy, Fluor, Chlor, Brom, C₁-C₅-Alkyl oder C₁-C₅-Alkoxy substituiert sein kann; oder R₇ und R₈ zusammengenommen mit dem Stickstoffatom einen gesättigten oder teilweise ungesättigten 5- bis 7-gliedrigen Ring bilden, der eines von O, S, NH oder N(C₁-C₆-Alkyl) enthalten kann und der mit C₁-C₆-Alkyl, Hydroxy oder Phenyl substituiert sein kann, worin Doppelbindung(en) nicht zu Heteroatomen benachbart sind;
(d) 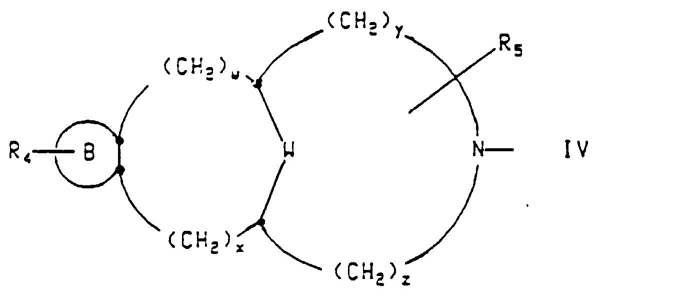 worin B, R₄ und R₅ wie vorstehend definiert sind, w, x, y und z jeweils unabhängig 1 oder 2 sind und W (CH₂)_{q}, worin q wie vorstehend definiert ist, N(C₁-C₆-Alkyl) oder Sauerstoff darstellt;
(e) 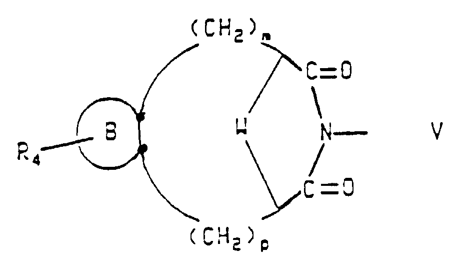 worin W, B, R₄, m und p wie vorstehend definiert sind;
(f) 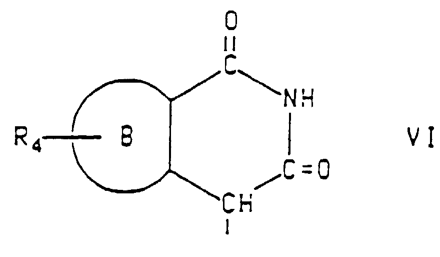 worin B und R₄ wie vorstehend definiert sind;
(g)
O(CH₂)ᵥR₁₁,
worin v 0 bis 3 ist und R₁₁ lineares C₁-C₆-Alkyl, verzweigtes C₃-C₈-Alkyl, Phenyl, Naphthyl, 1,2,3,4-Tetrahydronaphthyl, Thienyl, Benzothienyl, Pyridyl, Chinolyl, Pyrazinolyl, Pyrimidyl, Imidazolyl, Benzimidazolyl, Furanyl, Benzofuranyl, Thiazolyl, Benzothiazolyl, Isothiazolyl, Benzisothiazolyl, Isoxazolyl, Benzisoxazolyl, Triazolyl, Pyrazolyl, Pyrrolyl, Indolyl, Azaindolyl, Oxazolyl, Benzoxazolyl, Pyrrolidinyl, Thiazolidinyl, Morpholinyl, Piperidinyl oder Thienyl darstellt, wobei jeder davon mit einem oder zwei von einem von Fluor, Chlor, Brom, Methyl oder Trifluormethyl substituiert sein kann;
(h) 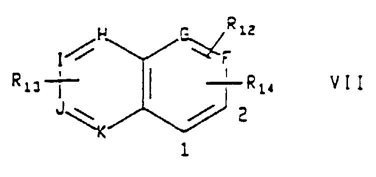 worin der Rest der Formel VII über Stellung 1 oder 2 mit dem in Formel I gezeigten Rest A verbunden ist, während R₁₄ an Stellung 2 bzw. 1 gebunden ist; F, G, H, I, J und K unabhängig C oder N darstellen, mit der Maßgabe, daß nicht mehr als drei von H, I, J und K N mit nicht mehr als zwei benachbarten Stickstoffatomen sind; R₁₂ und R₁₃ jeweils unabhängig Wasserstoff, lineares C₁-C₆-Alkyl, verzweigtes C₃-C₈-Alkyl, C₃-C₈-Alkenyl, Fluor, Chlor, Brom, Trifluormethyl, Hydroxy, Thiol, C₁-C₁₂-Alkoxy, C₁-C₁₂-Thioalkanyl, C₃-C₁₂-Alkenoxy oder C₃-C₁₂-Thioalkenyl darstellen, worin die Doppelbindung zu dem Sauerstoff oder Schwefel nicht benachbart ist, und R₁₄ Hydroxy, C₁-C₁₂-Alkoxy, C₃-C₁₂-Alkenoxy, worin die Doppelbindung zu dem Sauerstoff nicht benachbart ist oder -X₂-(CH₂)ᵣQ₂R₆, worin X₂, r, Q₂ und R₆ wie vorstehend in Absatz (a) definiert sind, mit der Ausnahme, daß Q₂ nicht Schwefel darstellt, bedeutet, oder R₁₄ NR₁₅R₁₆ bedeutet, worin R₁₅ und R₁₆ jeweils unabhängig Wasserstoff, lineares C₁-C₆-Alkyl, verzweigtes C₃-C₈-Alkyl, C₃-C₈-Alkenyl, worin die Doppelbindung zu dem Stickstoff nicht benachbart ist, oder C₃-C₇-Cycloalkyl-(CH₂)ₙ, worin n wie vorstehend definiert ist, darstellen, oder R₁₅ und R₁₆ zusammen mit dem Stickstoff einen gesättigten fünf- oder sechsgliedrigen Ring, gegebenenfalls kondensiert mit Benzo, bilden; oder
(i) 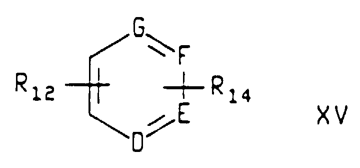 worin D, E, F und G unabhängig C oder N darstellen, mit der Maßgabe, daß nicht mehr als zwei von D, E, F und G N darstellen und R₁₂ und R₁₄ wie in Absatz (h) definiert sind, A wie vorstehend definiert, an ein Kohlenstoffatom in Formel XV gebunden ist; und R₁₄ an ein Kohlenstoffatom, das zu dem Kohlenstoffatom, an das A gebunden ist, benachbart ist, gebunden ist.

2. Verbindung nach Anspruch 1, worin Y 2,4,6-trisubstituiertes Phenyl darstellt.

3. Verbindung nach Anspruch 2, worin Y 2,4,6-Trichlorphenyl, 2,6-Dichlor-4-trifluormethylphenyl oder 2,6-Dibrom-4-fluorphenyl darstellt.

4. Verbindung nach Anspruch 1 oder 2, worin X₁-R₃ Ethyl oder Methylthio darstellt.

5. Verbindung nach einem der Ansprüche 1 bis 4, worin R₁ und R₂ jeweils Methyl darstellen.

6. Verbindung nach einem der Ansprüche 1 bis 5, worin Z NR₇R₈ bedeutet, worin R₇ Phenyl oder Phenyl, substituiert mit einem von Fluor, Chlor, Nitro, Methyl oder Methoxy, darstellt.

7. Verbindung nach Anspruch 6, worin R₈ CH₂CH₂CH₂OH, CH₂CH₂OH oder Methyl darstellt.

8. Verbindung nach einem der Ansprüche 1 bis 5, worin Z 1,2,3,4-Tetrahydroisochinolin-2-yl darstellt und R₅ (CH₂)ₒ-X₂-(CH₂)ᵣ-Q₂-R₆ darstellt.

9. Verbindung nach Anspruch 8, worin R₅ (CH₂)ₖOH, worin k 1 oder 4 ist, oder CH₂OCH₂CH₂OR₆ darstellt.

10. Verbindung nach einem der Ansprüche 1 bis 5, worin Z 1,2,3,4-Tetrahydroisochinolin-2-yl, worin R₅ in Stellung 3 substituiert ist, und die absolute Konfiguration in der 3-Stellung S oder R oder R,S ist, darstellt.

11. Verbindung nach einem der Ansprüche 1 bis 5, worin Z die Formel 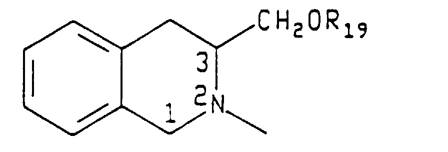 aufweist, mit der absoluten Konfiguration in Stellung 3, bestimmt durch ihre Derivatisierung mit (+)-3-Hydroxymethyl-1,2,3,4-tetrahydroisochinolin, worin R₁₉ Methyl, Ethyl, Isopropyl, Cyclopropylmethylen oder Hydroxyethylen darstellt.

12. Verbindung nach einem der Ansprüche 1 bis 5, worin Z wie in (h) definiert ist.

13. Verbindung nach Anspruch 12, worin A in Stellung 1 gebunden ist, R₁₄ in Stellung 2 ist und X₂ -(CH₂)ᵣQ₂R₆ darstellt.

14. Verbindung nach Anspruch 13, worin F, G, H, I und J jeweils Kohlenstoff darstellen, K Kohlenstoff oder Stickstoff darstellt und R₁₄ 2-Methoxy, 2-Ethoxy, 2-Isopropoxy oder 2-Cyclopropylmethoxy darstellt.

15. Verbindung nach einem der Ansprüche 1 bis 5, worin Z 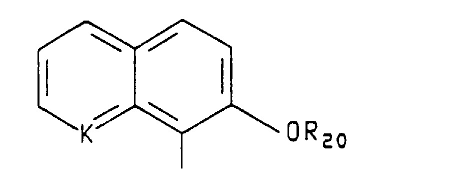 worin K C oder N darstellt und R₂₀ Methyl, Ethyl, Isopropyl, Cyclopropylmethylen oder Hydroxyethylen darstellt, bedeutet.

16. Verbindung nach einem der Ansprüche 1 bis 5, worin Z wie in (a) definiert ist, B Phenyl darstellt, p und m jeweils 1 sind und R₅ CH₂OCH₃ darstellt.

17. Verbindung nach einem der Ansprüche 1 bis 5, worin Z 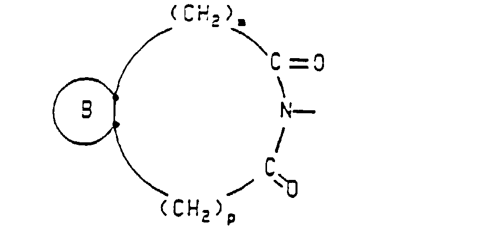 darstellt, worin B Phenyl darstellt, m 0 ist und p 1 ist.

18. Verbindung nach Anspruch 1, worin die Verbindung 2-{1-[1-(2,6-Dichlor-4-trifluormethylphenyl)-5-dimethylamino-3-ethyl-1H-pyrazol-4-ylmethyl]-naphthalin-2-yloxy}-ethanol;
enantiomeres [4-(3-Methoxymethyl-3,4-dihydro-1H-isochinolin-2-ylmethyl)-5-methylsulfanyl-2-(2,4,6-trichlorphenyl)-2H-pyrazol-3-yl]-dimethylamin, abgeleitet von (+)-3-Hydroxymethyl-1,2,3,4-tetrahydroisochinolin;
enantiomeres [2-(2,6-Dichlor-4-trifluormethylphenyl)-4-(3-ethoxymethyl-3,4-dihydro-1H-isochinolin-2-ylmethyl)-5-ethyl-2H-pyrazol-3-yl]-dimethylamin, abgeleitet von (+)-3-Hydroxymethyl-1,2,3,4-tetrahydroisochinolin;
[2-(2,6-Dichlor-4-trifluormethylphenyl)-5-ethyl-4-(7-methoxychinolin-8-ylmethyl)-2H-pyrazol-3-yl]-dimethylamin;
[2-(2,6-Dichlor-4-trifluormethylphenyl)-4-(2-ethoxynaphthalin-1-ylmethyl)-5-ethyl-2H-pyrazol-3-yl]-dimethylamin;
[4-(2-Ethoxynaphthalin-1-ylmethyl)-5-ethyl-2-(2,4,6-trichlorphenyl)-2H-pyrazol-3-yl]-dimethylamin;
[4-(7-Methoxychinolin-8-ylmethyl)-5-methylsulfanyl-2-(2,4,6-trichlorphenyl)-2H-pyrazol-3-yl]-dimethylamin;
2-{1-[5-Dimethylamino-3-methylsulfanyl-1-(2,4,6-trichlorphenyl)-1H-pyrazol-4-ylmethyl]-naphthalin-2-yloxy)-ethanol;
enantiomeres [2-(2,6-Dichlor-4-trifluormethylphenyl)-5-ethyl-4-(3-methoxymethyl-3,4-dihydro-1H-isochinolin-2-ylmethyl)-2H-pyrazol-3-yl]-dimethylamin, abgeleitet von (+)-3-Hydroxymethyl-1,2,3,4-tetrahydroisochinolin;
[4-(2-Cyclopropylmethoxynaphthalin-1-ylmethyl)-5-methylsulfanyl-2-(2,4,6-trichlorphenyl)-2H-pyrazol-3-yl]-dimethylamin, ist.

19. Zusammensetzung für die Behandlung von (a) Erkrankungen, induziert oder begünstigt durch Corticotropin-freisetzenden Faktor oder (b) entzündlichen Erkrankungen, durch Streß- und Angstzustände ausgelösten Erkrankungen, einschließlich durch Streß verursachter Depression und durch Streß verursachtem Kopfschmerz, abdominalen Darmsyndrom, Immunsuppression, HIV-Infektionen, Alzheimer Krankheit, gastrointestinalen Erkrankungen, nervöser Appetitlosigkeit, hämorrhagischem Streß, Drogen- und Alkoholentzugssymptomen, Drogensucht und Fertilitätsproblemen, umfassend eine Verbindung der Formel I, wie in einem der Ansprüche 1 bis 18 definiert, und einen pharmazeutisch verträglichen Träger.

20. Verbindung der Formel 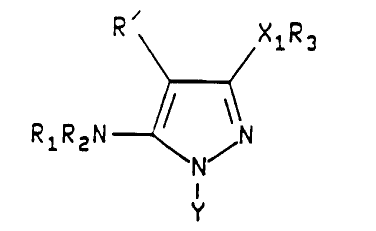 worin R' CH₂OH oder C(O)O(C₁-C₃-Alkyl) darstellt, R₁, R₂ und R₃ jeweils unabhängig lineares C₁-C₆-Alkyl, verzweigtes C₃-C₈-Alkyl, C₃-C₈-Alkenyl, worin die Doppelbindung zu dem N oder X₁, wenn X₁ Sauerstoff oder Schwefel darstellt, nicht benachbart ist, C₃-C₇-Cycloalkyl-(CH₂)ₙ, worin n 0, 1, 2, 3 oder 4 ist, darstellen; oder R₁ und R₂, wenn zusammengenommen mit dem Stickstoffatom einen gesättigten vier-, fünf- oder sechsgliedrigen Ring, gegebenenfalls kondensiert mit Benzo, bilden;
X₁ eine kovalente Bindung, CH₂NR, worin R Wasserstoff oder lineares C₁-C₆-Alkyl, 0 oder S darstellt, bedeutet; und
Y Phenyl, Thienyl, Benzothienyl, Pyridyl, Chinolyl, Pyrazinolyl, Pyrimidyl, Imidazolyl, Benzimidazolyl, Furanyl, Benzofuranyl, Thiazolyl, Benzothiazolyl, Isothiazolyl, Benzisothiazolyl, Isoxazolyl, Benzisoxazolyl, Triazolyl, Pyrazolyl, Pyrrolyl, Indolyl, Azaindolyl, Oxazolyl, Benzoxazolyl, Pyrrolidinyl, Thiazolidinyl, Morpholinyl oder Piperidinyl darstellt, wobei jeder davon mit einem bis drei von einem von Fluor, Chlor, Brom oder Methyl oder einem von Trifluormethyl substituiert sein kann, darstellt; mit der Maßgabe, daß Y nicht unsubstituiertes Phenyl darstellt.

21. Verbindung nach einem der Ansprüche 1 bis 18 zur Verwendung als Arzneimittel.

22. Verwendung einer Verbindung nach einem der Ansprüche 1 bis 18 zur Herstellung eines Arzneimittels für die Behandlung von (a) Erkrankungen, induziert oder begünstigt durch Corticotropin-freisetzenden Faktor oder (b) durch Streß- und Angstzustände ausgelösten Erkrankungen, einschließlich durch Streß verursachter Depression und durch Streß verursachtem Kopfschmerz, abdominalen Darmsyndrom, entzündlichen Erkrankungen, Immunsuppression, HIV-Infektionen, Alzheimer Krankheit, gastrointestinalen Erkrankungen, nervöser Appetitlosigkeit, hämorrhagischem Streß, Drogen- und Alkoholentzugssymptomen, Drogensucht und Fertilitätsproblemen, insbesondere Depression.

## Revendications

1. Un composé de formule: 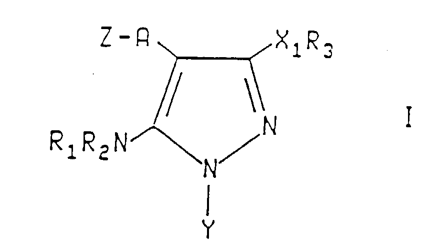 ainsi que les sels d'addition d'acide pharmaceutiquement acceptables en dérivant, formule dans laquelle:
A est CH₂;
X₁ est une liaison covalente, CH₂, NR où R est un atome d'hydrogène, un radical alkyle linéaire en C₁ à C₆, un radical alkyle ramifié en C₃ à C₈, O ou S:
R₁, R₂ et R₃, indépendamment l'un de l'autre, sont des radicaux alkyle linéaire en C₁ à C₆, alkyle ramifié en C₃ à C₈, alkényle en C₃ à C₈ dans lequel la double liaison n'est pas adjacente à l'atome d'azote, ni à X₁ lorsque X₁ est un atome d'oxygène ou de soufre, ou cycloalkyle en C₃ à C₇, (CH₂)ₙ avec n étant égal à 0, 1, 2, 3 ou 4; ou encore R₁ et R₂ pris ensemble avec l'atome d'azote auquel ils sont liés forment un cycle à quatre, cinq ou six atomes éventuellement condensé avec un cycle benzo et R₃ peut être également (CH₂)_{q}Q₁R₁₉ avec q étant 0, 1 ou 2, Q₁ étant O, S, NH ou N(alkyle en C₁ à C₆) lorsque X₁ est une liaison covalente, ou Q₁ étant une liaison covalente lorsque X₁ n'est pas une liaison covalente, et R₁₉ est un atome d'hydrogène, un radical alkyle linéaire en C₁ à C₆ linéaire, un radical alkyle ramifié en C₃ à C₈, alkényle en C₃ à C₈ ramifié ou cycloalkyle en C₃ à C₆, (CH₂)ₙ avec n étant compris entre 0 et 4, bornes comprises;
Y est un radical phényle, thiényle, benzothiényle, pyridyle, quinolyle, pyrazinolyle, pyrimidyle, imidazolyle, benzimidazolyle, furanyle, benzofuranyle, thiazolyle, benzothiazolyle, isothiazolyle, benzisothiazolyle, isoxazolyle, benzisoxazolyle, triazolyle, pyrazolyle, pyrrolyle, indolyle, azaindolyle, oxazolyle, benzoxazolyle, pyrrolidinyle, thiazolidinyle, morpholinyle ou pipéridinyle, chacun d'entre eux pouvant être substitué par un à trois de l'un quelconque des groupes fluoro, chloro, bromo ou méthyle, ou un radical trifluorométhyle: à la condition que Y ne soit pas un radical phényle non substitué; et
Z est:
(a) 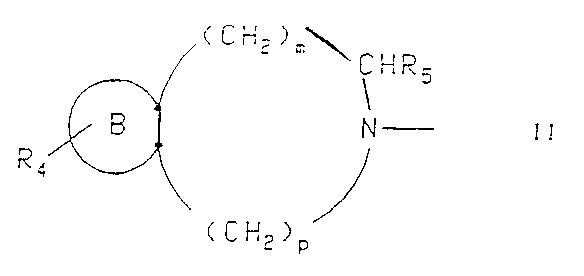 formule dans laquelle:
le cycle B est un cycle phényle, naphtyle, pyridyle, pyrimidinyle, pyrazinyle, pyridazinyle, triazinyle, pyrrolyle, pyrazolyle, imidazolyle, triazolyle, thiényle ou indoyle, chacun de ces cycles pouvant être substitué par un radical méthyle, méthoxy, fluoro, chloro, bromo ou iodo; ou un cycle carbocyclique à 5 à 6 atomes saturé, ou un cycle partiellement insaturé, comprenant une ou deux doubles liaisons;
R₄ est un atome d'hydrogène, un radical alkyle en C₁ à C₆, alkoxy en C₁ à C₆ ou un groupe hydroxy, fluoro, chloro, bromo, iodo ou tritluorométhyle;
R₅ est un atome d'hydrogène, un radical alkyle linéaire en C₁ à C₆, alkyle ramifié en C₃ à C₈, alkényle en C₃ à C₈, ou (CH₂)ₒ-X₂-(CH₂)ᵣ-Q₂-R₆;
R₆ est un atome d'hydrogène, un radical alkyle linéaire en C₁ à C₆, alkyle ramifié en C₃ à C₈, ou alkényle en C₃ à C₈;
X₂ et Q₂, indépendamment l'un de l'autre, consistent en O, S, NH, N(alkyle en C₁ à C₆), ou l'un des groupes X₂ et Q peut être une liaison covalente;
m est égal à 0 ou 1;
o est égal à 1 ou 2;
p est égal à 1 ou 2;
r est égal à 0, 1 ou 2;
(b) 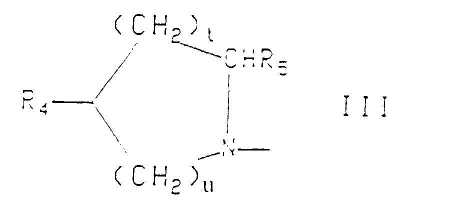 formule dans laquelle:
R₄ et R₅ sont tels que définis ci-dessus; et
t et u, indépendamment l'un de l'autre, sont égaux à 1 ou 2;
(c)
-NR₇R₈
formule dans laquelle:
R₇ et R₈ sont, indépendamment l'un de l'autre, un atome d'hydrogène, un radical alkyle linéaire en C₁ à C₆, alkyle ramifié en C₃ à C₈, alkényle en C₃ à C₈, (CH₂)ᵥCH₂OH, (CH₂)ᵥNR₉R₁₀, formules dans lesquelles v est compris entre 0 et 3 et R₉ et R₁₀ sont indépendamment l'un de l'autre un atome d'hydrogène ou un radical alkyle linéaire en C₁ à C₆, cycloalkyle en C₁ à C₁₂, cycloalkyle en C₃ à C₁₂ (CH₂)ₙ, bicycloalkyle en C₆ à C₁₀, (CH₂)ₙ, formules dans lesquelles n est compris entre 0 et 4, bornes incluses, un radical cycloalkyle en C₃ à C₆ condensé avec un groupe benzo, hydroxyalkyle en C₁ à C₆, phényle, phényl(alkylène en C₃ à C₆), chacun d'entre eux pouvant être substitué par un ou deux groupes hydroxy, fluoro, chloro, bromo, alkyle en C₁ à C₅ ou alkoxy en C₁ à C₅; ou R₇ et R₈, ensemble avec l'atome d'azote auquel ils sont liés, peuvent former un cycle saturé ou partiellement saturé comprenant 5 à 7 atomes qui peut comprendre l'un des groupes consistant en O, S, NH ou N(alkyle en C₁ à C₆) et qui peut être substitué par un radical alkyle en C₁ à C₆, hydroxy ou phényle, dans lequel aucune double liaison n'est adjacente à l'un quelconque des hétéroatomes;
(d) 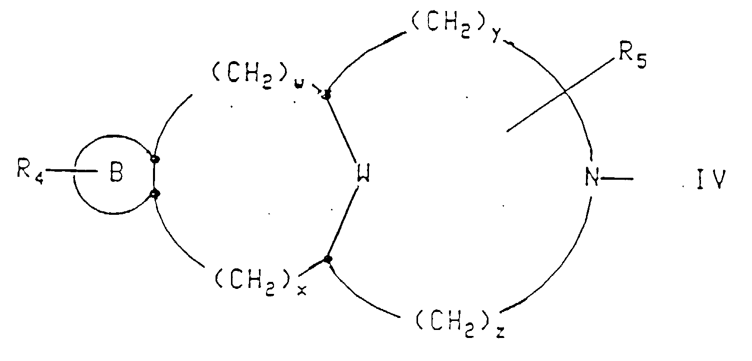 formule dans laquelle:
B, R₄ et R₅ sont tels que définis ci-dessus,
w, x, y et z sont indépendamment les uns des autres, égaux à 1 ou 2, et
W est (CH₂)_{q}, formule dans laquelle q est tel que défini ci-dessus, N(alkyle en C₁ à C₆) ou un atome d'oxygène;
(e) 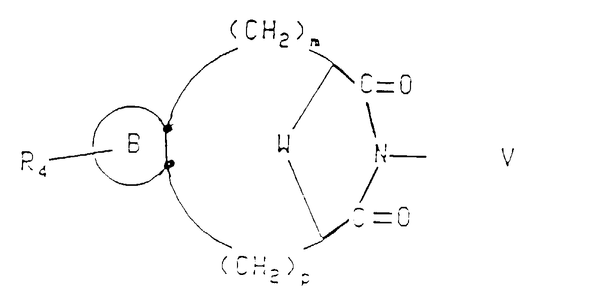 formule dans laquelle:
W, B, R₄, m et p sont tels que définis ci-dessus;
(f) 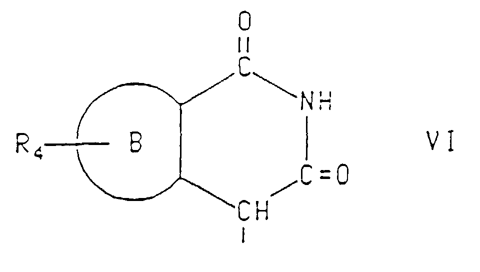 formule dans laquelle:
B et R₄ sont tels que définis ci-dessus;
(g)
O(CH₂)ᵥR₁₁
formule dans laquelle
v est compris entre 0 et 3, bornes incluses, et
R₁₁ est un radical alkyle linéaire en C₁ à C₆, alkyle ramifié en C₃ à C₈, alkyle, phényle, naphtyle, 1,2,3,4-tétrahydronaphtyle, thiényle, benzothiényle, pyridyle, quinolyle, pyrazinolyle, pyrimidyle, imidazolyle, benzimidazolyle, furanyle, benzofuranyle, thiazolyle, benzothiazolyle, isothiazolyle, benzisothiazolyle, isoxazolyle, benzisoxazolyle, triazolyle, pyrazolyle, pyrrolyle, indolyle, azaindolyle, oxazolyle, benzoxazolyle, pyrrolidinyle, thiazolidinyle, morpholinyle, pipéridinyle ou thiényle, chacun d'entre eux pouvant être substitué par un ou deux de l'un des groupes fluoro, chloro, bromo, méthyle ou trifluorométhyle;
(h) 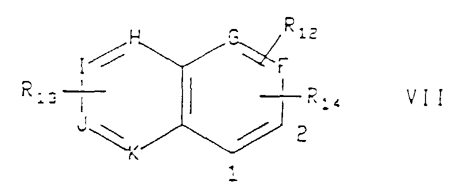 formule dans laquelle:
la partie de formule VII est liée par la position 1 ou 2 à la partie A représentée dans la formule I, tandis que R₁₄ est lié en position 2 ou 1, respectivement;
F, G, H, I, J et K, indépendamment les uns des autres, consistent en C ou N, à condition que pas plus de trois des groupes H, I, J et K ne soient de l'azote et qu'il n'y ait pas plus de deux atomes d'azote adjacents;
R₁₂ et R₁₃, indépendamment l'un de l'autre, consistent en un atome d'hydrogène, un radical alkyl linéaire en C₁ à C₆, alkyle ramifié en C₃ à C₈, alkényle en C₃ à C₈, fluoro, chloro, bromo, trifluorométhyle, hydroxy, thiol, alkoxy en C₁ à C₁₂, thioalkanyle en C₁ à C₁₂, alkénoxy en C₃ à C₁₂ ou thioalkényle en C₃ à C₁₂, dans lesquels la double liaison n'est pas adjacente à l'atome d'oxygène ou de soufre; et
R₁₄ est un groupe hydroxy, alkoxy en C₁ à C₁₂, alkénoxy en C₃ à C₁₂ dans lequel la double liaison n'est pas adjacente à l'atome d'oxygène, ou X₂-(CH₂)ᵣQ₂R₆, formule dans laquelle X₂, r, Q₂ et R₆ sont tels que définis ci-dessus dans le paragraphe (a), si ce n'est que Q₂ n'est pas du soufre, ou R₁₄ est NR₁₅R₁₆, formule dans laquelle R₁₅ et R₁₆, indépendamment l'un de l'autre, consistent en un atome d'hydrogène, un radical alkyle linéaire en C₁ à C₆, alkyle ramifié en C₃ à C₈, alkényle en C₃ à C₈, dans lequel la double liaison n'est pas adjacente à l'atome d'azote, ou cycloalkyle en C₃ à C₇, (CH₂)ₙ formule dans laquelle n est tel que défini ci-dessus, ou R₁₅ et R₁₆, ensemble avec l'atome d'azote auquel ils sont liés, forment un cycle saturé à cinq ou six atomes, éventuellement condensé avec un groupe benzo; ou
(i) 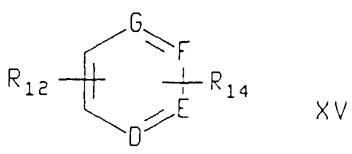 formule dans laquelle:
D, E, F et G, indépendamment l'un de l'autre, consistent en C ou N, à la condition que pas plus de deux des groupes D. E, F et G ne consistent en un atome d'azote, et
R₁₂ et R₁₄ sont tels que définis dans le paragraphe (h), A défini ci-dessus, étant lié à un atome de carbone dans la formule XV; et
R₁₄ étant lié à un atome de carbone adjacent à l'atome de carbone auquel A est lié.

2. Un composé selon la revendication 1, dans lequel Y est un radical phényle trisubstitué en position 2,4,6.

3. Un composé selon la revendication 2, dans lequel Y est un 2,4,6-trichlorophényle, 2,6-dichloro-4-trifluorométhylphényle ou 2,6-dibromo-4-fluorophényle.

4. Un composé selon la revendication 1 ou 2, dans lequel X₁-R₃ est un radical éthyle ou méthylthio.

5. Un composé selon l'une quelconque des revendications 1 à 4, dans lequel R₁ et R₂ sont des radicaux méthyle.

6. Un composé selon l'une quelconque des revendications 1 à 5, dans lequel Z est NR₇R₈ avec R₇ étant un radical phényle ou phényle substitué par un groupe fluoro, chloro, nitro, méthyle ou méthoxy.

7. Un composé selon la revendication 6, dans lequel R₈ est CH₂CH₂CH₂OH, CH₂CH₂OH ou un radical méthyle.

8. Un composé selon l'une quelconque des revendications 1 à 5, dans lequel Z est le 1,2,3,4-tétrahydroisoquinolin-2-yle et R₅ est (CH₂)ₒ-X₂-(CH₂)ᵣ-Q₂-R₆.

9. Un composé selon la revendication 8. dans lequel R₅ est (CH₂)ₖOH avec k étant égal à 1 à 4, bornes incluses, ou CH₂OCH₂CH₂OR₆.

10. Un composé selon l'une quelconque des revendications 1 à 5, dans lequel Z est la 1,2,3,4-tétrahydroisoquinolin-2-yle, dans lequel R₅ est substitué en position 3 et la configuration absolue en position 3 est S ou R ou R,S.

11. Un composé selon l'une quelconque des revendications 1 à 5, dans lequel Z présente la formule: 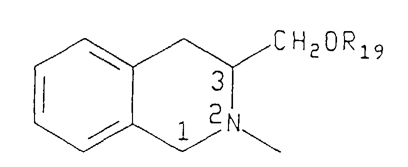 formule dans laquelle la configuration absolue en position 3 est déterminée par sa dérivation du (+)-3-hydroxyméthyl-1,2,3,4-tétrahydroisoquinoline, avec R₁₉ étant un radical méthyle, éthyle, isopropyle, cyclopropylméthylène, ou hydroxyéthylène.

12. Un composé selon l'une quelconque des revendications 1 à 5, dans lequel Z est tel que défini en (h).

13. Un composé selon la revendication 12, dans lequel A est lié en position 1, R₁₄ est lié en position 2 et est X₂-(CH₂)ᵣQ₂R₆.

14. Un composé selon la revendication 13, dans lequel F, G, H, I et J sont chacun un atome de carbone, K est un atome de carbone ou d'azote et R₁₄ est un radical 2-méthoxy, 2-éthoxy, 2-isopropoxy ou 2-cyclopropylméthoxy.

15. Un composé selon l'une quelconque des revendications 1 à 5, dans lequel Z est: 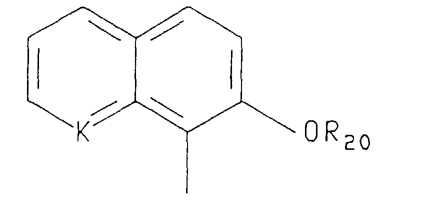 formule dans laquelle:
K est C ou N; et
R₂₀ est un groupe méthyle, éthyle, isopropyle, cyclopropylméthylène ou hydroxyéthylène.

16. Un composé selon l'une quelconque des revendications 1 à 5, dans lequel Z est tel que défini dans (a), B est un groupe phényle, p et m sont chacun égaux à un, et R₅ est CH₂OCH₃.

17. Un composé selon l'une quelconque des revendications 1 à 5, dans lequel Z est: 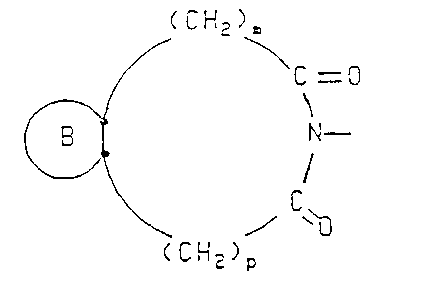 formule dans laquelle:
B est un radical phényle;
m est égal à 0; et
p est égal à 1.

18. Un composé selon la revendication 1, dans lequel ledit composé consiste en:
- 2-{1-[1-(2,6-dichloro-4-trifluorométhylphényl)-5-diméthylamino-3-éthyl-1H-pyrazol-4-ylméthyl]-naphtalen-2-yloxy}-éthanol;
- [4-(3-méthoxyméthyl-3,4-dihydro-1H-isoquinolin-2-ylméthyl)-5-méthylsulfanyl-2-(2,4,6-trifluorophényl)-2H-pyrazol-3-yl]-diméthylamine énantiomère dérivé de (+)-3-hydroxyméthyl-1,2,3,4-tétrahydroisoquinoline;
- [2-(2,6-dichloro-4-trifluorométhylphényl)-4-(3-éthoxyméthyl-3,4-dihydro-1H-isoquinolin-2-ylméthyl)-5-éthyl-2H-pyrazol-3-yl]-diméthylamine énantiomère dérivé de (+)-3-hydroxyméthyl-1,2,3,4-tétrahydroisoquinoline;
- [2-(2,6-dichloro-4-tritluorométhylphényl)-5-éthyl-4-(7-méthoxyquinolin-8-ylméthyl)-2H-pyrazol-3-yl]-diméthylamine;
- [2-(2,6-dichloro-4-trifluorométhylphényl)-4-(2-éthoxy-naphtalèn-1-yl-méthyl)-5-éthyl-2H-pyrazol-3-yl]-diméthylamine;
- [4-(2-éthoxynaphtalèn-1-ylméthyl)-5-éthyl-2-(2,4-trichlorophényl)-2H-pyrazol-3-yl]-diméthylamine;
- [4-(7-méthoxyquinolin-8-ylméthyl)-5-méthylsulfanyl-2-(2,4,6-trichlorophényl)-2H-pyrazol-3-yl]-diméthylamine;
- 2-{1-[5-diméthylamino-3-méthylsulfanyl-1-(2,4,6-trichlorophényl)-1H-pyrazol-4-ylméthyl]-naphtalèn-2-yloxy}-éthanol;
- [2-(2,6-dichloro-4-trifluorométhylphényl)-5-éthyl-4-(3-méthoxyméthyl-3,4-dihydro-1H-isoquinolin-2-ylméthyl)-2H-pyrazol-3-yl]-diméthylamine énantiomère dérivé de (+)-3-hydroxyméthyl-1,2,3,4-tétrahydroisoquinoline; et
- [4-(2-cyclopropylméthoxynaphtalèn-1-ylméthyl)-5-méthylsulfanyl-2-(2,4,6-trichlorophényl)-2H-pyrazol-3-yl]-diméthylamine.

19. Une composition pour le traitement:
(a) de maladies induites ou facilitées par le facteur de libération de corticotropine, ou
(b) de désordres inflammatoires, de désordres associés au stress et à l'anxiété, incluant les maux de tête et la dépression induits par le stress, du syndrome du bol abdominal, de la suppression immunitaire, des infections liées au virus d'immunodéficience humaine HIV, de la maladie d'Alzheimer, des maladies gastro-intestinales, de l'anorexie mentale, du stress hémorragique, des symptomes liés au sevrage de l'alcool et des drogues, de la dépendance à la drogue et des problèmes de fertilité.
qui comprend un composé de formule I tel que défini dans l'une quelconque des revendications 1 à 18, ainsi qu'un support pharmaceutiquement acceptable.

20. Un composé de formule: 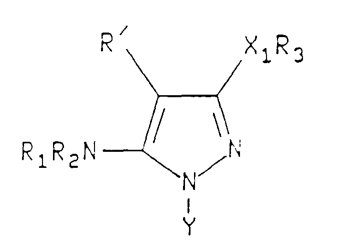 formule dans laquelle:
R' est CH₂OH ou C(O)O(alkyle en C₁ à C₃),
R₁, R₂ et R₃, indépendamment l'un de l'autre sont des radicaux alkyles linéaires en C₁ à C₆, alkyles ramifiés en C₃ à C₈, alkényle en C₃ à C₈ dans laquelle la double liaison n'est pas adjacente à l'atome d'azote ni à X₁ lorsque X₁ est un atome d'oxygène ou de soufre, cycloalkyle en C₃ à C₇, (CH₂)ₙ, formule dans laquelle n est égal à 0, 1, 2, 3 ou 4; ou R₁ et R₂ pris ensemble avec l'atome d'azote auquel ils sont liés, forment un cycle saturé à quatre, cinq ou six atomes, éventuellement condensé avec un groupe benzo;
X₁ est une liaison covalente, CH₂NR, avec R étant un atome d'hydrogène ou un radical alkyle linéaire en C₁ à C₆, O ou S; et
Y est un radical phényle, thiényle, benzothiényle, pyridyle, quinolyle, pyrazinolyle, pyrimidyle, imidazolyle, benzimidazolyle, furanyle, benzofuranyle, thiazolyle, benzothiazolyle, isothiazolyle, benzisothiazolyle, isoxazolyle, benzisoxazolyle, triazolyle, pyrazolyle, pyrrolyle, indolyle, azaindolyle, oxazolyle, benzoxazolyle, pyrrolidinyle, thiazolidinyle, morpholinyle ou pipéridinyle, chacun d'entre eux pouvant être substitué par un à trois de l'un quelconque des groupes tluoro, chloro, bromo, ou méthyle, ou un groupe trifluorométhyle, à la condition que Y ne soit pas un radical phényle non substitué.

21. Un composé tel que revendiqué dans l'une quelconque des revendications 1 à 18, destiné à être utilisé en tant que médicament.

22. L'utilisation d'un composé ainsi que revendiqué dans l'une quelconque des revendications 1 à 18, pour la fabrication d'un médicament pour le traitement:
(a) de maladies induites ou facilitées par le facteur de libération de corticotropine, ou
(b) de désordres inflammatoires, de désordres associés au stress et *à* l'anxiété, incluant les maux de tête et la dépression induits par le stress, du syndrome du bol abdominal, de la suppression immunitaire, des infections liés au virus d'immunodéficience humaine HIV, de la maladie d'Alzheimer, des maladies gastro-intestinales, de l'anorexie mentale, du stress hémorragique et des symptômes liés au sevrage de l'alcool et des drogues. de la dépendance à la drogue et des problèmes de fertilité, notamment la dépression.
